# EUROPEAN PATENT APPLICATION

(11) **EP 4 799 619 A1**
(43) Date of publication of application: **02.09.2026**
(21) Application number: 24858868.3
(22) Date of filing: 23.08.2024
(51) Int. Cl.: A61K 31/404, A61K 31/17, A61K 31/415, A61K 31/352, A61P 17/00, A61P 29/00, C07D 209/40

(54) **COMPOUND TARGETING EGR-1 AND USE THEREOF FOR TREATMENT OF ATOPIC DERMATITIS**

(30) Priority: 25.08.2023 KR 20230112067
(71) Applicant: AgeraMedi, Inc., Seoul 05029 (KR)
(72) Inventor: SHIN, Soon Young, Gwangin-gu, Seoul 05006 (KR); KOH, Dongsoo, Seongnam-si, Gyeonggi-do 13523 (KR); LIM, Yoongho, Gwanak-gu, Seoul 08845 (KR)
(74) Representative: ABG Intellectual Property Law, S.L.
(86) International application number: PCT/IB2024/058204
(87) International publication number: WO 2025/046420

(57) **Abstract**

The present invention relates to a compound targeting EGR-1 and a use thereof for treatment of atopic dermatitis. The compound targeting EGR-1, an isomer thereof, or a pharmaceutically acceptable salt thereof, and a composition comprising same, according to the present invention, inhibit the DNA binding capacity of the transcription factor EGR-1, thereby reducing inflammatory cytokines produced by the involvement of EGR-1, and thus may be useful for the prevention, alleviation, or treatment of atopic dermatitis.

## Description

### TECHNICAL FIELD

The present invention relates to a compound targeting EGR-1, an isomer thereof or a pharmaceutically acceptable salt thereof, and a composition for preventing, ameliorating, or treating atopic dermatitis comprising the same.

### BACKGROUND

Atopic dermatitis is a chronic and recurrent skin inflammatory disease in which allergic symptoms occur on the skin due to an immune hypersensitivity reaction, resulting in weakening of the skin barrier and dryness of the skin with severe itching. It is known that atopic dermatitis causes overexpression of inflammatory cytokines in lesion sites, thereby worsening inflammation and itching symptoms. Cytokines are proteins secreted from cells related to microbial infection or immunity, and are factors affecting interactions among cells involved in the immune system. When production and secretion of cytokines are excessive or not properly regulated, they may mediate or worsen various diseases related to deficiency of immune regulation function and physiological conditions (e.g., inflammation).

In the lesions of acute atopic dermatitis, thymic stromal lymphopoietin (TSLP) secreted from keratinocytes, interleukin-4 (IL-4) and interleukin-13 (IL-13) secreted from CD4⁺ helper type 2 T-lymphocytes (Th2), and interleukin-31 (IL-31) are highly increased, and in the lesions of chronic atopic dermatitis, cytokines such as interferon-gamma (IFNγ) and tumor necrosis factor-alpha (TNFα) secreted from Th1-lymphocytes are increased.

In the past, steroid-based topical agents have been mainly used as therapeutic agents for atopic dermatitis. Steroid agents are artificially synthesized from cortisol, a hormone secreted from the adrenal cortex, and can inhibit hypersensitivity immune response to reduce production of cytokines and relieve itching sensation in the skin. However, in particular, when infants or young children use steroid agents for a long time, their immunity may be weakened and side effects such as skin atrophy or growth retardation may occur. As a result, immunosuppressive agents such as calcineurin inhibitors including cyclosporin, which are non-steroidal agents, were developed and commercialized, but these also cause various side effects such as cancer development due to weakening of body immunity when they are used for a long period (J Invest Dermatol 2007;127:808-816). Therefore, studies on materials that alleviate symptoms of atopic dermatitis by methods other than those using conventional steroid agents have been conducted.

Early growth response-1 (EGR-1) is a protein encoded by the EGR-1 gene and is an important transcription factor for cell growth, differentiation, survival, apoptosis, and immune responses. EGR-1 has three Cys2-His2 type zinc finger DNA binding domains, and transcription starts and gene expression can occur only when EGR-1 binds to DNA. In other words, EGR-1 regulates the production of proteins including cytokines.

EGR-1 regulates inflammatory responses in various tissues and its expression is increased in dermal wound sites and psoriatic tissue in the skin. In addition, EGR-1 mediates the production of thymic stromal lymphopoietin (TSLP) induced by IL-33 in human keratinocytes and increases production of psoriasin (S100A7), a psoriasis inducer produced by IL-17, thereby being considered an important factor in skin inflammatory responses (Exp Dermatol 2015;24:857-63; Exp Dermatol 2014;23:890-5).

The inventors have found through previous studies that when the skin is exposed to an inflammatory environment, EGR-1 expressed in keratinocytes is a key transcription factor that increases production of cytokines (e.g., TSLP, IL-1β, IL-6, IL-17, IL-23) and chemokines (CXCL1 and CCL5) that attract inflammatory cells such as T-lymphocytes and mast cells to the lesion site, and increases expression of the POMC gene, a precursor of beta-endorphin, a neurotransmitter that stimulates skin sensory nerves and transmits itch to the brain (see Non-Patent Literature 1 and 2). Therefore, substances that inhibit the DNA binding ability of EGR-1 may be an important strategy for treating atopic dermatitis.

### PROBLEMS

An object of the invention is to provide a compound targeting a transcription factor EGR-1, an isomer thereof, or a pharmaceutically acceptable salt thereof.

Another object of the present invention is to provide a composition for preventing, ameliorating, or treating atopic dermatitis comprising a compound targeting EGR-1, an isomer thereof, or a pharmaceutically acceptable salt thereof.

### SOLUTIONS

In order to achieve the above objects, the present invention provides a pharmaceutical composition for preventing, ameliorating, or treating atopic dermatitis comprising as an active ingredient a compound represented by Formula 1 below, an isomer thereof, or a pharmaceutically acceptable salt thereof.

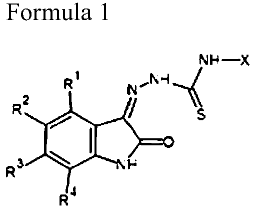

in Formula 1,
X is hydrogen, C₁-C₆ alkyl, -CH₂-C_{6 -10} aryl, or 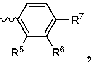 where C₁-C₆ alkyl and -CH₂-C₆₋₁₀ aryl may each independently be unsubstituted or substituted with one to three halogen atoms,
R¹ is hydrogen or halogen,
R² is hydrogen, halogen, hydroxy, C₁-C₆ alkyl, or C₁-C₆ alkoxy, where C₁-C₆ alkyl and C₁-C₆ alkoxy may each independently be unsubstituted or substituted with one to three halogen atoms,
R³, R⁴, and R⁵ are each independently hydrogen, halogen, hydroxy, or C₁-C₆ alkoxy, where C₁-C₆ alkoxy may be unsubstituted or substituted with one to three halogen atoms,
R⁶ is hydrogen, halogen, hydroxy, -NO₂, C₁-C₆ alkyl, or C₁-C₆ alkoxy, where C₁-C₆ alkyl and C₁-C₆ alkoxy may each independently be unsubstituted or substituted with one to three halogen atoms, and
R⁷ is hydrogen, halogen, hydroxy, -NO₂, -CN, C₁-C₆ alkyl, or C₁-C₆ alkoxy, where C₁-C₆ alkyl and C₁-C₆ alkoxy may each independently be unsubstituted or substituted with one to three halogen atoms.

The present invention provides a quasi-drug composition for preventing or ameliorating atopic dermatitis comprising the compound represented by Formula 1, an isomer thereof, or a pharmaceutically acceptable salt thereof as an active ingredient.

In addition, the present invention provides any one compound selected from the group consisting of the following novel compounds targeting EGR-1, an isomer thereof, or a pharmaceutically acceptable salt thereof:
(Z)-N-(4-Cyanophenyl)-2-(7-fluoro-2-oxindoline-3-ylidene)hydrazinecarbothioamide;
(Z)-2-(7-Fluoro-2-oxoindolin-3-ylidene)-N-(4-(trifluoromethyl)phenyl)hydrazinecarbothioamide;
(Z)-2-(6-Methoxy-2-oxoindolin-3-ylidene)-N-phenylhydrazinethioamide;
(Z)-2-(6-Methoxy-2-oxoindolin-3-ylidene)-N-(3-nitrophenyl)hydrazinethioamide;
(Z)-2-(6-Methoxy-2-oxoindolin-3-ylidene)-N-(2-methoxyphenyl)hydrazinethioamide;
(Z)-N-(4-Cyanophenyl)-2-(6-methoxy-2-oxoindolin-3-ylidene)hydrazinethioamide;
(Z)-2-(6-Methoxy-2-oxoindolin-3-ylidene)-N-(4-(trifluoromethyl)phenyl)hydrazinethioamide;
(Z)-N-Ethyl-2-(6-methoxy-2-oxoindolin-3-ylidene)hydrazinethioamide;
(Z)-2-(5-Methyl-2-oxoindolin-3-ylidene)-N-(3-nitrophenyl)hydrazinethioamide;
(Z)-2-(4-Bromo-2-oxoindolin-3-ylidene)-N-(3-nitrophenyl)hydrazinethioamide;
(Z)-2-(6-Bromo-2-oxoindolin-3-ylidene)-N-(3-nitrophenyl)hydrazinethioamide;
(Z)-2-(4-Bromo-2-oxoindolin-3-ylidene)-N-(4-nitrophenyl)hydrazinethioamide;
(Z)-2-(4-Bromo-2-oxoindolin-3-ylidene)-N-phenylhydrazinethioamide;
(Z)-2-(6-Bromo-2-oxoindolin-3-ylidene)-N-phenylhydrazinethioamide;
(Z)-2-(7-Bromo-2-oxoindolin-3-ylidene)-N-(3-nitrophenyl)hydrazinecarbothioamide;
(Z)-2-(4-Bromo-2-oxoindolin-3-ylidene)-N-(3-methoxyphenyl)hydrazinecarbothioamide;
(Z)-2-(4-Bromo-2-oxoindolin-3-ylidene)-N-(4-methoxyphenyl)hydrazinecarbothioamide;
(Z)-2-(6-Bromo-2-oxoindolin-3-ylidene)-N-(4-nitrophenyl)hydrazinecarbothioamide;
(Z)-2-(4-Chloro-2-oxoindolin-3-ylidene)-N-(2-methoxyphenyl)hydrazinethioamide;
(Z)-2-(7-Fluoro-2-oxoindolin-3-ylidene)-N-(4-nitrophenyl)hydrazinethioamide;
(Z)-2-(6-Methoxy-2-oxoindolin-3-ylidene)-N-(3-(trifluoromethyl)phenyl)hydrazinecarbothioamide;
(Z)-N-(4-Fluorophenyl)-2-(6-methoxy-2-oxoindolin-3-ylidene)hydrazine-1-carbothioamide;
(Z)-N-(4-Bromophenyl)-2-(6-methoxy-2-oxoindolin-3-ylidene)hydrazine-1-carbothioamide;
(Z)-N-Benzyl-2-(6-methoxy-2-oxoindolin-3-ylidene)hydrazine-1-carbothioamide;
(Z)-N-(4-Cyanophenyl)-2-(5-methyl-2-oxoindolin-3-ylidene)hydrazinecarbothioamide; and
(Z)-2-(5-Methyl-2-oxoindolin-3-ylidene)-N-(m-tolyl)hydrazinecarbothioamide.

The present invention also provides a composition comprising any one of the compounds selected from the group consisting of the following compounds, an isomer thereof, or a pharmaceutically acceptable salt thereof:
(Z)-N-(4-Cyanophenyl)-2-(7-fluoro-2-oxoindolin-3-ylidene)hydrazinecarbothioamide;
(Z)-2-(7-Fluoro-2-oxoindolin-3-ylidene)-N-(4-(trifluoromethyl)phenyl)hydrazinecarbothioamide;
(Z)-2-(6-Methoxy-2-oxoindolin-3-ylidene)-N-phenylhydrazinecarbothioamide;
(Z)-2-(6-Methoxy-2-oxoindolin-3-ylidene)-N-(3-nitrophenyl)hydrazine-1-carbothioamide;
(Z)-2-(6-Methoxy-2-oxoindolin-3-ylidene)-N-(2-methoxyphenyl)hydrazine-1-carbothioamide;
(Z)-N-(4-Cyanophenyl)-2-(6-methoxy-2-oxoindolin-3-ylidene)hydrazinecarbothioamide;
(Z)-2-(6-Methoxy-2-oxoindolin-3-ylidene)-N-(4-(trifluoromethyl)phenyl)hydrazinecarbothioamide;
(Z)-N-Ethyl-2-(6-methoxy-2-oxoindolin-3-ylidene)hydrazinecarbothioamide;
(Z)-2-(5-Methyl-2-oxoindolin-3-ylidene)-N-(3-nitrophenyl)hydrazinecarbothioamide;
(Z)-2-(4-Bromo-2-oxoindolin-3-ylidene)-N-(3-nitrophenyl)hydrazinecarbothioamide;
(Z)-2-(6-Bromo-2-oxoindolin-3-ylidene)-N-(3-nitrophenyl)hydrazinecarbothioamide;
(Z)-2-(4-Bromo-2-oxoindolin-3-ylidene)-N-(4-nitrophenyl)hydrazinecarbothioamide;
(Z)-2-(4-Bromo-2-oxoindolin-3-ylidene)-N-phenylhydrazinecarbothioamide;
(Z)-2-(6-Bromo-2-oxoindolin-3-ylidene)-N-phenylhydrazinecarbothioamide;
(Z)-2-(7-Bromo-2-oxoindolin-3-ylidene)-N-(3-nitrophenyl)hydrazinecarbothioamide;
(Z)-2-(4-Bromo-2-oxoindolin-3-ylidene)-N-(3-methoxyphenyl)hydrazinethioamide;
(Z)-2-(4-Bromo-2-oxoindolin-3-ylidene)-N-(4-methoxyphenyl)hydrazinethioamide;
(Z)-2-(6-Bromo-2-oxoindolin-3-ylidene)-N-(4-nitrophenyl)hydrazinethioamide;
(Z)-2-(4-Chloro-2-oxoindolin-3-ylidene)-N-(2-methoxyphenyl)hydrazinethioamide;
(Z)-2-(7-Fluoro-2-oxoindolin-3-ylidene)-N-(4-nitrophenyl)hydrazinethioamide;
(Z)-2-(6-Methoxy-2-oxoindolin-3-ylidene)-N-(3-(trifluoromethyl)phenyl)hydrazinethioamide;
(Z)-N-(4-Fluorophenyl)-2-(6-methoxy-2-oxoindolin-3-ylidene)hydrazine-1-carbothioamide;
(Z)-N-(4-Bromophenyl)-2-(6-methoxy-2-oxoindolin-3-ylidene)hydrazine-1-carbothioamide;
(Z)-N-Benzyl-2-(6-methoxy-2-oxoindolin-3-ylidene)hydrazine-1-carbothioamide;
(Z)-N-(4-Cyanophenyl)-2-(5-methyl-2-oxoindolin-3-ylidene)hydrazinethioamide; and
(Z)-2-(5-Methyl-2-oxoindolin-3-ylidene)-N-(m-tolyl)hydrazinecarbothioamide.

### EFFECTS

A compound targeting EGR-1, an isomer thereof or a pharmaceutically acceptable salt thereof, and a composition comprising the same according to the present invention can be usefully used for preventing, ameliorating, or treating atopic dermatitis by inhibiting formation of cytokines causing skin inflammation through binding to a binding site of DNA of transcription factor EGR-1.

### BRIEF DESCRIPTION OF DRAWINGS

FIG. 1 schematically shows an EGR1-DNA binding activity assay (EDBA) experimental method.
FIG. 2 is a graph showing the results of measuring an inhibition rate of DNA binding activity of EGR-1 by compounds according to an embodiment of the present invention.
FIG. 3 is a graph showing the results of an in vitro cytotoxicity assay of the compounds according to the embodiment of the present invention.
FIG. 4 shows interactions between compound 21 and amino acid residues of EGR-1.
FIG. 5 is an image showing a three-dimensional structure of a complex between compound 21 and EGR-1 predicted using the PyMOL program.
FIG. 6 shows interactions between compound 23 and amino acid residues of EGR-1.
FIG. 7 is an image showing a three-dimensional structure of a complex between Compound 23 and EGR-1 predicted using the program PyMOL.
FIG. 8 shows interactions between Compound 25 and amino acid residues of EGR-1.
FIG. 9 is an image showing a three-dimensional structure of a complex between Compound 25 and EGR-1 predicted using the PyMOL program.

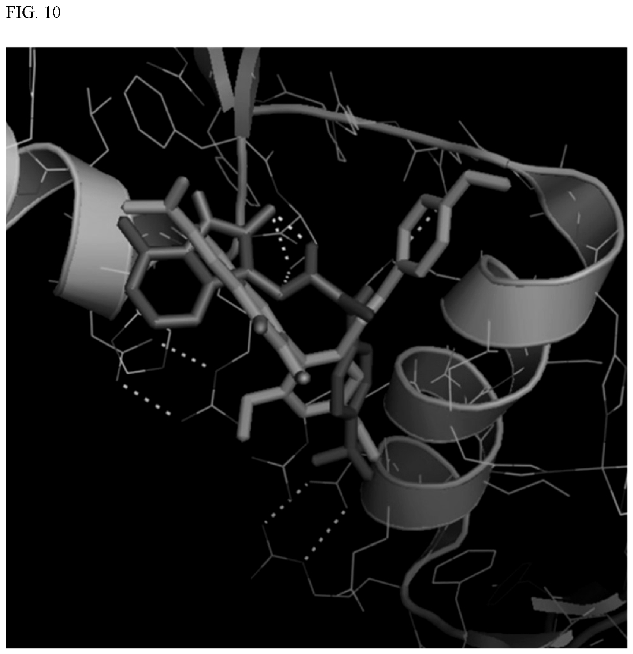

FIG. 10 shows the results of a molecular docking experiment of Compound 21.

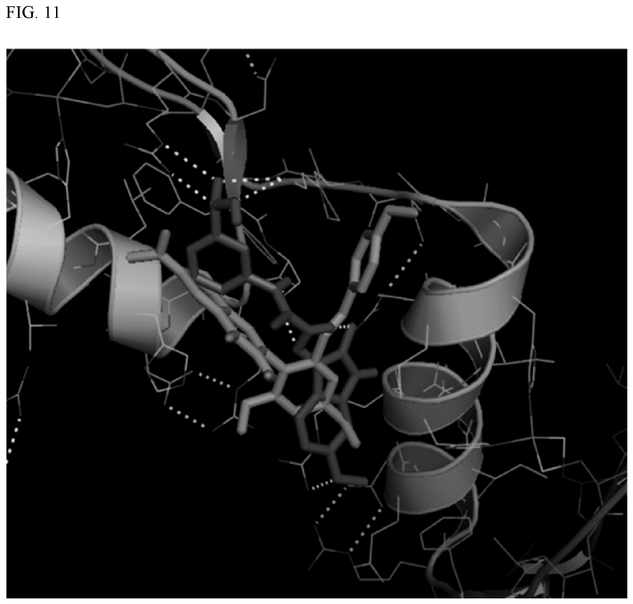

FIG. 11 shows the results of a molecular docking experiment of Compound 23.

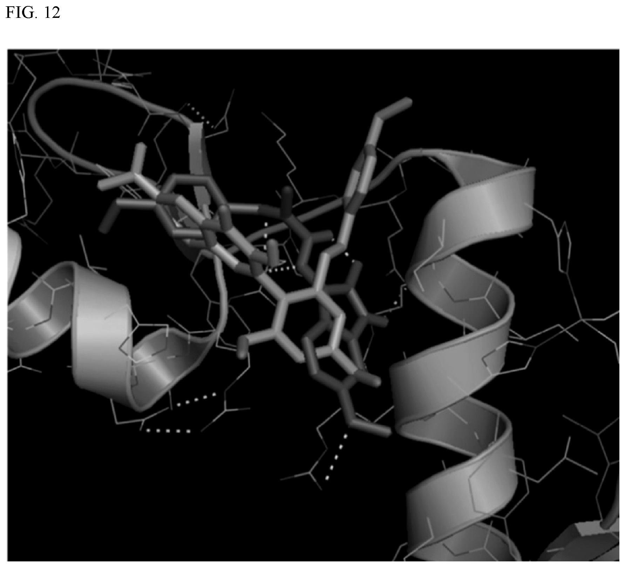

FIG. 12 shows the results of a molecular docking experiment of Compound 25.
FIG. 13 shows the results of an electrophoretic mobility shift assay (EMSA) performed on keratinocytes treated with the compounds according to one embodiment of the present invention.
FIG. 14 shows the results of reverse transcription-polymerase chain reaction (RT-PCR) performed after treating keratinocytes with the compounds according to one embodiment of the present invention.
FIGS. 15 to 17 show the therapeutic effects of three compounds (Compounds 21, 23, and 25) on atopic dermatitis in an animal model of atopic dermatitis.

### EMBODIMENTS

The present invention will now be described in more detail.

The term "halogen" in the present invention refers to F, Cl, Br, or I unless otherwise stated.

The term "hydroxy" refers to an -OH group.

The term "cyano" refers to a -C≡N group.

The term "alkyl" refers to a linear or branched saturated hydrocarbon functional group. For example, "C₁-C₆ alkyl" has 1 to 6 carbon atoms. Specifically, C₁-C₆ alkyl includes methyl, ethyl, n-propyl, isopropyl, n-butyl, isobutyl, tert-butyl, sec-butyl, n-pentyl, isopentyl, neopentyl, tertpentyl, 1-methylbutyl, n-hexyl, 1-methylpentyl, 2-methylpentyl, 4-methyl-2-pentyl, 3,3-dimethylbutyl, 2-ethylbutyl, and the like, but is not limited thereto. In an embodiment, the alkyl group can be independently substituted with one or more substituents such as 1 to 3 halogens, hydroxy, C₁-C₆ alkyl, and other hydrocarbon groups.

The term "alkoxy" refers to an -O-alkyl group, where the alkoxy group may be independently substituted with one or more substituents. For example, C₁-C₆ alkoxy includes methoxy, ethoxy, n-propoxy, isopropoxy, n-butoxy, isobutoxy, tert-butoxy, n-pentoxy, isopentoxy, tert-pentoxy, sec-pentoxy, neopentoxy, hexyloxy, and the like, but is not limited thereto. In an embodiment, the alkoxy group may be independently substituted with one or more substituents such as 1 to 3 halogens, hydroxy, C₁-C₆ alkyl, and other hydrocarbon groups.

The term "aryl" refers to a hydrocarbon monocyclic or bicyclic aromatic ring. That is, aryl herein may include phenyl, naphthyl, and the like as well as biaryls unless otherwise defined. In one embodiment of the invention, C₆-C₁₀ aryl refers to an aromatic ring having 6 to 10 carbon atoms. In one embodiment, in each ring of an aryl group, 0, 1, 2, 3, 4, 5, or 6 atoms may be substituted with substituents.

The term "substituted" refers to the replacement of hydrogen atoms in a molecular structure with substituents such that the resulting compound is chemically stable and does not exceed the valences of the designated atom. For example, the expression "group A is substituted with substituent B" means that a hydrogen atom bonded to an atom, such as a carbon atom, constituting the backbone of group A is replaced with substituent B, thereby forming a covalent bond between group A and substituent B.

The term "substituent" is a group bonded to the parent group and may be one or more substituents. When there are plural substituents, each substituent may be identical or different from each other. When both the parent group and the substituent are hydrocarbon groups, the number of carbon atoms in the parent group does not include the number of carbon atoms in the substituent. For example, a butyl group (-C₄H₉) having a methoxy group (-OCH₃) as a substituent is classified as a C1 alkoxy group and a C4 alkyl group.

Unless defined otherwise, all technical and scientific terms used herein have the same meaning as commonly understood by one of ordinary skill in the art to which the present invention belongs. In this specification, unless context requires otherwise, singular forms are intended to include pluralities. Although any methods and materials similar or equivalent to those described herein can be used in the practice or testing of the present invention, suitable methods and materials will be described below. All publications, patent applications, patents, and other references mentioned herein are incorporated by reference. The references cited herein are not admitted as prior art to the subject matter that is being claimed. In the event of any inconsistency, this specification, including the definitions set forth herein, shall control. Furthermore, the materials, methods, and examples are provided for illustrative purposes only and are not intended to limit the present invention.

The present invention provides a pharmaceutical composition for preventing, ameliorating, or treating atopic dermatitis comprising the compound represented by Formula 1, an isomer thereof, or a pharmaceutically acceptable salt thereof as an active ingredient. 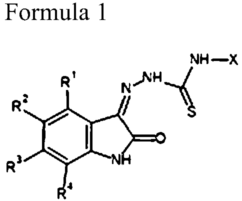
in Formula 1,
X is hydrogen, C₁-C₆ alkyl, -CH₂-C₆₋₁₀ aryl, or 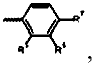 where C₁-C₆ alkyl and -CH₂-C₆₋₁₀ aryl may each independently be unsubstituted or substituted with 1 to 3 halogens,
R¹ is hydrogen or halogen,
R² is hydrogen, halogen, hydroxy, C₁-C₆ alkyl, or C₁-C₆ alkoxy, where C₁-C₆ alkyl and C₁-C₆ alkoxy may each independently be unsubstituted or substituted with 1 to 3 halogens,
R³, R⁴, and R⁵ are each independently hydrogen, halogen, hydroxy, or C₁-C₆ alkoxy, where C₁-C₆ alkoxy may be unsubstituted or substituted with 1 to 3 halogens,
R⁶ is hydrogen, halogen, hydroxy, -NO₂, C₁-C₆ alkyl, or C₁-C₆ alkoxy, where C₁-C₆ alkyl and C₁-C₆ alkoxy may each independently be unsubstituted or substituted with 1 to 3 halogens, and
R⁷ is hydrogen, halogen, hydroxy, -NO₂, -CN, C₁-C₆ alkyl, or C₁-C₆ alkoxy, where C₁-C₆ alkyl and C₁-C₆ alkoxy may each independently be unsubstituted or substituted with 1 to 3 halogens.

In addition, the present invention provides a quasi-drug composition for preventing or ameliorating atopic dermatitis comprising a compound represented by Formula 1, an isomer thereof, or a pharmaceutically acceptable salt thereof as an active ingredient.

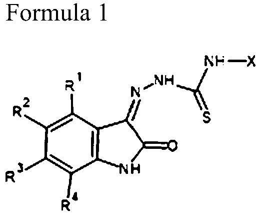

in Formula 1,
X is hydrogen, C₁-C₆ alkyl, -CH₂-C₆₋₁₀ aryl, or 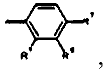 where C₁-C₆ alkyl and -CH₂-C₆₋₁₀ aryl may each independently be unsubstituted or substituted with 1 to 3 halogens,
R¹ is hydrogen or halogen,
R² is hydrogen, halogen, hydroxy, C₁-C₆ alkyl, or C₁-C₆ alkoxy, where C₁-C₆ alkyl and C₁-C₆ alkoxy may each independently be unsubstituted or substituted with 1 to 3 halogens,
R³, R⁴, and R⁵ are each independently hydrogen, halogen, hydroxy, or C₁-C₆ alkoxy, where the alkoxy may be unsubstituted or substituted with 1 to 3 halogens,
R⁶ is hydrogen, halogen, hydroxy, -NO₂, C₁-C₆ alkyl, or C₁-C₆ alkoxy, where C₁-C₆ alkyl and C₁-C₆ alkoxy may each independently be unsubstituted or substituted with 1 to 3 halogens, and
R⁷ is hydrogen, halogen, hydroxy, -NO₂, -CN, C₁-C₆ alkyl, or C₁-C₆ alkoxy, where C₁-C₆ alkyl and C₁-C₆ alkoxy may each independently be unsubstituted or substituted with 1 to 3 halogens.

In one embodiment, in the compound represented by Formula 1, X is hydrogen, methyl, ethyl, benzyl, or 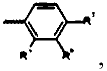 where the methyl and benzyl may each independently be unsubstituted or substituted with 1 to 3 halogens.

In addition, in one embodiment, R¹ of the compound represented by Formula 1 may be hydrogen or halogen. Specifically, R¹ may be H, Br, or Cl, but is not limited thereto.

In one embodiment, in the compound represented by Formula 1, R² is hydrogen, halogen, hydroxy, methyl, or methoxy, where the methyl and methoxy may each independently be unsubstituted or substituted with 1 to 3 halogens. For example, R² may be H, F, Br, CH₃, -OCH₃, or -OCF₃, but is not limited thereto.

In one embodiment, in the compound represented by Formula 1, R³ is hydrogen, halogen, hydroxy, or methoxy, where the methoxy may each independently be unsubstituted or substituted with 1 to 3 halogens. For example, R³ may be H, Br, Cl, or -OCH₃, but is not limited thereto.

In one embodiment, in the compound represented by Formula 1, R⁴ may be hydrogen, halogen, or hydroxy. For example, R⁴ may be H, F, or Br, but is not limited thereto.

In one embodiment, in the compound represented by Formula 1, R⁵ is hydrogen, hydroxy, or methoxy, where the methoxy may each independently be unsubstituted or substituted with 1 to 3 halogens. For example, R⁵ may be H, or -OCH₃, but is not limited thereto.

In one embodiment, in the compound represented by Formula 1, R⁶ is hydrogen, halogen, hydroxy, -NO₂, methyl, or methoxy, where the methyl and methoxy may each independently be unsubstituted or substituted with 1 to 3 halogens. For example, R⁶ may be H, -NO₂, CH₃, -OCH₃, or CF₃, but is not limited thereto.

In one embodiment, in the compound represented by Formula 1, R⁷ is hydrogen, halogen, hydroxy, -NO₂, -CN, methyl, or methoxy, where the methyl and methoxy may each independently be unsubstituted or substituted with 1 to 3 halogens. For example, R⁷ may be H, F, Br, -NO₂, -CN, -OCH₃, or CF₃, but is not limited thereto.

In addition, specific examples of the compound represented by Formula 1 are as follows, but are not limited thereto.
N-(3-(Trifluoromethyl)phenyl)hydrazinethioamide;
N-(4-fluorophenyl)hydrazinethioamide;
N-(4-Bromophenyl)hydrazinethioamide;
N-(4-Cyanophenyl)hydrazinethioamide;
N-(4-(trifluoromethyl)phenyl)hydrazinethioamide;
N-ethylhydrazinethioamide;
N-isopropylhydrazinethioamide;
N-isobutylhydrazinethioamide;
N-(hydrazinocarbonothioyl)benzamide;
N-benzylhydrazinethioamide;
N-(m-tolyl)hydrazinethioamide;
(Z)-2-(5-fluoro-2-oxindolin-3-ylidene)-N-phenylhydrazinethioamide;
(Z)-2-(5-fluoro-2-oxindolin-3-ylidene)-N-(3-nitrophenyl)hydrazinethioamide;
(Z)-2-(5-Fluoro-2-oxindoline-3-ylidene)-N-(4-fluorophenyl)hydrazinecarbothioamide;
(Z)-N-(4-Bromophenyl)-2-(5-fluoro-2-oxindoline-3-ylidene)hydrazinecarbothioamide;
(Z)-N-(4-Cyanophenyl)-2-(5-fluoro-2-oxindoline-3-ylidene)hydrazinecarbothioamide;
(Z)-2-(5-Fluoro-2-oxindoline-3-ylidene)-N-(4-(trifluoromethyl)phenyl)hydrazinecarbothioamide;
(Z)-2-(7-Fluoro-2-oxindoline-3-ylidene)-N-(4-fluorophenyl)hydrazinecarbothioamide;
(Z)-N-(4-Bromophenyl)-2-(7-fluoro-2-oxindoline-3-ylidene)hydrazinecarbothioamide;
(Z)-N-(4-Cyanophenyl)-2-(7-fluoro-2-oxindoline-3-ylidene)hydrazinecarbothioamide;
(Z)-2-(7-Fluoro-2-oxindolin-3-ylidene)-N-(4-(trifluoromethyl)phenyl)hydrazinecarbothioamide;
(Z)-2-(6-Methoxy-2-oxoindolin-3-ylidene)-N-phenylhydrazinecarbothioamide;
(Z)-2-(6-Methoxy-2-oxoindolin-3-ylidene)-N-(3-nitrophenyl)hydrazinecarbothioamide;
(Z)-2-(6-Methoxy-2-oxoindolin-3-ylidene)-N-(2-methoxyphenyl)hydrazinethioamide;
(Z)-N-(4-Cyanophenyl)-2-(6-methoxy-2-oxoindolin-3-ylidene)hydrazinecarbothioamide;
(Z)-2-(6-Methoxy-2-oxoindolin-3-ylidene)-N-(4-(trifluoromethyl)phenyl)hydrazinecarbothioamide;
(Z)-N-Ethyl-2-(6-methoxy-2-oxoindolin-3-ylidene)hydrazinethioamide.
(Z)-2-(6-Methoxy-2-oxoindolin-3-ylidene)-N-(m-tolyl)hydrazinecarbothioamide;
(Z)-2-(5-Methyl-2-oxoindolin-3-ylidene)-N-phenylhydrazinethioamide;
(Z)-2-(5-Methyl-2-oxoindolin-3-ylidene)-N-(3-nitrophenyl)hydrazinecarbothioamide;
(Z)-N-(2-Methoxyphenyl)-2-(5-methyl-2-oxindoline-3-ylidene)hydrazinecarbothioamide;
(Z)-2-(5-Methyl-2-oxoindolin-3-ylidene)-N-(3-(trifluoromethyl)phenyl)hydrazinecarbothioamide;
(Z)-N-(4-Fluorophenyl)-2-(5-methyl-2-oxoindolin-3-ylidene)hydrazinecarbothioamide;
(Z)-2-(5-Bromo-2-oxoindolin-3-ylidene)hydrazinecarbothioamide;
(Z)-2-(4-Bromo-2-oxoindolin-3-ylidene)-N-(3-nitrophenyl)hydrazinecarbothioamide;
(Z)-2-(6-Bromo-2-oxoindolin-3-ylidene)-N-(3-nitrophenyl)hydrazinecarbothioamide;
(Z)-2-(4-Bromo-2-oxoindolin-3-ylidene)-N-(4-nitrophenyl)hydrazinecarbothioamide;
(Z)-2-(5-Bromo-2-oxoindolin-3-ylidene)-N-(4-nitrophenyl)hydrazinecarbothioamide;
(Z)-N-(2-Methoxyphenyl)-2-(2-oxindoline-3-ylidene)hydrazinecarbothioamide;
(Z)-2-(4-Bromo-2-oxoindolin-3-ylidene)hydrazinecarbothioamide;
(Z)-2-(6-Bromo-2-oxoindolin-3-ylidene)hydrazinecarbothioamide;
(Z)-2-(7-Bromo-2-oxoindolin-3-ylidene)hydrazinecarbothioamide;
(Z)-2-(4-Bromo-2-oxoindolin-3-ylidene)-N-phenylhydrazinecarbothioamide;
(Z)-2-(5-Bromo-2-oxoindolin-3-ylidene)-N-phenylhydrazinethioamide;
(Z)-2-(6-Bromo-2-oxoindolin-3-ylidene)-N-phenylhydrazinethioamide;
(Z)-2-(7-Bromo-2-oxoindolin-3-ylidene)-N-phenylhydrazinethioamide;
(Z)-N-(3-Nitrophenyl)-2-(2-oxoindolin-3-ylidene)hydrazinethioamide;
(Z)-2-(5-Bromo-2-oxoindolin-3-ylidene)-N-(3-nitrophenyl)hydrazinethioamide;
(Z)-2-(7-Bromo-2-oxoindolin-3-ylidene)-N-(3-nitrophenyl)hydrazinethioamide;
(Z)-N-(3-Methoxyphenyl)-2-(2-oxoindolin-3-ylidene)hydrazinethioamide;
(Z)-2-(4-Bromo-2-oxoindolin-3-ylidene)-N-(3-methoxyphenyl)hydrazinethioamide;
(Z)-2-(5-Bromo-2-oxoindolin-3-ylidene)-N-(3-methoxyphenyl)hydrazinethioamide;
(Z)-N-(4-Methoxyphenyl)-2-(2-oxoindolin-3-ylidene)hydrazinethioamide;
(Z)-2-(4-Bromo-2-oxoindolin-3-ylidene)-N-(4-methoxyphenyl)hydrazinethioamide;
(Z)-2-(5-Bromo-2-oxoindolin-3-ylidene)-N-(4-methoxyphenyl)hydrazinethioamide;
N-(3,4-Dimethoxyphenyl)hydrazinethioamide;
(Z)-N-(4-Fluorophenyl)-2-(2-oxoindolin-3-ylidene)hydrazinethioamide;
(Z)-2-(6-Bromo-2-oxoindolin-3-ylidene)-N-(4-nitrophenyl)hydrazinethioamide;
(Z)-2-(4-Chloro-2-oxoindolin-3-ylidene)-N-phenylhydrazinethioamide;
(Z)-2-(6-Chloro-2-oxoindolin-3-ylidene)-N-phenylhydrazinethioamide;
(Z)-2-(4-Chloro-2-oxoindolin-3-ylidene)-N-(2-methoxyphenyl)hydrazinethioamide;
(Z)-2-(5-Fluoro-2-oxoindolin-3-ylidene)-N-(3-(trifluoromethyl)phenyl)hydrazinethioamide;
(Z)-N-Benzyl-2-(5-fluoro-2-oxoindolin-3-ylidene)hydrazinethioamide;
(Z)-2-(5-Fluoro-2-oxoindolin-3-ylidene)-N-(m-tolyl)hydrazinethioamide;
(Z)-2-(7-Fluoro-2-oxoindolin-3-ylidene)-N-(4-nitrophenyl)hydrazinecarbothioamide;
(Z)-2-(6-Methoxy-2-oxoindolin-3-ylidene)-N-(3-(trifluoromethyl)phenyl)hydrazinecarbothioamide;
(Z)-N-(4-Fluorophenyl)-2-(6-methoxy-2-oxoindolin-3-ylidene)hydrazinethioamide;
(Z)-N-(4-Bromophenyl)-2-(6-methoxy-2-oxoindolin-3-ylidene)hydrazinethioamide;
(Z)-N-Benzyl-2-(6-methoxy-2-oxoindolin-3-ylidene)hydrazinethioamide;
(Z)-N-(4-Bromophenyl)-2-(5-methyl-2-oxindoline-3-ylidene)hydrazinecarbothioamide;
(Z)-N-(4-Cyanophenyl)-2-(5-methyl-2-oxoindolin-3-ylidene)hydrazinecarbothioamide;
(Z)-2-(5-Methyl-2-oxoindolin-3-ylidene)-N-phenylhydrazinethioamide;
(Z)-N-ethyl-2-(5-methyl-2-oxoindolin-3-ylidene)hydrazinethioamide;
(Z)-N-Benzyl-2-(5-methyl-2-oxoindolin-3-ylidene)hydrazinecarbothioamide;
(Z)-2-(5-Methyl-2-oxoindolin-3-ylidene)-N-(m-tolyl)hydrazinecarbothioamide;
(Z)-2-(2-oxo-5-(trifluoromethoxy)indolin-3-ylidene)-N-phenylhydrazinethioamide;
(Z)-N-(3-Nitrophenyl)-2-(2-oxo-5-(trifluoromethoxy)indolin-3-ylidene)hydrazinecarbothioamide;
(Z)-N-(4-Fluorophenyl)-2-(2-oxo-5-(trifluoromethoxy)indolin-3-ylidene)hydrazinecarbothioamide; and
(Z)-N-(4-Bromophenyl)-2-(2-oxo-5-(trifluoromethoxy)indolin-3-ylidene)hydrazinecarbothioamide.

The present invention includes the "pharmaceutically acceptable salts" of the above-mentioned compounds.

The pharmaceutically acceptable salt should have low toxicity to humans and not adversely affect the biological activity or physicochemical properties of the parent compound. For example, the pharmaceutically acceptable salt may be an acid addition salt formed with a pharmaceutically acceptable free acid.

The free acid may be an inorganic or organic acid, where the inorganic acid may be hydrochloric acid, sulfuric acid, nitric acid, phosphoric acid, perchloric acid, bromic acid (HBrO3), and the like, and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, and the like.

The acid addition salt can be prepared by conventional methods such as dissolving the compound represented by Formula 1 in an aqueous solution of excess acid and precipitating the salt with a water-immiscible organic solvent such as methanol, ethanol, acetone, or acetonitrile.

In addition, the pharmaceutically acceptable salt may be an alkali metal salt (e.g., sodium salt) or an alkaline earth metal salt (e.g., potassium salt).

The alkali metal salt or the alkaline earth metal salt may be obtained by dissolving the compound represented by Formula 1 in an excess amount of an aqueous solution of an alkali metal hydroxide or an alkaline earth metal hydroxide and filtering out undissolved compound salts to obtain a filtrate, followed by evaporating and drying the filtrate.

In addition, the compounds of the present invention may have chiral carbon centers and therefore exist in the form of R or S isomers, racemic compounds, individual enantiomers or mixtures, individual diastereomers or mixtures, all stereoisomeric forms and their mixtures being within the scope of the present invention.

In addition, the compounds of the present invention may include hydrates and solvates of the compound represented by Formula 1. The hydrate and solvate can be prepared using a known method, and preferably are non-toxic and water-soluble. In particular, it is preferable that the hydrate and solvate each have 1 to 5 molecules of water or an alcoholic solvent (especially ethanol).

Since the compound, the isomer thereof, or the pharmaceutically acceptable salt according to the embodiment of the present invention has excellent inhibitory effect on the activity of EGR-1, the compound or a pharmaceutical composition containing the same may be usefully used for treating atopic dermatitis which is caused by and aggravated by increased inflammatory cytokines due to EGR-1.

Specifically, the compound or pharmaceutical composition thereof according to the present invention is a material targeting transcription factor EGR-1 involved in immune system such as differentiation of immune cells and production of cytokines, which inhibits binding of EGR-1 to DNA, thereby reducing production and secretion of inflammatory cytokines and thus ameliorating skin inflammation and pruritus resulting from a hypersensitive immune response.

The compounds can be usefully used as an atopic dermatitis treatment agent by replacing the conventional steroid agents to prevent immune suppression and other side effects caused by the conventional steroid-based treatments.

The term "atopic dermatitis" as used herein may be used interchangeably with the terms "atopy", "atopic disease", etc., and refers to a chronic allergic inflammatory skin disorder characterized by symptoms such as dry skin, scaly skin, inflammation of the skin, increased skin permeability, susceptibility to superficial infections on the skin, relapses, and pruritus.

The term "prevention" as used herein refers to any action which inhibits or delays the onset, spread, and recurrence of a disease by administering the compound or pharmaceutical composition according to the present invention; the term "amelioration" refers to any action that reduces at least one parameter associated with the alleviation or treatment of a disease state, for example the severity of symptoms, by administering the compound or pharmaceutical composition according to the present invention; and the term "treatment" refers to any action that ameliorates or otherwise beneficially alters the condition of said disease by administering the compound or pharmaceutical composition according to the present invention, including inhibiting, relieving, or curing the disease.

The present invention provides the use of a compound targeting EGR-1, an isomer thereof, or a pharmaceutically acceptable salt thereof for preventing, ameliorating, or treating atopic dermatitis.

The present invention provides the use of a compound targeting EGR-1, an isomer thereof, or a pharmaceutically acceptable salt thereof for inhibiting the activity of transcription factor EGR-1.

The present invention provides the use of a compound targeting EGR-1, an isomer thereof, or a pharmaceutically acceptable salt thereof for the manufacture of a medicament for preventing, ameliorating, or treating atopic dermatitis.

The present invention provides the use of a compound targeting EGR-1, an isomer thereof, or a pharmaceutically acceptable salt thereof for the manufacture of a medicament for inhibiting the activity of EGR-1.

The present invention also provides a method of preventing, ameliorating, or treating atopic dermatitis comprising administering to a subject in need thereof a compound targeting EGR-1, an isomer thereof, or a pharmaceutically acceptable salt thereof.

The present invention also provides a method of inhibiting the activity of EGR-1 comprising administering to an individual in need thereof a compound targeting EGR-1, an isomer thereof, or a pharmaceutically acceptable salt thereof.

The pharmaceutical composition of the present invention can inhibit the activity of EGR-1 and production of cytokines accordingly. As used herein, the term "inhibition" refers to inhibition of any step involved in gene transcription, mRNA processing, translation, translocation, or maturation, or inhibition of protein-protein interactions, protein activation, or signal transduction mediated thereby.

The pharmaceutical composition of the present invention may include a pharmaceutically acceptable carrier in addition to an active ingredient. The pharmaceutically acceptable carrier includes those conventionally used for formulation, such as lactose, dextrose, sucrose, sorbitol, mannitol, starch, gum arabic, calcium phosphate, alginate, gelatin, calcium silicate, microcrystalline cellulose, polyvinylpyrrolidone, cellulose, water, syrup, methylcellulose, methylhydroxybenzoate, propyl hydroxybenzoate, talc, magnesium stearate, and mineral oil, but is not limited thereto. In addition, the composition may further comprise lubricants, humectants, sweeteners, flavors, emulsifiers, suspending agents, preservatives, etc., in addition to the above components.

The pharmaceutical composition of the present invention may be administered orally or parenterally according to a desired method, and the parenteral administration may include intravenous injection, subcutaneous injection, intraperitoneal injection, intramuscular injection, or topical application such as dermal application. The dosage varies depending on the patient's condition and weight, severity of the disease, drug form, route and timing of administration, but can be appropriately selected by those skilled in the art.

The pharmaceutical composition of the present invention is administered in a pharmaceutically effective amount. As used herein, the term "pharmaceutically effective amount" refers to an amount sufficient to treat disease with a reasonable benefit/risk ratio applicable for medical treatment, and the effective dosage level can be determined according to various factors including the type, severity and extent of the disease being treated, the physical conditions of the patient, the activity of the drug, sensitivity of the patient to the drug, administration time, route of administration and rate of excretion, duration of treatment, drugs used simultaneously, and other factors well known in the art.

The pharmaceutical composition according to the present invention may be administered as a single agent or in combination with other therapeutic agents and sequentially or simultaneously with conventional therapeutic agents, and can be administered once or multiple times. It is important that an amount of the active ingredient which provides maximum effect at minimum dose without side effects should be determined by those skilled in the art.

The present invention also provides a method for ameliorating, controlling, or treating atopic dermatitis comprising administering the pharmaceutical composition to a subject in need thereof. As used herein, the term "subject" refers to an individual that needs treatment of a disease and more specifically refers to a mammal such as human or non-human primate, mouse, dog, cat, horse, and cow.

In one embodiment, the pharmaceutical composition according to the present invention may be a topical skin composition. The topical skin composition may be a cream, gel, ointment, emulsion for external use on the skin, suspension for external use on the skin, transdermal patch, lotion, or combinations thereof. The topical skin composition may include components conventionally used in cosmetics and pharmaceuticals such as an aqueous component, oily component, powder component, alcohol, moisturizer, thickener, ultraviolet absorber, whitening agent, preservative, antioxidant, surfactant, fragrance, colorant, various skin nutrients, or combinations thereof, as appropriate. The topical skin composition may also contain metal-sequestering agents such as edetate disodium, edetate trisodium, sodium citrate, sodium polyphosphate, sodium metaphosphate, gluconic acid, etc., drugs such as caffeine, tannin, verapamil, glycyrrhiza extract, glabridin, fruit of Calycanthus spinosa L., various herbal medicines, tocopheryl acetate, glycyrrhizic acid, tranexamic acid and its derivatives or salts, vitamins such as vitamin C, magnesium ascorbyl phosphate, ascorbic acid glucoside, arbutin, kojic acid, sugars such as glucose, fructose, trehalose, etc., as appropriate.

The skin includes all areas of the skin on the body including face, hands, arms, legs, feet, chest, abdomen, back, buttocks, and scalp.

The term "quasi-drug" as used herein refers to products that are intended for use in diagnosing, treating, mitigating, or preventing diseases of humans and animals but have a less significant effect than drugs. For example, according to the Pharmaceutical Affairs Act, the quasi-drug refers to products excluding those used as drugs, including fiber and rubber products used for treatment or prevention of diseases in humans and animals, products that have mild or no direct effect on the human body and are not instruments or machines, and disinfectants and insecticides for preventing infectious diseases.

The type or formulation of the quasi-drug composition of the present invention is not particularly limited and may be a bandage, gauze, cotton wool, adhesive plaster, disinfectant cleanser, shower foam, mouthwash, wet tissue, detergent soap, hand wash, humidifier filler, mask, filter filler, etc.

The quasi-drug composition according to the present invention may have an effect of preventing or ameliorating atopic dermatitis, but is not limited thereto.

The quasi-drug composition of the present invention may further include a pharmaceutically acceptable carrier, excipient or diluent as necessary in addition to the above-mentioned components. The pharmaceutically acceptable carrier, excipient or diluent is not limited so long as it does not affect the effect of the present invention and may include, for example, fillers, extenders, binders, wetting agents, disintegrating agents, surfactants, lubricants, sweeteners, fragrances, preservatives, etc.

When the composition of the present invention is included in a quasi-drug for preventing or ameliorating atopic dermatitis, it may be used as it is or together with other quasi-drug ingredients according to conventional methods. The amount of active ingredient(s) can be appropriately determined depending on its purpose.

The present invention provides a method for preparing the above compounds. Specifically, the compound represented by Formula 1 can be prepared by the methods shown in the following reaction schemes, but is not limited thereto. In particular, those skilled in the art will fully understand that the compound represented by Formula 1 of the present invention may be prepared by various methods using known techniques well-known in the art.

The following reaction schemes show a method of preparing representative compounds according to the present invention in a step-by-step manner, and various compounds of the present invention may be prepared by changing the reagents and solvents used in the following preparation steps or by changing the reaction order.

### <General procedure>

In the present invention, the compound represented by Formula 1 may be prepared by a method as shown in Reaction Scheme 1. The compound may be synthesized by reacting various isothiocyanates having chain or cyclic substituents with hydrazine monohydrate to obtain hydrazinethioamides (A and B) (step 1), followed by reaction of the hydrazinethioamide (A and B) with isatin (C) to give a compound represented by Formula D (step 2).

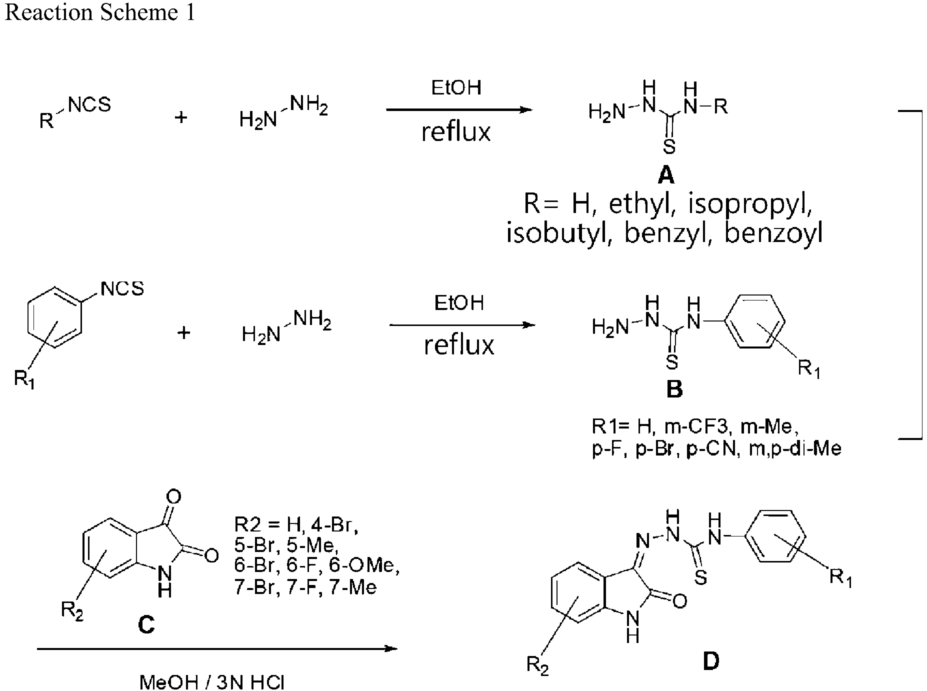

The target compound prepared in Reaction Scheme 1 may be isolated and purified by a conventional method such as column chromatography or recrystallization.

In another aspect, the present invention provides, as a novel compound that inhibits the activity of EGR-1, any one selected from the group consisting of the following compounds, an isomer thereof, or a pharmaceutically acceptable salt thereof:
(Z)-N-(4-Cyanophenyl)-2-(7-fluoro-2-oxindoline-3-ylidene)hydrazinecarbothioamide;
(Z)-2-(7-Fluoro-2-oxoindolin-3-ylidene)-N-(4-(trifluoromethyl)phenyl)hydrazinecarbothioamide;
(Z)-2-(6-Methoxy-2-oxoindolin-3-ylidene)-N-phenylhydrazinethioamide;
(Z)-2-(6-Methoxy-2-oxoindolin-3-ylidene)-N-(3-nitrophenyl)hydrazinethioamide;
(Z)-2-(6-Methoxy-2-oxoindolin-3-ylidene)-N-(2-methoxyphenyl)hydrazinethioamide;
(Z)-N-(4-Cyanophenyl)-2-(6-methoxy-2-oxoindolin-3-ylidene)hydrazinecarbothioamide;
(Z)-2-(6-Methoxy-2-oxoindolin-3-ylidene)-N-(4-(trifluoromethyl)phenyl)hydrazinecarbothioamide;
(Z)-N-Ethyl-2-(6-methoxy-2-oxoindolin-3-ylidene)hydrazinecarbothioamide;
(Z)-2-(5-Methyl-2-oxoindolin-3-ylidene)-N-(3-nitrophenyl)hydrazinecarbothioamide;
(Z)-2-(4-Bromo-2-oxoindolin-3-ylidene)-N-(3-nitrophenyl)hydrazinecarbothioamide;
(Z)-2-(6-Bromo-2-oxoindolin-3-ylidene)-N-(3-nitrophenyl)hydrazinethioamide;
(Z)-2-(4-Bromo-2-oxoindolin-3-ylidene)-N-(4-nitrophenyl)hydrazinecarbothioamide;
(Z)-2-(4-Bromo-2-oxoindolin-3-ylidene)-N-phenylhydrazinethioamide;
(Z)-2-(6-Bromo-2-oxoindolin-3-ylidene)-N-phenylhydrazinethioamide;
(Z)-2-(7-Bromo-2-oxoindolin-3-ylidene)-N-(3-nitrophenyl)hydrazinecarbothioamide;
(Z)-2-(4-Bromo-2-oxoindolin-3-ylidene)-N-(3-methoxyphenyl)hydrazinecarbothioamide;
(Z)-2-(4-Bromo-2-oxoindolin-3-ylidene)-N-(4-methoxyphenyl)hydrazinecarbothioamide;
(Z)-2-(6-Bromo-2-oxoindolin-3-ylidene)-N-(4-nitrophenyl)hydrazinecarbothioamide;
(Z)-2-(4-Chloro-2-oxoindolin-3-ylidene)-N-(2-methoxyphenyl)hydrazinecarbothioamide;
(Z)-2-(7-Fluoro-2-oxoindolin-3-ylidene)-N-(4-nitrophenyl)hydrazinecarbothioamide;
(Z)-2-(6-Methoxy-2-oxoindolin-3-ylidene)-N-(3-(trifluoromethyl)phenyl)hydrazinecarbothioamide;
(Z)-N-(4-Fluorophenyl)-2-(6-methoxy-2-oxoindolin-3-ylidene)hydrazine-1-carbothioamide;
(Z)-N-(4-Bromophenyl)-2-(6-methoxy-2-oxoindolin-3-ylidene)hydrazine-1-carbothioamide;
(Z)-N-Benzyl-2-(6-methoxy-2-oxoindolin-3-ylidene)hydrazine-1-carbothioamide;
(Z)-N-(4-Cyanophenyl)-2-(5-methyl-2-oxoindolin-3-ylidene)hydrazinecarbothioamide; and
(Z)-2-(5-Methyl-2-oxoindolin-3-ylidene)-N-(m-tolyl)hydrazinecarbothioamide.

In the novel compounds inhibiting the activity of EGR-1 according to the present invention, an isomer thereof or a pharmaceutically acceptable salt thereof are as described above.

The present invention also provides any one selected from the group consisting of the following compounds, an isomer thereof, or a pharmaceutically acceptable salt:
(Z)-N-(4-Cyanophenyl)-2-(7-fluoro-2-oxoindolin-3-ylidene)hydrazinecarbothioamide;
(Z)-2-(7-Fluoro-2-oxoindolin-3-ylidene)-N-(4-(trifluoromethyl)phenyl)hydrazinecarbothioamide;
(Z)-2-(6-Methoxy-2-oxoindolin-3-ylidene)-N-phenylhydrazinethioamide
(Z)-2-(6-Methoxy-2-oxoindolin-3-ylidene)-N-(3-nitrophenyl)hydrazinethioamide;
(Z)-2-(6-Methoxy-2-oxoindolin-3-ylidene)-N-(2-methoxyphenyl)hydrazinethioamide;
(Z)-N-(4-Cyanophenyl)-2-(6-methoxy-2-oxoindolin-3-ylidene)hydrazinecarbothioamide;
(Z)-2-(6-Methoxy-2-oxoindolin-3-ylidene)-N-(4-(trifluoromethyl)phenyl)hydrazinethioamide;
(Z)-N-ethyl-2-(6-methoxy-2-oxoindolin-3-ylidene)hydrazinethioamide;
(Z)-2-(5-Methyl-2-oxoindolin-3-ylidene)-N-(3-nitrophenyl)hydrazinethioamide;
(Z)-2-(4-Bromo-2-oxoindolin-3-ylidene)-N-(3-nitrophenyl)hydrazinethioamide;
(Z)-2-(6-Bromo-2-oxoindolin-3-ylidene)-N-(3-nitrophenyl)hydrazinethioamide;
(Z)-2-(4-Bromo-2-oxoindolin-3-ylidene)-N-(4-nitrophenyl)hydrazinethioamide;
(Z)-2-(4-Bromo-2-oxoindolin-3-ylidene)-N-phenylhydrazinethioamide;
(Z)-2-(6-Bromo-2-oxoindolin-3-ylidene)-N-phenylhydrazinethioamide;
(Z)-2-(7-Bromo-2-oxoindolin-3-ylidene)-N-(3-nitrophenyl)hydrazinethioamide;
(Z)-2-(4-Bromo-2-oxoindolin-3-ylidene)-N-(3-methoxyphenyl)hydrazinethioamide;
(Z)-2-(4-Bromo-2-oxoindolin-3-ylidene)-N-(4-methoxyphenyl)hydrazinecarbothioamide;
(Z)-2-(6-Bromo-2-oxoindolin-3-ylidene)-N-(4-nitrophenyl)hydrazinecarbothioamide;
(Z)-2-(4-Chloro-2-oxoindolin-3-ylidene)-N-(2-methoxyphenyl)hydrazinecarbothioamide;
(Z)-2-(7-Fluoro-2-oxoindolin-3-ylidene)-N-(4-nitrophenyl)hydrazinecarbothioamide;
(Z)-2-(6-Methoxy-2-oxoindolin-3-ylidene)-N-(3-(trifluoromethyl)phenyl)hydrazinecarbothioamide;
(Z)-N-(4-Fluorophenyl)-2-(6-methoxy-2-oxoindolin-3-ylidene)hydrazine-1-carbothioamide;
(Z)-N-(4-Bromophenyl)-2-(6-methoxy-2-oxoindolin-3-ylidene)hydrazine-1-carbothioamide;
(Z)-N-Benzyl-2-(6-methoxy-2-oxoindolin-3-ylidene)hydrazine-1-carbothioamide;
(Z)-N-(4-Cyanophenyl)-2-(5-methyl-2-oxoindolin-3-ylidene)hydrazinecarbothioamide; and
(Z)-2-(5-Methyl-2-oxoindolin-3-ylidene)-N-(m-tolyl)hydrazinecarbothioamide.

Hereinafter, the present invention will be described in more detail with reference to examples and experimental examples. However, the following examples and experimental examples are merely illustrative of the present invention and do not limit the scope of the present invention.

Accordingly, the method for preparing the compound represented by Formula 1 is described by using the synthesis process of N-benzylhydrazinethioamide (Compound 10), which is one of compounds of Group VI, and (Z)-N-(4-cyanophenyl)-2-(6-methoxy-2-oxoindolin-3-ylidene)hydrazinethioamide (Compound 25), which is one of compounds of Group VII.

### <Examples>

### Example 1. Synthesis of N-benzylhydrazinethioamide (Compound 10)

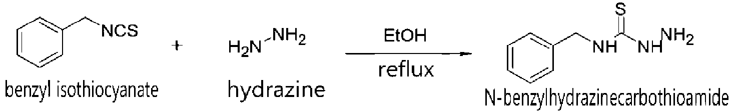

Benzyl isothiocyanate (1.49 g, 10 mmol) was added to a round-bottom flask and dissolved in 40 mL of ethyl alcohol. Hydrazine monohydrate (0.6 g, 12 mmol) was then added to the solution at room temperature, followed by heating and stirring the reaction mixture at 90 °C for 8 hours. The completion of the reaction was confirmed through thin layer chromatography, after which the reaction mixture was cooled down and the solvent was removed under reduced pressure to obtain a solid product. This compound was recrystallized to give a title compound (1.37 g, 76 %).

¹H NMR (700 MHz, dmso) δ 8.74 (s, 1H), 8.30 (s, 1H), 7.35 - 7.26 (m, 4H), 7.25 - 7.17 (m, 1H), 4.71 (d, J = 6.0 Hz, 2H), 4.51 (s, 2H). ¹³C NMR (175 MHz, dmso) δ 181.57, 139.78, 128.05, 127.30, 126.61, 46.16.

ESI-MS. Calcd for C₈H₁₁N₃S (M⁺ +H): m/z 182.0674. Found: m/z 182.0767.

### Example 2. Synthesis of (Z)-N-(4-Cyanophenyl)-2-(6-methoxy-2-oxoindolin-3-ylidene)hydrazinethioamide (Compound 25)

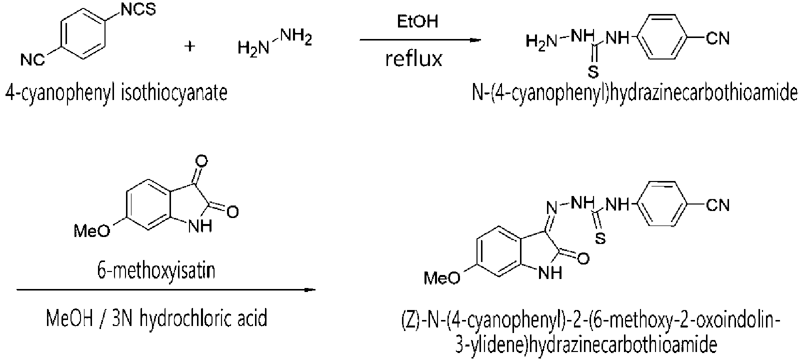

The reaction of 4-cyanophenyl isothiocyanate and hydrazine monohydrate according to the method of Example 1 gives a synthetic intermediate N-(4-cyanophenyl)hydrazinethioamide. N-(4-cyanophenyl)hydrazinethioamide (135 mg, 0.7 mmol) was added to a round-bottom flask and dissolved in 15 mL of ethyl alcohol. 6-methoxyisatin (89 mg, 0.5 mmol) was added to the solution and dissolved, followed by adding 2 mL of 3N hydrochloric acid solution and stirring it at room temperature for 5 hours. As the reaction proceeded, an orange solid was formed, and after completion of the reaction, filtration under reduced pressure gave a title compound (138 mg, 79%).

¹H NMR (700 MHz, dmso) δ 12.83 (s, 1H), 11.26 (s, 1H), 10.90 (s, 1H), 7.97 (t, J = 9.6 Hz, 2H), 7.93 - 7.82 (m, 2H), 7.68 (d, J = 8.4 Hz, 1H), 6.79 - 6.63 (m, 1H), 6.47 (dd, J = 32.7, 2.2 Hz, 1H), 3.82 (d, J = 10.5 Hz, 3H). ¹³C NMR (175 MHz, dmso) δ 175.80, 163.50, 162.86, 144.72, 142.99, 133.14, 132.64, 125.09, 123.29, 118.89, 112.15, 108.42, 107.62, 97.70, 55.76.

ESI-MS. Calcd for C₁₇H₁₃N₅O₂S (M⁺ +H): m/z 352.0790. Found: m/z 352.0877.

### Example 3. Synthesis of N-(3-(trifluoromethyl)phenyl)hydrazinecarbothioamide (Compound 1)

The title compound was synthesized according to the synthetic method of Example 1 using 3-(trifluoromethyl)phenyl isothiocyanate and hydrazine as starting materials.

¹H NMR (700 MHz, dmso) δ 9.36 (s, 1H), 8.25 (s, 1H), 7.87 (m, 2H), 7.51 (m, 1H), 7.42 (m, 1H), 4.90 (s, 2H).

### Example 4. Synthesis of N-(4-fluorophenyl)hydrazinecarbothioamide (Compound 2)

The title compound was synthesized according to the synthetic method of Example 1 using 4-fluorophenyl isothiocyanate and hydrazine as starting materials.

¹H NMR (700 MHz, dmso) δ 9.69 (s, 1H), 9.14 (s, 1H), 7.60 (s, 2H), 7.12 (m, 2H), 4.76 (s, 2H).

### Example 5. Synthesis of N-(4-bromophenyl)hydrazinecarbothioamide (Compound 3)

The title compound was synthesized according to the synthetic method of Example 1 using 4-bromophenyl isothiocyanate and hydrazine as starting materials.

¹H NMR (700 MHz, dmso) δ 9.70 (s, 1H), 9.24 (s, 1H), 7.65 (d, J = 7.5 Hz, 2H), 7.46 (d, J = 7.5 Hz, 2H), 4.80 (s, 2H).

### Example 6. Synthesis of N-(4-cyanophenyl)hydrazinethioamide (Compound 4)

The title compound was synthesized according to the synthetic method of Example 1 using 4-cyanophenyl isothiocyanate and hydrazine as starting materials.

¹H NMR (700 MHz, dmso) δ 9.50 (s, 1H), 8.05 (d, J = 7.9 Hz, 2H), 7.78 (d, J = 7.9 Hz, 2H), 7.64 (s, 1H), 5.09 (s, 2H).

### Example 7. Synthesis of N-(4-(trifluoromethyl)phenyl)hydrazinethioamide (Compound 5)

The title compound was synthesized according to the synthetic method of Example 1 using 4-(trifluoromethyl)phenyl isothiocyanate and hydrazine as starting materials.

¹H NMR (700 MHz, dmso) δ 9.40 (s, 1H), 7.98 (s, 2H), 7.70 - 7.58 (m, 3H), 4.93 (s, 2H).

### Example 8. Synthesis of N-ethylhydrazinethioamide (Compound 6)

The title compound was synthesized according to the synthetic method of Example 1 using ethyl isothiocyanate and hydrazine as starting materials.

¹H NMR (700 MHz, dmso) δ 8.49 (s, 1H), 7.81 (s, 1H), 4.42 (s, 2H), 3.45 (q, J = 7.1 Hz, 2H), 1.06 (t, J = 7.1 Hz, 3H).

### Example 9. Synthesis of N-isopropylhydrazinethioamide (Compound 7)

The title compound was synthesized according to the synthetic method of Example 1 using isopropylisothiocyanate and hydrazine as starting materials.

¹H NMR (700 MHz, dmso) δ 8.52 (s, 1H), 7.48 (s, 1H), 4.42 (s, 2H), 4.35 (m, J = 6.6 Hz, 1H), 1.12 (d, J = 6.6 Hz, 6H).

### Example 10. Synthesis of N-isobutylhydrazinethioamide (Compound 8)

The title compound was synthesized according to the synthetic method of Example 1 using isobutyl isothiocyanate and hydrazine as starting materials.

¹H NMR (700 MHz, dmso) δ 8.56 (s, 1H), 7.80 (s, 1H), 4.46 (s, 2H), 3.27 (m, 2H), 1.92 - 1.80 (m, 1H), 0.85 (d, J = 6.7 Hz, 6H).

### Example 11. Synthesis of N-(hydrazinecarbonothioyl)benzamide (Compound 9)

The title compound was synthesized according to the synthetic method of Example 1 using benzoyl isothiocyanate and hydrazine as starting materials.

¹H NMR (700 MHz, dmso) δ 12.75 (s, 1H), 12.10 - 9.22 (m, 3H), 8.12 (d, J = 7.4 Hz, 2H), 7.67 (t, J = 7.4 Hz, 1H), 7.57 (t, J = 7.4 Hz, 2H).

### Example 12. Synthesis of N-(m-tolyl)hydrazinecarbothioamide (Compound 11)

The title compound was synthesized according to the synthetic method of Example 1 using m-tolylisothiocyanate and hydrazine as starting materials.

¹H NMR (700 MHz, dmso) δ 9.62 (s, 1H), 9.10 (s, 1H), 7.45 (s, 2H), 7.21 (m, 1H), 6.92 (m, 1H), 4.77 (s, 2H), 2.28 (s, 3H).

### Example 13. Synthesis of (Z)-2-(5-Fluoro-2-oxoindolin-3-ylidene)-N-phenylhydrazinethioamide (Compound 12)

### According to the synthetic method of Example 2, the title compound was synthesized using phenylhydrazinethioamide and 5-fluoroisatin as starting materials.

¹H NMR (700 MHz, dmso) δ 12.67 (s, 1H), 11.26 (s, 1H), 10.86 (s, 1H), 7.64 (dd, J = 8.0, 2.6 Hz, 1H), 7.60 (d, J = 7.5 Hz, 2H), 7.44 (t, J = 8.0 Hz, 2H), 7.29 (t, J = 7.5Hz, 1H), 7.21 (m, 1H), 6.94 (dd, J = 8.6, 4.1 Hz, 1H).

### Example 14. Synthesis of (Z)-2-(5-Fluoro-2-oxoindolin-3-ylidene)-N-(3-nitrophenyl)hydrazinethioamide (Compound 13)

According to the synthetic method of Example 2, the title compound was synthesized using N-(3-nitrophenyl)hydrazinethioamide and 5-fluoroisatin as starting materials.

¹H NMR (700 MHz, dmso) δ 12.83 (s, 1H), 11.31 (s, 1H), 11.10 (s, 1H), 8.65 (t, J = 2.2 Hz, 1H), 8.20 - 8.15 (m, 1H), 8.13 (ddd, J = 8.3, 2.2, 0.9 Hz, 1H), 7.73 (t, J = 8.3 Hz, 1H), 7.61 (dd, J = 8.0, 2.5 Hz, 1H), 7.23 (td, J = 9.0, 2.7 Hz, 1H), 6.95 (dd, J = 8.8, 4.1 Hz, 1H).

### Example 15. Synthesis of (Z)-2-(5-Fluoro-2-oxoindolin-3-ylidene)-N-(4-fluorophenyl)hydrazinethioamide (Compound 14)

According to the synthetic method of Example 2, the title compound was synthesized using N-(4-fluorophenyl)hydrazinethioamide and 5-fluoroisatin as starting materials.

¹H NMR (700 MHz, dmso) δ 12.68 (s, 1H), 11.27 (s, 1H), 10.86 (s, 1H), 7.74 - 7.50 (m, 3H), 7.28 (t, J = 9.0 Hz, 2H), 7.21 (td, J = 9.0, 2.7 Hz, 1H), 6.94 (dd, J = 8.6, 4.1 Hz, 1H).

### Example 16. Synthesis of (Z)-N-(4-bromophenyl)-2-(5-fluoro-2-oxindoline-3-ylidene)hydrazinecarbothioamide (Compound 15)

According to the synthetic method of Example 2, the title compound was synthesized using N-(4-bromophenyl)hydrazinethioamide and 5-fluoroisatin as starting materials.

¹H NMR (700 MHz, dmso) δ 12.72 (s, 1H), 11.28 (s, 1H), 10.87 (s, 1H), 7.70 - 7.54 (m, 5H), 7.22 (td, J = 9.2, 2.7 Hz, 1H), 6.94 (dd, J = 8.6, 4.1 Hz, 1H).

### Example 17. Synthesis of (Z)-N-(4-Cyanophenyl)-2-(5-fluoro-2-oxindoline-3-ylidene)hydrazinecarbothioamide (Compound 16)

According to the synthetic method of Example 2, the title compound was synthesized using N-(4-cyanophenyl)hydrazinethioamide and 5-fluoroisatin as starting materials.

¹H NMR (700 MHz, dmso) δ 12.83 (s, 1H), 11.30 (s, 1H), 11.01 (s, 1H), 7.97 (d, J = 8.7 Hz, 2H), 7.91 (d, J = 8.7 Hz, 2H), 7.62 (dd, J = 8.0, 2.6 Hz, 1H), 7.23 (td, J = 9.4, 2.7 Hz, 1H), 6.95 (dd, J = 8.6, 4.1 Hz, 1H).

### Example 18. Synthesis of (Z)-2-(5-Fluoro-2-oxindoline-3-ylidene)-N-(4-(trifluoromethyl)phenyl)hydrazinecarbothioamide (Compound 17)

According to the synthetic method of Example 2, the title compound was synthesized using N-(4-(trifluoromethyl)phenyl)hydrazinethioamide and 5-fluoroisatin as starting materials.

¹H NMR (700 MHz, dmso) δ 12.80 (s, 1H), 11.30 (s, 1H), 11.02 (s, 1H), 7.93 (d, J = 8.4 Hz, 2H), 7.81 (d, J = 8.5 Hz, 2H), 7.63 (dd, J = 8.6, 2.6 Hz, 1H), 7.23 (m, 1H), 6.95 (dd, J = 8.6, 4.1 Hz, 1H).

### Example 19. Synthesis of (Z)-2-(7-Fluoro-2-oxoindolin-3-ylidene)-N-(4-fluorophenyl)hydrazinecarbothioamide (Compound 18)

According to the synthetic method of Example 2, the title compound was synthesized using N-(4-fluorophenyl)hydrazinethioamide and 7-fluoroisatin as starting materials.

¹H NMR (700 MHz, dmso) δ 12.77 (s, 1H), 11.80 (s, 1H), 10.90 (s, 1H), 7.64 - 7.56 (m, 3H), 7.33 - 7.24 (m, 3H), 7.13 (m, 1H).

### Example 20. Synthesis of (Z)-N-(4-bromophenyl)-2-(7-fluoro-2-oxoindolin-3-ylidene)hydrazinecarbothioamide (Compound 19)

According to the synthetic method of Example 2, the title compound was synthesized using N-(4-bromophenyl)hydrazinethioamide and 7-fluoroisatin as starting materials.

¹H NMR (700 MHz, dmso) δ 12.80 (s, 1H), 11.81 (s, 1H), 10.92 (s, 1H), 7.61 (m, 5H), 7.30 (m, 1H), 7.13 (m, 1H).

### Example 21. Synthesis of (Z)-N-(4-Cyanophenyl)-2-(7-fluoro-2-oxindoline-3-ylidene)hydrazinecarbothioamide (Compound 20)

According to the synthetic method of Example 2, the title compound was synthesized using N-(4-cyanophenyl)hydrazinethioamide and 7-fluoroisatin as starting materials.

¹H NMR (700 MHz, dmso) δ 12.91 (s, 1H), 11.83 (s, 1H), 11.07 (s, 1H), 7.98 - 7.94 (m, 2H), 7.92 - 7.88 (m, 2H), 7.62 (m, 1H), 7.32 (m, 1H), 7.14 (m, 1H).

### Example 22. Synthesis of (Z)-2-(7-Fluoro-2-oxoindolin-3-ylidene)-N-(4-(trifluoromethyl)phenyl)hydrazinecarbothioamide (Compound 21)

According to the synthetic method of Example 2, the title compound was synthesized using N-(4-(trifluoromethyl)phenyl)hydrazinethioamide and 7-fluoroisatin as starting materials.

¹H NMR (700 MHz, dmso) δ 12.88 (s, 1H), 11.83 (s, 1H), 11.07 (s, 1H), 7.92 (d, J = 8.4 Hz, 2H), 7.80 (d, J = 8.5 Hz, 2H), 7.63 (d, J = 7.5 Hz, 1H), 7.31 (m, 1H), 7.14 (m, 1H). ¹³C NMR (175 MHz, dmso) δ 176.48, 162.64, 147.71, 146.32, 142.21, 132.24 (d, J = 4.9 Hz), 129.51 (d, J = 13.3 Hz), 126.12 (d, J = 32.1 Hz), 125.72, 125.63 (q, J = 3.7 Hz), 125.05, 123.51, 123.42, 122.89 (d, J = 4.7 Hz), 118.33 (d, J = 17.5 Hz), 117.64.

ESI-MS. Calcd for C₁₇H₁₃N₅O₂S (M⁺ +H): m/z 383.0511. Found: m/z 383.0605.

### Example 23. Synthesis of (Z)-2-(6-Methoxy-2-oxoindolin-3-ylidene)-N-phenylhydrazinethioamide (Compound 22)

According to the synthetic method of Example 2, the title compound was synthesized using phenylhydrazinethioamide and 6-methoxyisatin as starting materials.

¹H NMR (700 MHz, dmso) δ 12.67 (s, 1H), 11.22 (s, 1H), 10.70 (s, 1H), 7.68 (d, J = 8.4 Hz, 1H), 7.60 (d, J = 7.8 Hz, 2H), 7.42 (t, J = 7.9 Hz, 2H), 7.26 (m, 1H), 6.69 (dd, J = 8.4, 2.2 Hz, 1H), 6.51 - 6.47 (m, 1H), 3.81 (s, 3H).

### Example 24. Synthesis of (Z)-2-(6-Methoxy-2-oxoindolin-3-ylidene)-N-(3-nitrophenyl)hydrazine-1-carbothioamide (Compound 23)

According to the synthetic method of Example 2, the title compound was synthesized using N-(3-nitrophenyl)hydrazinethioamide and 6-methoxyisatin as starting materials.

¹H NMR (700 MHz, dmso) δ 12.83 (s, 1H), 11.25 (s, 1H), 10.95 (s, 1H), 8.67 (s, 1H), 8.18 (dd, J = 8.0, 2.0 Hz, 1H), 8.13 - 8.06 (m, 1H), 7.69 (m, 2H), 6.71 (dd, J = 8.4, 2.0 Hz, 1H), 6.47 (d, J = 2.0 Hz, 1H), 3.82 (s, 3H). ¹³C NMR (175 MHz, dmso) δ 176.13, 163.39, 162.74, 147.41, 144.60, 139.75, 133.01, 131.41, 129.55, 123.08, 120.25, 119.43, 112.08, 108.30, 97.60, 55.65.

ESI-MS. Calcd for C₁₆H₁₃N₅O₄S (M⁺ +H): m/z 372.0688. Found: m/z 372.0773.

### Example 25. Synthesis of (Z)-2-(6-Methoxy-2-oxoindolin-3-ylidene)-N-(2-methoxyphenyl)hydrazinethioamide (Compound 24)

According to the synthetic method of Example 2, the title compound was synthesized using N-(2-methoxyphenyl)hydrazinethioamide and 6-methoxyisatin as starting materials.

¹H NMR (700 MHz, dmso) δ 12.67 (s, 1H), 11.23 (s, 1H), 10.33 (s, 1H), 7.87 (d, J = 7.7 Hz, 1H), 7.59 (d, J = 8.3 Hz, 1H), 7.27 (td, J = 8.3, 1.5 Hz, 1H), 7.15 - 7.12 (m, 1H), 6.99 (td, J = 7.7, 1.0 Hz, 1H), 6.68 (dd, J = 8.4, 2.2 Hz, 1H), 6.49 (d, J = 2.2 Hz, 1H), 3.86 (s, 3H), 3.81 (s, 3H).

### Example 26. Synthesis of (Z)-2-(6-Methoxy-2-oxoindolin-3-ylidene)-N-(4-(trifluoromethyl)phenyl)hydrazinethioamide (Compound 26)

According to the synthetic method of Example 2, the title compound was synthesized using N-(4-(trifluoromethyl)phenyl)hydrazinethioamide and 6-methoxyisatin as starting materials.

¹H NMR (700 MHz, dmso) δ 12.80 (s, 1H), 11.25 (s, 1H), 10.89 (s, 1H), 7.93 (d, J = 8.4 Hz, 2H), 7.78 (d, J = 8.5 Hz, 2H), 7.68 (d, J = 8.4 Hz, 1H), 6.70 (dd, J = 8.5, 2.2 Hz, 1H), 6.49 (d, J = 2.2 Hz, 1H), 3.82 (s, 3H).

### Example 27. Synthesis of (Z)-N-ethyl-2-(6-methoxy-2-oxoindolin-3-ylidene)hydrazinethioamide (Compound 27)

According to the synthetic method of Example 2, the title compound was synthesized using N-ethylhydrazinethioamide and 6-methoxyisatin as starting materials.

¹H NMR (700 MHz, dmso) δ 12.42 (s, 1H), 11.16 (s, 1H), 9.15 (m, 1H), 7.56 (d, J = 8.4 Hz, 1H), 6.67 (dd, J = 8.4, 2.2 Hz, 1H), 6.47 (d, J = 2.2 Hz, 1H), 3.80 (s, 3H), 3.65 - 3.59 (m, 2H), 1.18 (t, J = 7.2 Hz, 3H).

### Example 28. Synthesis of (Z)-2-(6-Methoxy-2-oxoindolin-3-ylidene)-N-(m-tolyl)hydrazinethioamide (Compound 28)

According to the synthetic method of Example 2, the title compound was synthesized using N-(3-methylphenyl)hydrazinethioamide and 6-methoxyisatin as starting materials.

¹H NMR (700 MHz, dmso) δ 12.64 (s, 1H), 11.21 (s, 1H), 10.64 (s, 1H), 7.69 (d, J = 8.4 Hz, 1H), 7.44 - 7.40 (m, 2H), 7.29 (t, J = 7.7 Hz, 1H), 7.08 (d, J = 7.5 Hz, 1H), 6.69 (dd, J = 8.4, 2.1 Hz, 1H), 6.49 (d, J = 2.2 Hz, 1H), 3.81 (s, 3H), 2.33 (s, 3H).

### Example 29. Synthesis of (Z)-2-(5-methyl-2-oxoindolin-3-ylidene)-N-phenylhydrazinethioamide (Compound 29)

According to the synthetic method of Example 2, the title compound was synthesized using phenylhydrazinethioamide and 5-methylisatin as starting materials.

¹H NMR (700 MHz, dmso) δ 12.77 (s, 1H), 11.15 (s, 1H), 10.81 (s, 1H), 7.64 - 7.58 (m, 3H), 7.43 (t, J = 7.9 Hz, 2H), 7.28 (m, 1H), 7.18 (dd, J = 8.0, 0.9 Hz, 1H), 6.83 (d, J = 7.9 Hz, 1H), 2.30 (s, 3H).

### Example 30. Synthesis of (Z)-2-(5-methyl-2-oxoindolin-3-ylidene)-N-(3-nitrophenyl)hydrazinethioamide (Compound 30)

According to the synthetic method of Example 2, the title compound was synthesized using N-(3-nitrophenyl)hydrazincarbothioamide and 5-methylisatin as starting materials.

¹H NMR (700 MHz, dmso) δ 12.94 (s, 1H), 11.21 (s, 1H), 11.08 (s, 1H), 8.65 (t, J = 2.1 Hz, 1H), 8.17 (m, 1H), 8.12 (m, 1H), 7.72 (t, J = 8.1 Hz, 1H), 7.60 (s, 1H), 7.20 (d, J = 7.9 Hz, 1H), 6.84 (d, J = 7.9 Hz, 1H), 2.31 (s, 3H).

### Example 31. Synthesis of (Z)-N-(2-methoxyphenyl)-2-(5-methyl-2-oxoindolin-3-ylidene)hydrazinethioamide (Compound 31)

According to the synthetic method of Example 2, the title compound was synthesized using N-(2-methoxyphenyl)hydrazinethioamide and 5-methylisatin as starting materials.

¹H NMR (700 MHz, dmso) δ 12.76 (s, 1H), 11.16 (s, 1H), 10.44 (s, 1H), 7.74 (d, J = 7.8 Hz, 1H), 7.52 (s, 1H), 7.29 (m, 1H), 7.18 (d, J = 7.8 Hz, 1H), 7.14 (d, J = 8.3 Hz, 1H), 7.00 (td, J = 8.0, 1.1 Hz, 1H), 6.84 (d, J = 8.0 Hz, 1H), 3.85 (s, 3H), 2.31 (s, 3H).

### Example 32. Synthesis of (Z)-2-(5-methyl-2-oxoindolin-3-ylidene)-N-(3-(trifluoromethyl)phenyl)hydrazinethioamide (Compound 32)

According to the synthetic method of Example 2, the title compound was synthesized using N-(3-(trifluoromethyl)phenyl)hydrazinethioamide and 5-methylisatin as starting materials.

¹H NMR (700 MHz, dmso) δ 12.89 (s, 1H), 11.20 (s, 1H), 10.99 (s, 1H), 7.20-7.17 (m, 2H), 6.95 (m, 1H), 6.88-6.77 (m, 4H), 2.26 (s, 3H).

### Example 33. Synthesis of (Z)-N-(4-fluorophenyl)-2-(5-methyl-2-oxoindolin-3-ylidene)hydrazinethioamide (Compound 33)

According to the synthetic method of Example 2, the title compound was synthesized using N-(4-fluorophenyl)hydrazinethioamide and 5-methylisatin as starting materials.

¹H NMR (700 MHz, dmso) δ 12.78 (s, 1H), 11.16 (s, 1H), 10.82 (s, 1H), 7.60-7.57 (m, 3H), 7.26 (m, 2H), 7.18 (dd, J = 7.9, 0.9 Hz, 1H), 6.83 (d, J = 7.9 Hz, 1H), 2.30 (s, 3H).

### Example 34. Synthesis of (Z)-2-(5-bromo-2-oxindoline-3-ylidene)hydrazinethioamide (Compound 34)

According to the synthetic method of Example 2, the title compound was synthesized using thiosemicarbazide and 5-bromoisatin as starting materials.

¹H NMR (500 MHz, dmso) δ 12.28 (s, 1H), 11.28 (s, 1H), 9.10 (s, 1H), 8.81 (s, 1H), 7.88 (d, J = 2.1 Hz, 1H), 7.51 - 7.48 (m, 1H), 6.90 - 6.87 (m, 1H).

### Example 35. Synthesis of (Z)-2-(4-Bromo-2-oxindoline-3-ylidene)-N-(3-nitrophenyl)hydrazinecarbothioamide (Compound 35)

The title compound was synthesized according to the synthetic method of Example 2 using N-(3-nitrophenyl)hydrazinethioamide and 4-bromoisatin as starting materials.

¹H NMR (500 MHz, dmso) δ 13.23 (s, 1H), 11.54 (s, 1H), 10.33 (s, 1H), 8.86 (s, 1H), 8.19 - 7.98 (m, 2H), 7.71 (t, J = 8.2 Hz, 1H), 7.36 - 7.23 (m, 2H), 6.97 (m, 1H).

### Example 36. Synthesis of (Z)-2-(6-Bromo-2-oxindoline-3-ylidene)-N-(3-nitrophenyl)hydrazinecarbothioamide (Compound 36)

The title compound was synthesized according to the synthetic method of Example 2 using N-(3-nitrophenyl)hydrazinethioamide and 6-bromoisatin as starting materials.

¹H NMR (500 MHz, dmso) δ 13.23 (s, 1H), 11.54 (s, 1H), 10.33 (s, 1H), 8.86 (t, J = 2.1 Hz, 1H), 8.19 - 7.98 (m, 2H), 7.71 (t, J = 8.2 Hz, 1H), 7.36 - 7.23 (m, 2H), 6.97 (m, 1H).

### Example 37. Synthesis of (Z)-2-(4-Bromo-2-oxindoline-3-ylidene)-N-(4-nitrophenyl)hydrazinecarbothioamide (Compound 37)

The title compound was synthesized according to the synthetic method of Example 2 using N-(4-nitrophenyl)hydrazinethioamide and 4-bromoisatin as starting materials.

¹H NMR (500 MHz, dmso) δ 13.22 (s, 1H), 11.55 (s, 1H), 10.47 (s, 1H), 8.31 - 8.26 (m, 2H), 8.17 - 8.12 (m, 2H), 7.34 - 7.27 (m, 2H), 6.98 (m, 1H).

### Example 38. Synthesis of (Z)-2-(5-Bromo-2-oxindoline-3-ylidene)-N-(4-nitrophenyl)hydrazinecarbothioamide (Compound 38)

According to the synthetic method of Example 2, the title compound was synthesized using N-(4-nitrophenyl)hydrazincarbothioamide and 5-bromoisatin as starting materials.

¹H NMR (500 MHz, dmso) δ 12.81 (s, 1H), 11.39 (s, 1H), 11.11 (s, 1H), 8.34 - 8.28 (m, 2H), 8.10 - 8.06 (m, 2H), 7.99 (d, J = 2.1 Hz, 1H), 7.54 (m, 1H), 6.91 (m, 1H).

### Example 39. Synthesis of (Z)-N-(2-methoxyphenyl)-2-(2-oxindoline-3-ylidene)hydrazinecarbothioamide (Compound 39)

The title compound was synthesized according to the synthetic method of Example 2 using N-(2-methoxyphenyl)hydrazinethioamide and isatin as starting materials.

¹H NMR (500 MHz, dmso) δ 12.79 (s, 1H), 11.25 (s, 1H), 10.43 (s, 1H), 7.86 (dd, J = 7.8, 1.2 Hz, 1H), 7.68 (d, J = 7.5 Hz, 1H), 7.38 (m, 1H), 7.29 (ddd, J = 8.2, 7.5, 1.7 Hz, 1H), 7.16 - 7.09 (m, 2H), 7.00 (td, J = 7.7, 1.2 Hz, 1H), 6.95 (m, 1H), 3.87 (s, 3H).

### Example 40. Synthesis of (Z)-2-(4-Bromo-2-oxindoline-3-ylidene)hydrazinecarbothioamide (Compound 40)

The title compound was synthesized according to the synthetic method of Example 2 using thiosemicarbazide and 4-bromoisatin as starting materials.

¹H NMR (500 MHz, dmso) δ 12.77 (s, 1H), 11.43 (s, 1H), 9.30 (s, 1H), 7.71 (s, 1H), 7.29 - 7.23 (m, 2H), 6.94 (m, 1H).

### Example 41. Synthesis of (Z)-2-(6-Bromo-2-oxindoline-3-ylidene)hydrazinecarbothioamide (Compound 41)

The title compound was synthesized according to the synthetic method of Example 2 using thiosemicarbazide and 6-bromoisatin as starting materials.

¹H NMR (500 MHz, dmso) δ 12.37 (s, 1H), 11.31 (s, 1H), 9.08 (s, 1H), 8.73 (s, 1H), 7.58 (d, J = 8.1 Hz, 1H), 7.30 (m, 1H), 7.08 (d, J = 1.7 Hz, 1H).

### Example 42. Synthesis of (Z)-2-(7-Bromo-2-oxindolin-3-ylidene)hydrazinethioamide (Compound 42)

The title compound was synthesized according to the synthetic method of Example 2 using thiosemicarbazide and 7-bromoisatin as starting materials.

¹H NMR (500 MHz, dmso) δ 12.40 (s, 1H), 11.50 (s, 1H), 9.11 (s, 1H), 8.76 (s, 1H), 7.66 (d, J = 7.3 Hz, 1H), 7.56 - 7.52 (m, 1H), 7.04 (dt, J = 7.0, 6.0 Hz, 1H).

### Example 43. Synthesis of (Z)-2-(4-Bromo-2-oxindolin-3-ylidene)-N-phenylhydrazinethioamide (Compound 43)

The title compound was synthesized according to the synthetic method of Example 2 using phenylhydrazinethioamide and 4-bromoisatin as starting materials.

¹H NMR (500 MHz, dmso) δ 13.04 (s, 1H), 11.50 (s, 1H), 9.98 (s, 1H), 7.75-7.71 (m, 2H), 7.45-7.40 (m, 2H), 7.32 - 7.24 (m, 3H), 6.97 (m, 1H).

### Example 44. Synthesis of (Z)-2-(5-Bromo-2-oxindolin-3-ylidene)-N-phenylhydrazinethioamide (Compound 44)

The title compound was synthesized according to the synthetic method of Example 2 using phenylhydrazinethioamide and 5-bromoisatin as starting materials.

¹H NMR (500 MHz, dmso) δ 12.61 (s, 1H), 11.34 (s, 1H), 10.89 (s, 1H), 8.01 (d, J = 2.1 Hz, 1H), 7.62 - 7.59 (m, 2H), 7.54 - 7.51 (m, 1H), 7.46 - 7.42 (m, 2H), 7.31 - 7.27 (m, 1H), 6.90 (m, 1H).

### Example 45. Synthesis of (Z)-2-(6-Bromo-2-oxoindolin-3-ylidene)-N-phenylhydrazinethioamide (Compound 45)

According to the synthetic method of Example 2, the title compound was synthesized using phenylhydrazinethioamide and 6-bromoisatin as starting materials.

¹H NMR (500 MHz, dmso) δ 12.69 (s, 1H), 11.37 (s, 1H), 10.86 (s, 1H), 7.71 (d, J = 8.1 Hz, 1H), 7.62-7.60 (m, 2H), 7.45 - 7.40 (m, 2H), 7.33 (dd, J = 8.1, 2.1 Hz, 1H), 7.28 (ddt, J = 5.4, 4.3, 2.1 Hz, 1H), 7.10 (d, J = 1.6 Hz, 1H).

### Example 46. Synthesis of (Z)-2-(7-Bromo-2-oxoindolin-3-ylidene)-N-phenylhydrazinethioamide (Compound 46)

The title compound was synthesized according to the synthetic method of Example 2 using phenylhydrazinethioamide and 7-bromoisatin as starting materials.

¹H NMR (500 MHz, dmso) δ 12.73 (s, 1H), 11.56 (s, 1H), 10.89 (s, 1H), 7.7(m, 1H), 7.62-7.58 (m, 2H), 7.57 (m, 1H), 7.46 - 7.41 (m, 2H), 7.29 (m, 1H), 7.07(m, 1H).

### Example 47. Synthesis of (Z)-N-(3-nitrophenyl)-2-(2-oxoindolin-3-ylidene)hydrazinethioamide (Compound 47)

According to the synthetic method of Example 2, the title compound was synthesized using N-(3-nitrophenyl)hydrazinethioamide and isatin as starting materials.

¹H NMR (500 MHz, dmso) δ 12.95 (s, 1H), 11.29 (s, 1H), 11.09 (s, 1H), 8.66 (t, J = 2.2 Hz, 1H), 8.18 (m, 1H), 8.12 (m, 1H), 7.77 (m, 1H), 7.72 (dd, J = 10.8, 5.5 Hz, 1H), 7.39 (m, 1H), 7.13 (m, 1H), 6.96 (m, 1H).

### Example 48. Synthesis of (Z)-2-(5-Bromo-2-oxoindolin-3-ylidene)-N-(3-nitrophenyl)hydrazinethioamide (Compound 48)

According to the synthetic method of Example 2, the title compound was synthesized using N-(3-nitrophenyl)hydrazinethioamide and 5-bromoisatin as starting materials.

¹H NMR (500 MHz, dmso) δ 12.76 (s, 1H), 11.39 (s, 1H), 11.10 (s, 1H), 8.65 (m, 1H), 8.17 (m, 1H), 8.13 (m, 1H), 7.98 (d, J = 2.1 Hz, 1H), 7.74 (m, 1H), 7.54 (m, 1H), 6.92 (m, 1H).

### Example 49. Synthesis of (Z)-2-(7-Bromo-2-oxoindolin-3-ylidene)-N-(3-nitrophenyl)hydrazinethioamide (Compound 49)

The title compound was synthesized according to the synthetic method of Example 2 using N-(3-nitrophenyl)hydrazinethioamide and 7-bromoisatin as starting materials.

¹H NMR (500 MHz, dmso) δ 12.88 (s, 1H), 11.60 (s, 1H), 11.15 (s, 1H), 8.65 (t, J = 2.2 Hz, 1H), 8.18 (m, 1H), 8.12 (m, 1H), 7.79 (dd, J = 9.2, 4.9 Hz, 1H), 7.73 (dd, J = 10.9, 5.4 Hz, 1H), 7.59 (m, 1H), 7.10 (m, 1H).

### Example 50. Synthesis of (Z)-N-(3-Methoxyphenyl)-2-(2-oxoindolin-3-ylidene)hydrazinethioamide (Compound 50)

The title compound was synthesized according to the synthetic method of Example 2 using N-(3-methoxyphenyl)hydrazinethioamide and isatin as starting materials.

¹H NMR (500 MHz, dmso) δ 12.80 (s, 1H), 11.25 (s, 1H), 10.76 (s, 1H), 7.79 (m, 1H), 7.37 (td, J = 7.7, 1.3 Hz, 1H), 7.34 (s, 1H), 7.30 (dd, J = 4.4, 2.2 Hz, 1H), 7.24 (m, 1H), 7.11 (m, 1H), 6.95 (m, 1H), 6.85 (ddd, J = 8.2, 2.6, 0.9 Hz, 1H), 3.78 (s, 3H).

### Example 51. Synthesis of (Z)-2-(4-Bromo-2-oxoindolin-3-ylidene)-N-(3-methoxyphenyl)hydrazinethioamide (Compound 51)

According to the synthetic method of Example 2, the title compound was synthesized using N-(3-methoxyphenyl)hydrazinethioamide and 4-bromoisatin as starting materials.

¹H NMR (500 MHz, dmso) δ 13.02 (s, 1H), 11.50 (s, 1H), 9.96 (s, 1H), 7.54 (t, J = 2.2 Hz, 1H), 7.32 (m, 1H), 7.30 - 7.25 (m, 2H), 7.22 (m, 1H), 6.96 (m, 1H), 6.84 (ddd, J = 8.3, 2.5, 0.9 Hz, 1H), 3.78 (s, 3H).

### Example 52. Synthesis of (Z)-2-(5-Bromo-2-oxindoline-3-ylidene)-N-(3-methoxyphenyl)hydrazinecarbothioamide (Compound 52)

The title compound was synthesized according to the synthetic method of Example 2 using N-(3-methoxyphenyl)hydrazinethioamide and 5-bromoisatin as starting materials.

¹H NMR (500 MHz, dmso) δ 12.62 (s, 1H), 11.35 (s, 1H), 10.84 (s, 1H), 8.02 (d, J = 2.1 Hz, 1H), 7.52 (m, 1H), 7.34 (m, 1H), 7.28 (t, J = 2.2 Hz, 1H), 7.22 (m, 1H), 6.90 (m, 1H), 6.87 (m, 1H), 3.79 (s, 3H).

### Example 53. Synthesis of (Z)-N-(4-methoxyphenyl)-2-(2-oxindoline-3-ylidene)hydrazinecarbothioamide (Compound 53)

According to the synthetic method of Example 2, the title compound was synthesized using N-(4-methoxyphenyl)hydrazinethioamide and isatin as starting materials.

¹H NMR (500 MHz, dmso) δ 12.75 (s, 1H), 11.24 (s, 1H), 10.72 (s, 1H), 7.77 (d, J = 7.5 Hz, 1H), 7.48 - 7.45 (m, 2H), 7.37 (td, J = 7.7, 1.3 Hz, 1H), 7.11 (m, 1H), 7.00 - 6.96 (m, 2H), 6.94 (dt, J = 7.9, 0.8 Hz, 1H), 3.78 (s, 3H).

### Example 54. Synthesis of (Z)-2-(4-Bromo-2-oxindoline-3-ylidene)-N-(4-methoxyphenyl)hydrazinecarbothioamide (Compound 54)

The title compound was synthesized according to the synthetic method of Example 2 using N-(4-methoxyphenyl)hydrazinethioamide and 4-bromoisatin as starting materials.

¹H NMR (500 MHz, dmso) δ 13.02 (s, 1H), 11.49 (s, 1H), 9.85 (s, 1H), 7.54 (m, 2H), 7.29 (m, 2H), 6.98 (m, 3H), 3.78 (s, 3H).

### Example 55. Synthesis of (Z)-2-(5-Bromo-2-oxindoline-3-ylidene)-N-(4-methoxyphenyl)hydrazinecarbothioamide (Compound 55)

According to the synthetic method of Example 2, the title compound was synthesized using N-(4-methoxyphenyl)hydrazinethioamide and 5-bromoisatin as starting materials.

¹H NMR (500 MHz, dmso) δ 12.56 (s, 1H), 11.33 (s, 1H), 10.80 (s, 1H), 8.00 (d, J = 1.9 Hz, 1H), 7.51 (m, 1H), 7.48 - 7.43 (m, 2H), 7.01 - 6.97 (m, 2H), 6.90 (m, 1H), 3.79 (s, 3H).

### Example 56. Synthesis of N-(3,4-dimethoxyphenyl)hydrazinecarbothioamide (Compound 56)

According to the synthetic method of Example 2, the title compound was synthesized using 3,4-dimethoxyphenyl isothiocyanate and hydrazine as starting materials.

¹H NMR (700 MHz, dmso) δ 9.53 (s, 1H), 9.00 (s, 1H), 7.36 (s, 1H), 7.10 (s, 1H), 6.87 (d, J = 8.6 Hz, 1H), 4.73 (s, 2H), 3.72 (s, 3H), 3.71 (s, 3H).

### Example 57. Synthesis of (Z)-N-(4-fluorophenyl)-2-(2-oxindoline-3-ylidene)hydrazinecarbothioamide (Compound 57)

The title compound was synthesized according to the synthetic method of Example 2 using N-(4-fluorophenyl)hydrazinethioamide and isatin as starting materials.

¹H NMR (700 MHz, dmso) δ 12.80 (s, 1H), 11.27 (s, 1H), 10.84 (s, 1H), 7.75 (d, J = 7.5 Hz, 1H), 7.62 - 7.57 (m, 2H), 7.37 (td, J = 7.7, 1.2 Hz, 1H), 7.29 - 7.24 (m, 2H), 7.11 (td, J = 7.5, 0.7 Hz, 1H), 6.95 (d, J = 7.8 Hz, 1H).

### Example 58. Synthesis of (Z)-2-(6-bromo-2-oxindoline-3-ylidene)-N-(4-nitrophenyl)hydrazinethioamide (Compound 58)

The title compound was synthesized according to the synthetic method of Example 2 using N-(4-nitrophenyl)hydrazincarbothioamide and 6-bromoisatin as starting materials.

¹H NMR (700 MHz, dmso) δ 12.89 (s, 1H), 11.43 (s, 1H), 11.15 (s, 1H), 8.36 - 8.22 (m, 2H), 8.15 - 8.00 (m, 2H), 7.70 (d, J = 8.1 Hz, 1H), 7.32 (ddd, J = 21.7, 11.6, 4.4 Hz, 1H), 7.12 (d, J = 1.6 Hz, 1H).

### Example 60. Synthesis of (Z)-2-(6-chloro-2-oxindoline-3-ylidene)-N-phenylhydrazinethioamide (Compound 60)

According to the synthetic method of Example 2, the title compound was synthesized using phenylhydrazinecarbothioamide and 6-chloro isatin as starting materials.

¹H NMR (700 MHz, dmso) δ 12.68 (s, 1H), 11.40 (s, 1H), 10.87 (s, 1H), 7.77 (d, J = 8.1 Hz, 1H), 7.60 (d, J = 7.7 Hz, 2H), 7.43 (t, J = 7.9 Hz, 2H), 7.28 (t, J = 7.4 Hz, 1H), 7.19 (dd, J = 8.1, 1.8 Hz, 1H), 6.98 (d, J = 1.8 Hz, 1H).

### Example 61. Synthesis of (Z)-2-(4-chloro-2-oxindoline-3-ylidene)-N-(2-methoxyphenyl)hydrazinethioamide (Compound 61)

According to the synthetic method of Example 2, the title compound was synthesized using N-(2-methoxyphenyl)hydrazinethioamide and 4-chloroisatin as starting materials.

¹H NMR (700 MHz, dmso) δ 12.96 (s, 1H), 11.53 (s, 1H), 10.35 (s, 1H), 8.83 (d, J = 7.7 Hz, 1H), 7.37 (t, J = 8.0 Hz, 1H), 7.20 (m, 1H), 7.16 - 7.13 (m, 2H), 7.01 (m, 1H), 6.92 (d, J = 7.8 Hz, 1H), 3.88 (s, 3H).

### Example 62. Synthesis of (Z)-2-(5-fluoro-2-oxindoline-3-ylidene)-N-(3-(trifluoromethyl)phenyl)hydrazinethioamide (Compound 62)

According to the synthetic method of Example 2, the title compound was synthesized using N-(3-(trifluoromethyl)phenyl)hydrazinethioamide and 5-fluoroisatin as starting materials.

¹H NMR (700 MHz, dmso) δ 12.78 (s, 1H), 11.29 (s, 1H), 11.00 (s, 1H), 8.15 - 7.91 (m, 2H), 7.76 - 7.55 (m, 3H), 7.23 (m, 1H), 6.95 (dd, J = 8.6, 4.1 Hz, 1H).

### Example 63. Synthesis of (Z)-N-Benzyl-2-(5-fluoro-2-oxindoline-3-ylidene)hydrazinecarbothioamide (Compound 63)

According to the synthetic method of Example 2, the title compound was synthesized using N-benzylhydrazinethioamide and 5-fluoroisatin as starting materials.

¹H NMR (700 MHz, dmso) δ 12.52 (s, 1H), 11.20 (s, 1H), 9.85 (t, J = 6.2 Hz, 1H), 7.45 (dd, J = 8.1, 2.6 Hz, 1H), 7.36 - 7.30 (m, 4H), 7.24 (dd, J = 9.5, 4.3 Hz, 1H), 7.16 (td, J = 9.4, 2.7 Hz, 1H), 6.90 (dd, J = 8.6, 4.1 Hz, 1H), 4.86 (d, J = 6.2 Hz, 2H).

### Example 64. Synthesis of (Z)-2-(5-Fluoro-2-oxindoline-3-ylidene)-N-(m-tolyl)hydrazinethioamide (Compound 64)

According to the synthetic method of Example 2, the title compound was synthesized using N-(3-methylphenyl)hydrazinethioamide and 5-fluoroisatin as starting materials.

¹H NMR (700 MHz, dmso) δ 12.65 (s, 1H), 11.26 (s, 1H), 10.79 (s, 1H), 7.65 (dd, J = 8.1, 2.5 Hz, 1H), 7.46 - 7.36 (m, 2H), 7.32 (t, J = 7.7 Hz, 1H), 7.21 (td, J = 9.3, 2.7 Hz, 1H), 7.10 (d, J = 7.5 Hz, 1H), 6.94 (dd, J = 8.6, 4.1 Hz, 1H), 2.34 (s, 3H).

### Example 65. Synthesis of (Z)-2-(7-Fluoro-2-oxoindolin-3-ylidene)-N-(4-nitrophenyl)hydrazinecarbothioamide (Compound 65)

According to the synthetic method of Example 2, the title compound was synthesized using N-(4-nitrophenyl)hydrazinethioamide and 7-fluoroisatin as starting materials.

¹H NMR (700 MHz, dmso) δ 12.96 (s, 1H), 11.84 (s, 1H), 11.17 (s, 1H), 8.37 - 8.20 (d, J = 9.1 Hz, 2H), 8.07 (d, J = 9.1 Hz, 2H), 7.63 (d, J = 7.5 Hz, 1H), 7.32 (dd, J = 9.9, 8.9 Hz, 1H), 7.14 (m, 1H).

### Example 66. Synthesis of (Z)-2-(6-Methoxy-2-oxoindolin-3-ylidene)-N-(3-(trifluoromethyl)phenyl)hydrazinecarbothioamide (Compound 66)

According to the synthetic method of Example 2, the title compound was synthesized using N-(3-(trifluoromethyl)phenyl)hydrazinethioamide and 6-methoxyisatin as starting materials.

¹H NMR (700 MHz, dmso) δ 12.77 (s, 1H), 11.25 (s, 1H), 10.87 (s, 1H), 8.06 (s, 1H), 8.00 (d, J = 8.0 Hz, 1H), 7.69 - 7.58 (m, 3H), 6.68 (m, 1H), 6.47 (d, J = 2.2 Hz, 1H), 3.82 (s, 3H).

### Example 67. Synthesis of (Z)-N-(4-fluorophenyl)-2-(6-methoxy-2-oxoindolin-3-ylidene)hydrazine-l-carbothioamide (Compound 67)

According to the synthetic method of Example 2, the title compound was synthesized using N-(4-fluorophenyl)hydrazinethioamide and 6-methoxyisatin as starting materials.

¹H NMR (700 MHz, dmso) δ 12.68 (s, 1H), 11.23 (s, 1H), 10.71 (s, 1H), 7.66 (d, J = 8.4 Hz, 1H), 7.62 - 7.55 (m, 2H), 7.29 - 7.21 (m, 2H), 6.67 (ddd, J = 34.4, 8.6, 2.3 Hz, 1H), 6.46 (d, J = 2.3 Hz, 1H), 3.81 (s, 3H).

### Example 68. Synthesis of (Z)-N-(4-bromophenyl)-2-(6-methoxy-2-oxoindolin-3-ylidene)hydrazine-l-carbothioamide (Compound 68)

The title compound was synthesized according to the synthetic method of Example 2 using N-(4-bromophenyl)hydrazinethioamide and 6-methoxyisatin as starting materials.

¹H NMR (700 MHz, dmso) δ 12.72 (s, 1H), 11.23 (s, 1H), 10.72 (s, 1H), 7.67 (d, J = 8.4 Hz, 1H), 7.60 (m, 4H), 6.68 (m, 1H), 6.46 (d, J = 2.3 Hz, 1H), 3.82 (s, 3H).

### Example 69. Synthesis of (Z)-N-Benzyl-2-(6-methoxy-2-oxoindolin-3-ylidene)hydrazinecarbothioamide (Compound 69)

According to the synthetic method of Example 2, the title compound was synthesized using N-benzylhydrazinethioamide and 6-methoxyisatin as starting materials.

¹H NMR (700 MHz, dmso) δ 12.53 (s, 1H), 11.18 (s, 1H), 9.69 (t, J = 6.2 Hz, 1H), 7.55 (d, J = 8.4 Hz, 1H), 7.37-7.32 (m, 4H), 7.26 (t, J = 7.1 Hz, 1H), 6.63 (m, 1H), 6.45 (d, J = 2.3 Hz, 1H), 4.86 (d, J = 6.5 Hz, 2H), 3.81 (s, 3H).

### Example 70. Synthesis of (Z)-N-(4-bromophenyl)-2-(5-methyl-2-oxoindolin-3-ylidene)hydrazinethioamide (Compound 70)

The title compound was synthesized according to the synthetic method of Example 2 using N-(4-bromophenyl)hydrazinethioamide and 5-methylisatin as starting materials.

¹H NMR (700 MHz, dmso) δ 12.82 (s, 1H), 11.18 (s, 1H), 10.84 (s, 1H), 7.71 - 7.51 (m, 5H), 7.19 (dd, J = 8.0 Hz, 1H), 6.82 (d, J = 8.0 Hz, 1H), 2.28 (s, 3H).

### Example 71. Synthesis of (Z)-N-(4-cyanophenyl)-2-(5-methyl-2-oxoindolin-3-ylidene)hydrazinethioamide (Compound 71)

According to the synthetic method of Example 2, the title compound was synthesized using N-(4-cyanophenyl)hydrazinethioamide and 5-methylisatin as starting materials.

¹H NMR (700 MHz, dmso) δ 12.94 (s, 1H), 11.19 (s, 1H), 10.96 (s, 1H), 7.97 (d, J = 8.6 Hz, 2H), 7.89 (m, J = 8.6 Hz, 2H), 7.60 (s, 1H), 7.20 (d, J = 7.9 Hz, 1H), 6.82 (d, J = 7.9 Hz, 1H), 2.28 (s, 3H).

### Example 72. Synthesis of (Z)-2-(5-methyl-2-oxoindolin-3-ylidene)-N-phenylhydrazinethioamide (Compound 72)

According to the synthetic method of Example 2, the title compound was synthesized using phenylhydrazinethioamide and 5-methylisatin as starting materials.

¹H NMR (700 MHz, dmso) δ 12.91 (s, 1H), 11.19 (s, 1H), 10.99 (s, 1H), 7.93 (d, J = 8.4 Hz, 2H), 7.79 (d, J = 8.4 Hz, 2H), 7.61 (s, 1H), 7.17 (m, 1H), 6.84 (d, J = 7.9 Hz, 1H), 2.31 (s, 3H).

### Example 73. Synthesis of (Z)-N-ethyl-2-(5-methyl-2-oxindoline-3-ylidene)hydrazinethioamide (Compound 73)

According to the synthetic method of Example 2, the title compound was synthesized using N-ethylhydrazinethioamide and 5-methylisatin as starting materials.

¹H NMR (700 MHz, dmso) δ 12.52 (s, 1H), 11.10 (s, 1H), 9.27 (t, J = 5.8 Hz, 1H), 7.49 (s, 1H), 7.19 (m, 1H), 6.81 (m, 1H), 3.73 - 3.53 (m, 2H), 2.31 (s, 3H), 1.19 (t, J = 7.2 Hz, 3H).

### Example 74. Synthesis of (Z)-N-Benzyl-2-(5-methyl-2-oxindoline-3-ylidene)hydrazinethioamide (Compound 74)

According to the synthetic method of Example 2, the title compound was synthesized using N-benzylhydrazinethioamide and 5-methylisatin as starting materials.

¹H NMR (700 MHz, dmso) δ 12.64 (s, 1H), 11.11 (s, 1H), 9.81 (t, J = 6.3 Hz, 1H), 7.48 (s, 1H), 7.35 (m, 4H), 7.25 (dd, J = 12.6, 12.4 Hz, 1H), 7.16 (dd, J = 7.9, 0.9 Hz, 1H), 6.82 (d, J = 7.9 Hz, 1H), 4.87 (d, J = 8.7 Hz, 2H), 2.28 (s, 3H).

### Example 75. Synthesis of (Z)-2-(5-methyl-2-oxindoline-3-ylidene)-N-(m-tolyl)hydrazinethioamide (Compound 75)

According to the synthetic method of Example 2, the title compound was synthesized using N-(3-methylphenyl)hydrazinethioamide and 5-methylisatin as starting materials.

¹H NMR (700 MHz, dmso) δ 12.75 (s, 1H), 11.15 (s, 1H), 10.75 (s, 1H), 7.62 (s, 1H), 7.48 - 7.38 (m, 2H), 7.30 (t, J = 7.7 Hz, 1H), 7.18 (dd, J = 7.9, 0.8 Hz, 1H), 7.09 (d, J = 7.5 Hz, 1H), 6.83 (d, J = 7.9 Hz, 1H), 2.34 (s, 3H), 2.30 (s, 3H).

### Example 76. Synthesis of (Z)-2-(2-oxo-5-(trifluoromethoxy)indolin-3-ylidene)-N-phenylhydrazinethioamide (Compound 76)

The title compound was synthesized according to the synthetic method of Example 2 using phenylhydrazinecarbothioamide and 5-(trifluoromethoxy)isatin as starting materials.

¹H NMR (700 MHz, dmso) δ 12.65 (s, 1H), 11.42 (s, 1H), 10.90 (s, 1H), 7.80 (s, 1H), 7.59 (d, J = 7.7 Hz, 2H), 7.44 (t, J = 7.7 Hz, 2H), 7.37 (dd, J = 8.5, 1.9 Hz, 1H), 7.30 (t, J = 7.4 Hz, 1H), 7.03 (d, J = 8.5 Hz, 1H).

### Example 77. Synthesis of (Z)-N-(3-nitrophenyl)-2-(2-oxo-5-(trifluoromethoxy)indolin-3- ylidene)hydrazinecarbothioamide (Compound 77)

According to the synthetic method of Example 2, the title compound was synthesized using N-(3-nitrophenyl)hydrazincarbothioamide and 5-(trifluoromethoxy)isatin as starting materials.

¹H NMR (700 MHz, dmso) δ 12.81 (s, 1H), 11.47 (s, 1H), 11.14 (s, 1H), 8.63 (t, J = 2.1 Hz, 1H), 8.15 (m, 2H), 7.77 (s, 1H), 7.73 (t, J = 8.2 Hz, 1H), 7.40 (dd, J = 8.5, 1.9 Hz, 1H), 7.04 (d, J = 8.5 Hz, 1H).

### Example 78. Synthesis of (Z)-N-(4-fluorophenyl)-2-(2-oxo-5-(trifluoromethoxy)indolin-3- ylidene)hydrazinecarbothioamide (Compound 78)

According to the synthetic method of Example 2, the title compound was synthesized by using N-(4-fluorophenyl)hydrazinethioamide and 5-(trifluoromethoxy)isatin as starting materials.

¹H NMR (700 MHz, dmso) δ 12.66 (s, 1H), 11.43 (s, 1H), 10.90 (s, 1H), 7.78 (s, 1H), 7.60-7.56 (m, 2H), 7.38 (d, J = 10.1 Hz, 1H), 7.30-7.26 (m, 2H), 7.04 (d, J = 8.5 Hz, 1H).

### Example 79. Synthesis of (Z)-N-(4-bromophenyl)-2-(2-oxo-5-(trifluoromethoxy)indolin-3-ylidene)hydrazinethioamide (Compound 79)

According to the synthetic method of Example 2, the title compound was synthesized using N-(4-bromophenyl)hydrazinethioamide and 5-(trifluoromethoxy)isatin as starting materials.

¹H NMR (700 MHz, dmso) δ 12.70 (s, 1H), 11.43 (s, 1H), 10.91 (s, 1H), 7.78 (s, 1H), 7.63 (d, J = 8.8 Hz, 2H), 7.59 (d, J = 8.8 Hz, 2H), 7.38 (dd, J = 8.5, 1.8 Hz, 1H), 7.03 (d, J = 8.5 Hz, 1H).

The compounds represented by Formula 1 are summarized in Table 1 below.

**Table 1**

| Compound No. | Generic Name |
|---|---|
| 1 | N-(3-(trifluoromethyl)phenyl)hydrazinethioamide 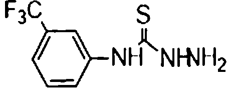 |
| 2 | N-(4-fluorophenyl)hydrazinethioamide 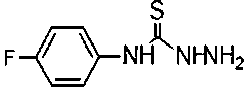 |
| 3 | N-(4-bromophenyl)hydrazinethioamide 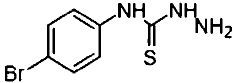 |
| 4 | N-(4-cyanophenyl)hydrazinethioamide 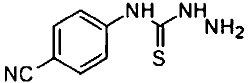 |
| 5 | N-(4-(trifluoromethyl)phenyl)hydrazinethioamide 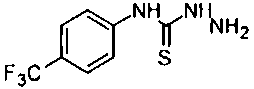 |
| 6 | N-ethylhydrazinethioamide 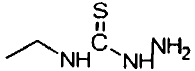 |
| 7 | N-isopropylhydrazinethioamide 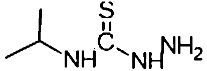 |
| 8 | N-isobutylhydrazinethioamide 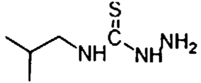 |
| 9 | N-(hydrazinocarbonothioyl)benzamide |
| | 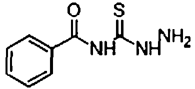 |
| 10 | N-benzylhydrazinethioamide 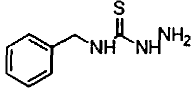 |
| 11 | N-(m-tolyl)hydrazinethioamide 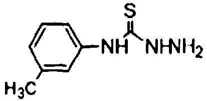 |
| 12 | (Z)-2-(5-fluoro-2-oxoindolin-3-ylidene)-N-phenylhydrazinethioamide 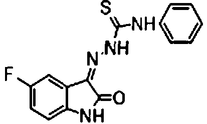 |
| 13 | (Z)-2-(5-fluoro-2-oxoindolin-3-ylidene)-N-(3-nitrophenyl)hydrazinethioamide 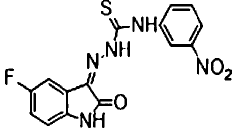 |
| 14 | (Z)-2-(5-fluoro-2-oxoindolin-3-ylidene)-N-(4-fluorophenyl)hydrazinethioamide 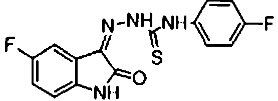 |
| 15 | (Z)-N-(4-bromophenyl)-2-(5-fluoro-2-oxoindolin-3-ylidene)hydrazinethioamide 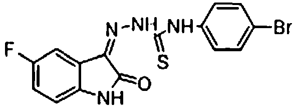 |
| 16 | (Z)-N-(4-cyanophenyl)-2-(5-fluoro-2-oxoindolin-3-ylidene)hydrazinethioamide 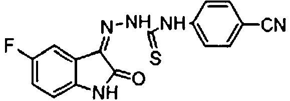 |
| 17 | (Z)-2-(5-fluoro-2-oxoindolin-3-ylidene)-N-(4-(trifluoromethyl)phenyl)hydrazinethioamide |
| | 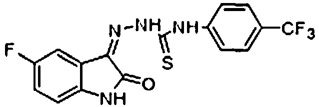 |
| 18 | (Z)-2-(7-fluoro-2-oxoindolin-3-ylidene)-N-(4-fluorophenyl)hydrazinethioamide 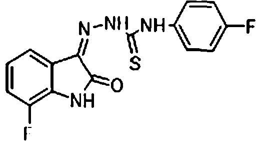 |
| 19 | (Z)-N-(4-bromophenyl)-2-(7-fluoro-2-oxoindolin-3-ylidene)hydrazinethioamide 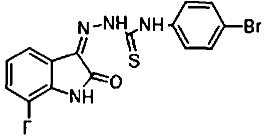 |
| 20 | (Z)-N-(4-cyanophenyl)-2-(7-fluoro-2-oxoindolin-3-ylidene)hydrazinethioamide 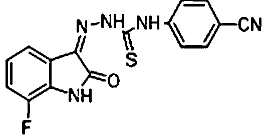 |
| 21 | (Z)-2-(7-fluoro-2-oxoindolin-3-ylidene)-N-(4-(trifluoromethyl)phenyl)hydrazinethioamide 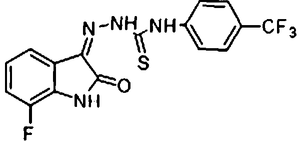 |
| 22 | (Z)-2-(6-methoxy-2-oxoindolin-3-ylidene)-N-phenylhydrazinethioamide 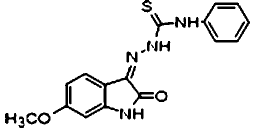 |
| 23 | (Z)-2-(6-methoxy-2-oxoindolin-3-ylidene)-N-(3-nitrophenyl)hydrazin-1-carbothioamide 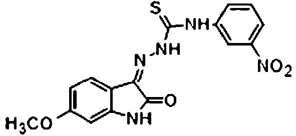 |
| 24 | (Z)-2-(6-methoxy-2-oxoindolin-3-ylidene)-N-(2-methoxyphenyl)hydrazin-1-carbothioamide 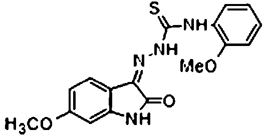 |
| 25 | (Z)-N-(4-cyanophenyl)-2-(6-methoxy-2-oxoindolin-3-ylidene)hydrazinethioamide 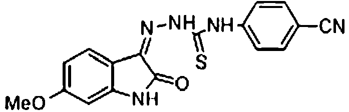 |
| 26 | (Z)-2-(6-methoxy-2-oxoindolin-3-ylidene)-N-(4-(trifluoromethyl)phenyl)hydrazinethioamide 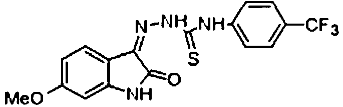 |
| 27 | (Z)-N-ethyl-2-(6-methoxy-2-oxoindolin-3-ylidene)hydrazinethioamide 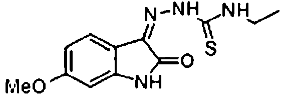 |
| 28 | (Z)-2-(6-methoxy-2-oxoindolin-3-ylidene)-N-(m-tolyl)hydrazinethioamide 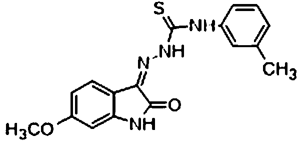 |
| 29 | (Z)-2-(5-methyl-2-oxoindolin-3-ylidene)-N-phenylhydrazinethioamide 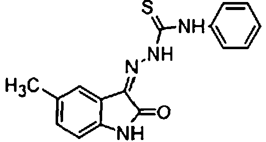 |
| 30 | (Z)-2-(5-methyl-2-oxoindolin-3-ylidene)-N-(3-nitrophenyl)hydrazinethioamide 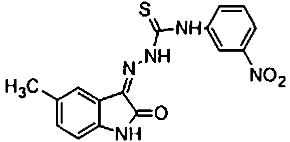 |
| 31 | (Z)-N-(2-methoxyphenyl)-2-(5-methyl-2-oxoindolin-3-ylidene)hydrazinethioamide 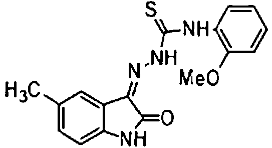 |
| 32 | (Z)-2-(5-methyl-2-oxoindolin-3-ylidene)-N-(3-(trifluoromethyl)phenyl)hydrazinethioamide 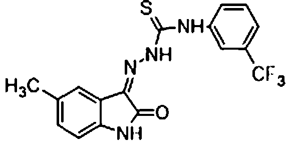 |
| 33 | (Z)-N-(4-fluorophenyl)-2-(5-methyl-2-oxoindolin-3-ylidene)hydrazinethioamide 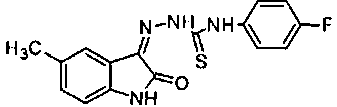 |
| 34 | (Z)-2-(5-bromo-2-oxoindolin-3-ylidene)hydrazinethioamide 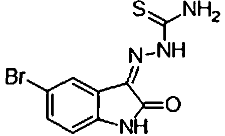 |
| 35 | (Z)-2-(4-bromo-2-oxoindolin-3-ylidene)-N-(3-nitrophenyl)hydrazinethioamide 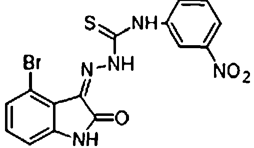 |
| 36 | (Z)-2-(6-bromo-2-oxoindolin-3-ylidene)-N-(3-nitrophenyl)hydrazinethioamide 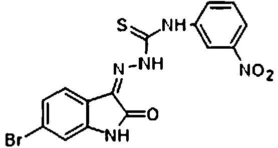 |
| 37 | (Z)-2-(4-bromo-2-oxoindolin-3-ylidene)-N-(4-nitrophenyl)hydrazinethioamide |
| | 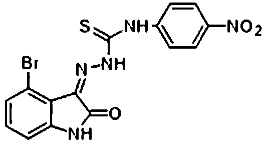 |
| 38 | (Z)-2-(5-bromo-2-oxoindolin-3-ylidene)-N-(4-nitrophenyl)hydrazinethioamide 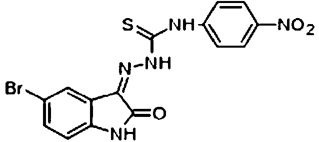 |
| 39 | (Z)-N-(2-methoxyphenyl)-2-(2-oxoindolin-3-ylidene)hydrazinethioamide 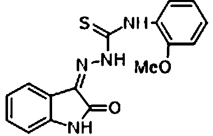 |
| 40 | (Z)-2-(4-bromo-2-oxoindolin-3-ylidene)hydrazinethioamide 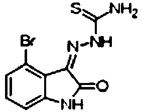 |
| 41 | (Z)-2-(6-bromo-2-oxoindolin-3-ylidene)hydrazinethioamide 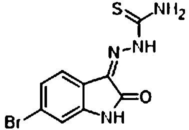 |
| 42 | (Z)-2-(7-bromo-2-oxoindolin-3-ylidene)hydrazinethioamide 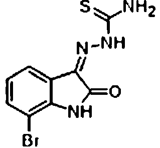 |
| 43 | (Z)-2-(4-bromo-2-oxoindolin-3-ylidene)-N-phenylhydrazinethioamide 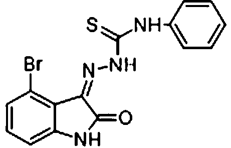 |
| 44 | (Z)-2-(5-bromo-2-oxoindolin-3-ylidene)-N-phenylhydrazinethioamide |
| | 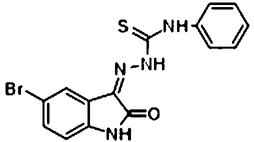 |
| 45 | (Z)-2-(6-bromo-2-oxoindolin-3-ylidene)-N-phenylhydrazinethioamide 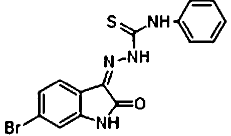 |
| 46 | (Z)-2-(7-bromo-2-oxoindolin-3-ylidene)-N-phenylhydrazinethioamide 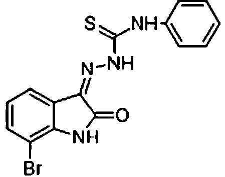 |
| 47 | (Z)-N-(3-nitrophenyl)-2-(2-oxoindolin-3-ylidene)hydrazinethioamide 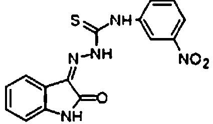 |
| 48 | (Z)-2-(5-bromo-2-oxoindolin-3-ylidene)-N-(3-nitrophenyl)hydrazinethioamide 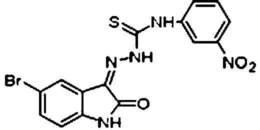 |
| 49 | (Z)-2-(7-bromo-2-oxoindolin-3-ylidene)-N-(3-nitrophenyl)hydrazinethioamide 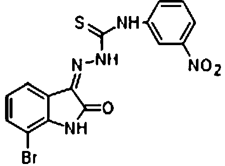 |
| 50 | (Z)-N-(3-methoxyphenyl)-2-(2-oxoindolin-3-ylidene)hydrazinethioamide 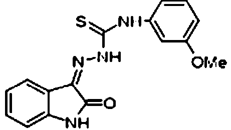 |
| 51 | (Z)-2-(4-bromo-2-oxoindolin-3-ylidene)-N-(3-methoxyphenyl)hydrazinethioamide 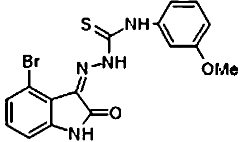 |
| 52 | (Z)-2-(5-bromo-2-oxoindolin-3-ylidene)-N-(3-methoxyphenyl)hydrazinethioamide 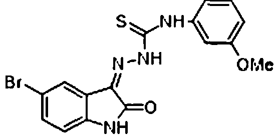 |
| 53 | (Z)-N-(4-methoxyphenyl)-2-(2-oxoindolin-3-ylidene)hydrazinethioamide 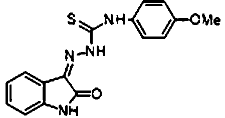 |
| 54 | (Z)-2-(4-bromo-2-oxoindolin-3-ylidene)-N-(4-methoxyphenyl)hydrazinethioamide 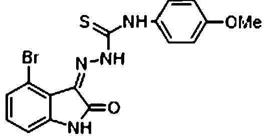 |
| 55 | (Z)-2-(5-bromo-2-oxoindolin-3-ylidene)-N-(4-methoxyphenyl)hydrazinethioamide 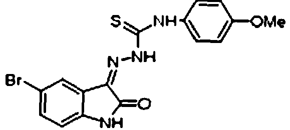 |
| 56 | N-(3,4-dimethoxyphenyl)hydrazinethioamide 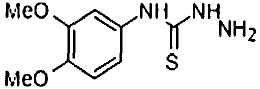 |
| 57 | (Z)-N-(4-fluorophenyl)-2-(2-oxoindolin-3-ylidene)hydrazinethioamide 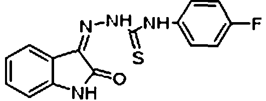 |
| 58 | (Z)-2-(6-bromo-2-oxoindolin-3-ylidene)-N-(4-nitrophenyl)hydrazinethioamide 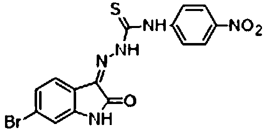 |
| 59 | (Z)-2-(4-chloro-2-oxoindolin-3-ylidene)-N-phenylhydrazinethioamide 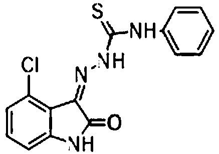 |
| 60 | (Z)-2-(6-chloro-2-oxoindolin-3-ylidene)-N-phenylhydrazinethioamide 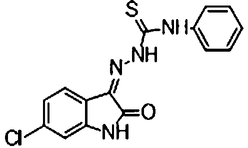 |
| 61 | (Z)-2-(4-chloro-2-oxoindolin-3-ylidene)-N-(2-methoxyphenyl)hydrazinethioamide 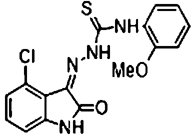 |
| 62 | (Z)-2-(5-fluoro-2-oxoindolin-3-ylidene)-N-(3-(trifluoromethyl)phenyl)hydrazinethioamide 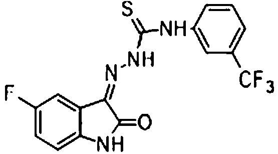 |
| 63 | (Z)-N-benzyl-2-(5-fluoro-2-oxoindolin-3-ylidene)hydrazinethioamide 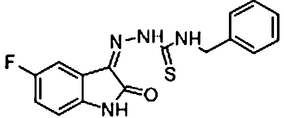 |
| 64 | (Z)-2-(5-fluoro-2-oxoindolin-3-ylidene)-N-(m-tolyl)hydrazinethioamide 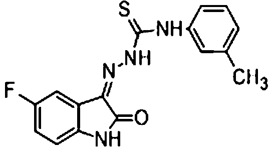 |
| 65 | (Z)-2-(7-fluoro-2-oxoindolin-3-ylidene)-N-(4-nitrophenyl)hydrazinethioamide 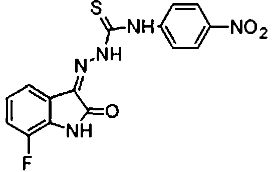 |
| 66 | (Z)-2-(6-methoxy-2-oxoindolin-3-ylidene)-N-(3-(trifluoromethyl)phenyl)hydrazinethioamide 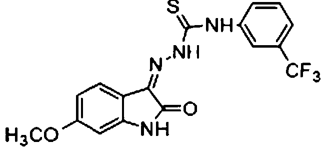 |
| 67 | (Z)-N-(4-fluorophenyl)-2-(6-methoxy-2-oxoindolin-3-ylidene)hydrazin-1-carbothioamide 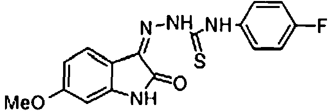 |
| 68 | (Z)-N-(4-bromophenyl)-2-(6-methoxy-2-oxoindolin-3-ylidene)hydrazin-1-carbothioamide 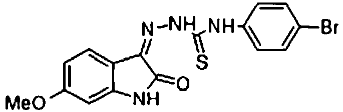 |
| 69 | (Z)-N-benzyl-2-(6-methoxy-2-oxoindolin-3-ylidene)hydrazin-1-carbothioamide 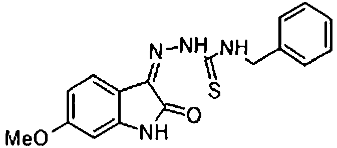 |
| 70 | (Z)-N-(4-bromophenyl)-2-(5-methyl-2-oxoindolin-3-ylidene)hydrazinethioamide 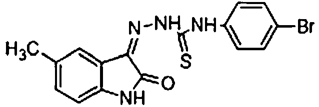 |
| 71 | (Z)-N-(4-cyanophenyl)-2-(5-methyl-2-oxoindolin-3-ylidene)hydrazinethioamide 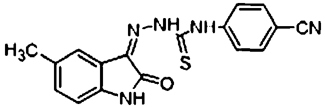 |
| 72 | (Z)-2-(5-methyl-2-oxoindolin-3-ylidene)-N-phenylhydrazinethioamide 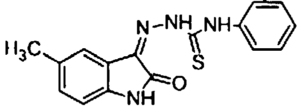 |
| 73 | (Z)-N-ethyl-2-(5-methyl-2-oxoindolin-3-ylidene)hydrazinethioamide 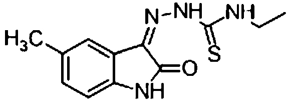 |
| 74 | (Z)-N-benzyl-2-(5-methyl-2-oxoindolin-3-ylidene)hydrazinethioamide 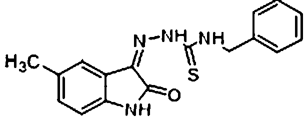 |
| 75 | (Z)-2-(5-methyl-2-oxoindolin-3-ylidene)-N-(m-tolyl)hydrazinethioamide 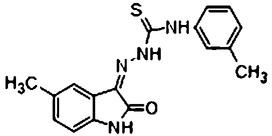 |
| 76 | (Z)-2-(2-oxo-5-(trifluoromethoxy)indolin-3-ylidene)-N-phenylhydrazinethioamide 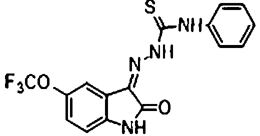 |
| 77 | (Z)-N-(3-nitrophenyl)-2-(2-oxo-5-(trifluoromethoxy)indolin-3-ylidene)hydrazinethioamide 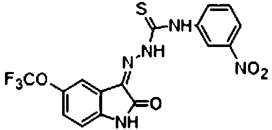 |
| 78 | (Z)-N-(4-fluorophenyl)-2-(2-oxo-5-(trifluoromethoxy)indolin-3-ylidene)hydrazinethioamide 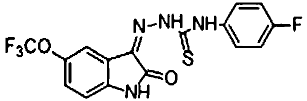 |
| 79 | (Z)-N-(4-bromophenyl)-2-(2-oxo-5-(trifluoromethoxy)indolin-3-ylidene)hydrazinethioamide 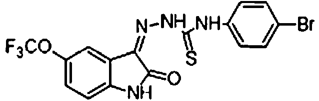 |

### <Experimental Examples>

### Experimental Example 1: Evaluation of the inhibitory effect of EDBA on DNA binding of EGR-1

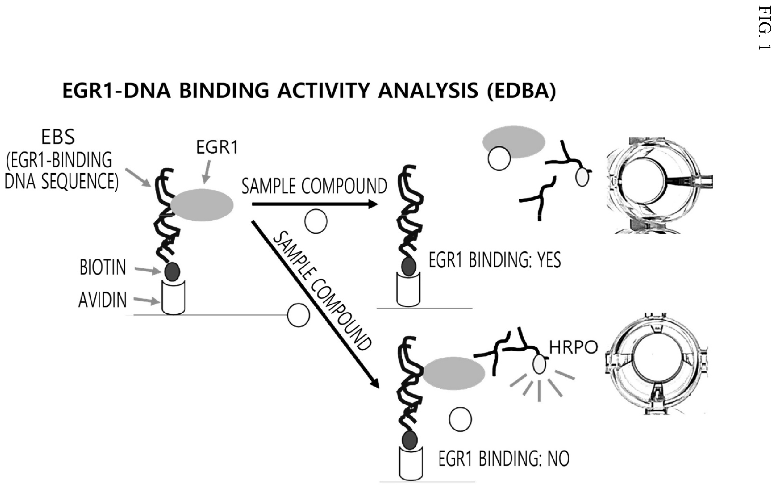

In this experiment, the inhibitory effect of the compounds of the present invention on EGR-1 binding activity was evaluated using an EGR-1 DNA binding activity assay (EDBA). The EDBA experimental method is briefly shown in FIG. 1.

### (1) Preparation of a biotin-labeled EGR-1 binding sequence (biotin-EBS) oligonucleotide

Single-stranded sense and antisense oligonucleotides of the following EGR-1 binding sequence (EBS) were prepared by outsourcing to Bioneer Co., Ltd. (Daejeon, Korea). In this case, the 5'-end of the sense oligonucleotide was labeled with biotin. The sense and antisense oligonucleotides were heated at 95 °C for 5 minutes and then left overnight at room temperature to hybridize the sense and antisense oligonucleotides. Hereinafter, the hybridized sense and antisense oligonucleotides are referred to as biotin-EBS.
Sense sequence: biotin-5'-TCGCCCCCGCTCGCCCCCGCTGGATCC-3'
Antisense sequence: 5'-GGATCCAGCGGGGGCGAGCGGGGGCGA-3'

### (2) Preparation of biotin-EBS oligonucleotide coated plate

Avidin protein was coated on a 96-well plate (purchased from Thermo Fisher Scientific), and biotin-EBS was added to the plate at a concentration of 0.5 pmol/50 µL/well, followed by reaction at 37 °C for 1 hour to allow biotin-EBS oligonucleotide to be attached to the plate. After washing three times with phosphate buffer saline containing 0.05% Tween 20 (PBS/Tween 20) to remove unattached biotin-EBS oligonucleotides, 200 µL of PBS/Tween 20 solution containing 2% bovine serum albumin (BSA) was added to each well, followed by reaction at 37 °C for 1 hour to block the surface of the plate where no oligonucleotide was attached.

### (3) Analysis of EGR-1 binding inhibition

The solution containing the sample compound and the EGR-1 protein was added to the biotin-EBS oligonucleotide coated plate prepared in step (2). The compounds were selected based on a principle that when the sample compound does not bind to the EGR-1 protein, the color development reaction occurs due to the reaction of the anti-EGR-1 antibody; however, when the sample compound binds to the EGR-1 protein and inhibits the binding of EGR-1 to DNA, no color development reaction occurs.

The compound sample (1 µL) was reacted with the EGR-1 overexpressing cell lysate containing 10 mM Tris, pH 7.5, 50 mM KCl, 2% BSA and 8% glycerol at room temperature for 30 minutes, and then added to a reaction well coated with biotin-EBS and further reacted at room temperature for one hour. After washing three times with PBS/Tween 20 solution, an EGR-1 antibody was added to the PBS solution supplemented with 2% BSA and 5 mM NaCl in an amount of 50 µL per each well. The mixture was incubated at room temperature for one hour, washed three times with PBS/Tween 20 solution, and then treated with horseradish peroxidase-conjugated secondary antibody (Goat anti-rabbit IgG antibody, Cell Signaling Technology company, Danvers, Mass., USA) diluted by 1:1000 at room temperature for one hour. After washing five times with PBS/Tween 20 solution, a substrate tetramethylbenzidine solution was added to each well in an amount of 50 µL. After reacting for 5 minutes, the reaction was stopped by adding 100 µL of 2 N sulfuric acid solution. The DNA binding inhibitory activity of EGR-1 was measured by absorbance at 450 nm according to Equation 1 below.$EGR -1 DNA binding inhibitory activity \left(%\right) = 100 - \left\{\left(As/Ap\right)\times 100\right\}$

As: absorbance of a sample prepared by reacting an EGR-1-overexpressing cell lysate with the compound.

Ap: absorbance of the sample treated only with EGR-1-overexpressing cell lysate.

The results of measuring the DNA binding inhibition activity of EGR-1 using EDBA for the compounds prepared in the above examples are shown in Table 2.

**Table 2**

| Compound No. | Inhibition Rate (%) | Compound No. | Inhibition Rate (%) |
|---|---|---|---|
| 1 | 23.60 | 41 | 49.00 |
| 2 | 25.70 | 42 | 53.00 |
| 3 | 20.73 | 43 | 23.00 |
| 4 | 16.27 | 44 | 5.67 |
| 5 | 6.33 | 45 | 9.33 |
| 6 | 27.00 | 46 | 11.33 |
| 7 | 23.33 | 47 | 35.33 |
| 8 | 38.73 | 48 | 52.00 |
| 9 | 42.17 | 49 | 50.33 |
| 10 | 35.47 | 50 | 27.33 |
| 11 | 18.67 | 51 | 28.67 |
| 12 | 28.97 | 52 | 9.00 |
| 13 | 25.57 | 53 | 10.00 |
| 14 | 17.80 | 54 | 12.67 |
| 15 | 37.07 | 55 | 11.00 |
| 16 | 40.57 | 56 | 12.67 |
| 17 | 25.57 | 57 | 40.10 |
| 18 | 33.80 | 58 | 46.97 |
| 19 | 47.77 | 59 | 40.17 |
| 20 | 44.93 | 60 | 48.07 |
| 21 | 70.20 | 61 | 43.03 |
| 22 | 51.33 | 62 | 10.00 |
| 23 | 67.63 | 63 | 48.77 |
| 24 | 42.00 | 64 | 37.90 |
| 25 | 76.13 | 65 | 43.03 |
| 26 | 49.67 | 66 | 11.70 |
| 27 | 44.00 | 67 | 25.90 |
| 28 | 28.67 | 68 | 17.80 |
| 29 | 27.50 | 69 | 14.17 |
| 30 | 32.70 | 70 | 29.60 |
| 31 | 42.73 | 71 | 29.93 |
| 32 | 40.93 | 72 | 19.57 |
| 33 | 37.77 | 73 | 29.80 |
| 34 | 46.00 | 74 | 40.60 |
| 35 | 55.33 | 75 | 39.27 |
| 36 | 46.33 | 76 | 17.23 |
| 37 | 36.33 | 77 | 37.37 |
| 38 | 51.33 | 78 | 33.93 |
| 39 | 47.33 | 79 | 42.27 |
| 40 | 27.33 | - | - |

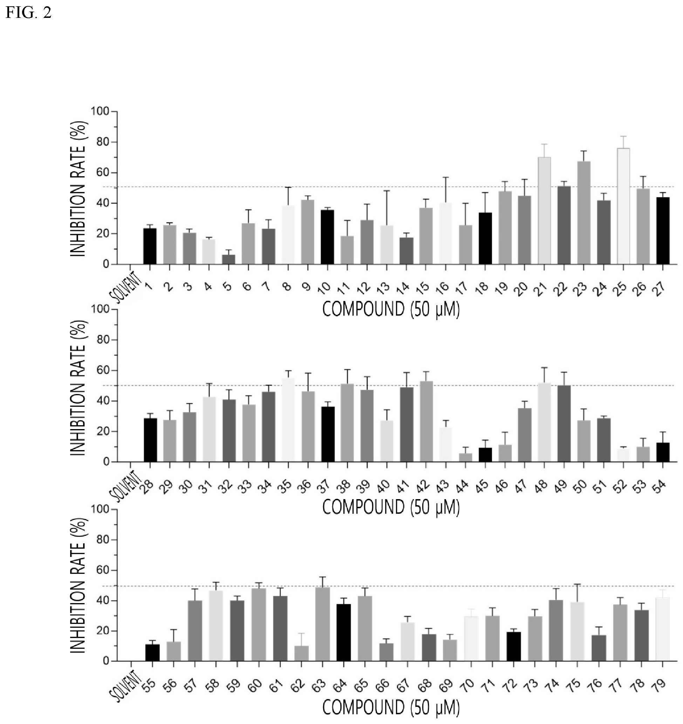

In addition, the inhibition rates of EGR-1 DNA binding for the compounds (a total of 79 compounds) represented by Formula 1 were measured and the results are shown in FIG 2 as a graph. Among the total of 79 compounds represented by Formula 1, Compounds 21, 23, and 25 showed the best DNA binding inhibitory activity of EGR-1.

### Experimental Example 2: In vitro cytotoxicity assay of the compounds of the present invention

In this experiment, the in vitro cytotoxicity of the compounds represented by Formula 1 was analyzed. Specifically, for the 79 compounds represented by Formula 1, cell viability was measured in HaCaT keratinocyte cells using a Cell Counting Kit-8 (CCK-8; Sigma-Aldrich), and compounds exhibiting a cell viability of 80% or less relative to a solvent-treated control were considered to be cytotoxic. The HaCaT keratinocyte cells were seeded at 1×10⁴ cells per well in a 96-well cell culture plate, and each compound was treated at a concentration of 100 µM and incubated for 24 hours, after which 10 µL of CCK-8 solution was added. After 2 hours, the absorbance of the culture medium was measured at 450 nm, and cell viability was calculated according to Equation 2 below.$Cell viability \left(%\right) = \left(As/Av\right) \times 100$

As: absorbance of the sample treated with the compound.

Av: absorbance of the sample treated with only solvent.

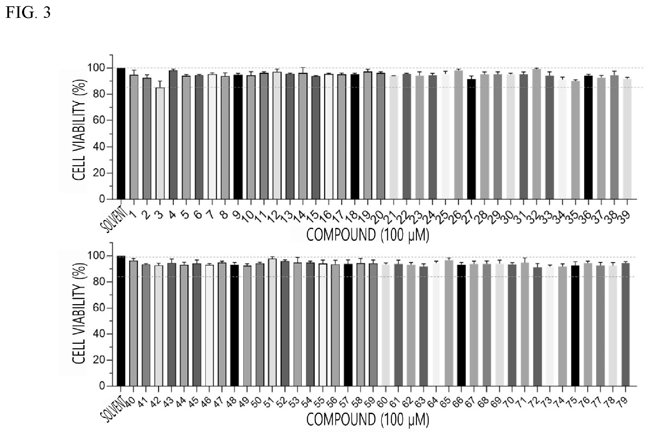

As a result of the experiment, as shown in FIG 3, when the cell viability of the control group treated with 0.1% dimethyl sulfoxide (DMSO) was regarded as 100%, all 79 compounds represented by Formula 1 of the present invention showed a cell viability of 90% or more. These results suggest that the compounds represented by Formula 1 of the present invention do not exhibit toxicity to human keratinocyte cells at a concentration of 100 µM.

### Experimental Example 3: Prediction of an EGR-1 binding site of the compounds of the present invention

In this experiment, the EGR-1 binding site of the compounds of the present invention was predicted using molecular docking.

To determine whether the compound is bound to EGR-1, an in silico molecular docking experiment was performed using Autodock vina program [J. Comp. Chem. 31: 455 (2010)] and Sybyl program (Tripos, St. Louis, MO).

The tertiary structure of EGR-1 has been registered in several types of Protein Data Bank (PDB). Among them, 5n14.pdb is a structure determined by nuclear magnetic resonance (NMR) spectroscopy and includes only 25 amino acid residues. 4x9j.pdb and 4r2a.pdb are structures determined by X-ray crystallography, and since 4r2a.pdb contains slightly more amino acid residues than 4x9j.pdb, the present experiment used 4r2a.pdb [Genes Dev. 28: 2304 (2014)]. The structure of 4r2a.pdb includes amino acid residues between E335 and D423, thus including ZnF1 (338 to 362), ZnF2 (368 to 390), and ZnF3 (396 to 418). Since 4r2a.pdb does not include a ligand, the binding site was determined using the MOLCAD module included in the Sybyl program (F377, S378, H382, T385, H386, T389, R407). The binding site determined in this way was included in the docking box by using the GRIDbox module of the AutoDockTools program [J. Comp. Chem. 31: 455 (2010)], with the size of the docking box set to 24, 24, and 24 for x, y, and z, respectively, and the center of the docking box set to -8.194, -4.889, and -6.333 for x, y, and z, respectively, and an in silico docking experiment was performed using the Autodock vina program.

Docking results were analyzed using the LigPlot program provided by the European Bioinformatics Institute [Protein. 1999; 37: 228], and images of three-dimensional structures were generated using the PyMOL program (The PyMOL Molecular Graphics System, Version 1.0r1, Schrφdinger, LLC). To obtain the apo-protein of EGR-1, oligonucleotides included in 4r2a.pdb were removed using the Sybyl program.

The three compounds having the highest EGR-1 DNA binding inhibition rate in Experimental Example 1 (i.e., Compounds 21, 23, and 25) were selected to analyze their binding states with EGR-1. The tertiary structures of Compounds 21, 23, and 25 were obtained using a PyMOL program by using 1-[(7-fluoro-2-hydroxy-1H-indol-3-yl)imino]-3-(4-fluorophenyl)thiourea (PubChem CID=136269038) registered as a tertiary structure on PubChem (https://pubchem.ncbi.nlm.nih.gov/) as a template. The obtained tertiary structures were subjected to energy minimization using a UCSF Chimera program to obtain a tertiary structure having the lowest energy [J Comput Chem. 2004 Oct;25(13):1605].

Since the AutoDock vina program performs docking experiments nine times, nine compound 21 complexes bound to EGR-1 were obtained. The binding energies of these nine complexes ranged from -6.9 kcal/mol to -5.7 kcal/mol, indicating stable binding. Among these nine complexes, the complex with the lowest binding energy showed a pose where Compound 21 was stably bound to EGR-1, and thus this was determined as the result of Compound 21 binding to EGR-1. This result was analyzed using the LigPlot program to analyze the interaction between Compound 21 and amino acid residues of EGR-1.

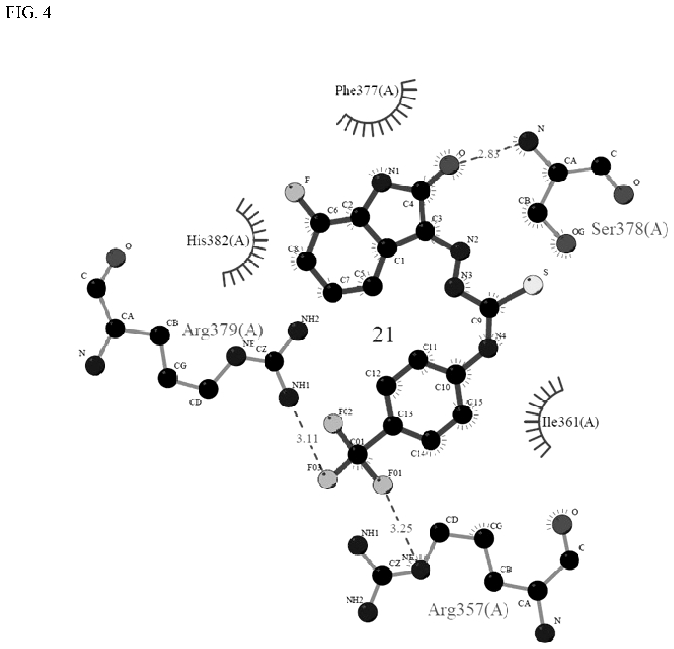

FIG. 4 shows the interactions between Compound 21 and amino acid residues of EGR-1. Referring to FIG. 4, Compound 21 shows hydrophobic interactions with I361, F377, and H382, and forms hydrogen bonds with R357, S378, and R379. The distances of the hydrogen bonds with R357, S378, and R379 are 3.25 Å, 2.83 Å, and 3.11 Å, respectively. These residues are included in the binding site determined using an MOLCAD module included in the Sybyl program mentioned above, and thus, it was determined that Compound 21 was well bound to EGR-1.

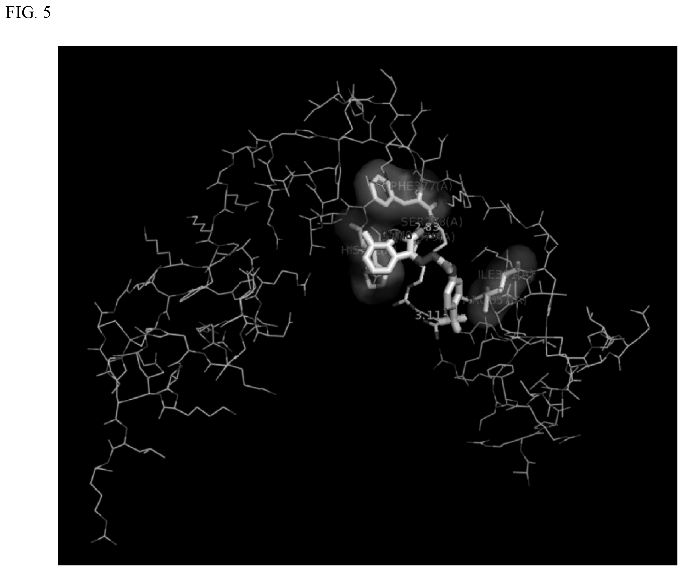

In addition, FIG. 5 is an image of a three-dimensional structure of the complex between Compound 21 and EGR-1 predicted by using the PyMOL program. Referring to FIG 5, it was confirmed that Compound 21 represented in white was well bound within EGR-1 composed of green amino acids. The results of forming hydrogen bonds with S378 and R379 are shown as distances of 2.83 Å and 3.11 Å on the three-dimensional image.

The same docking experiment as described above was performed using Compound 23. All nine complexes of Compound 23 bound to EGR-1 were obtained. The binding energies of the nine complexes ranged from -6.7 kcal/mol to -5.8 kcal/mol, indicating that they are stably bound. Among the nine complexes, the complex exhibiting the lowest binding energy showed a stable binding pose of Compound 23 to EGR-1. Accordingly, this complex was determined to represent the binding mode of Compound 23 to EGR-1.

The results were analyzed for interactions between Compound 23 and residues of EGR-1 using the program LigPlot.

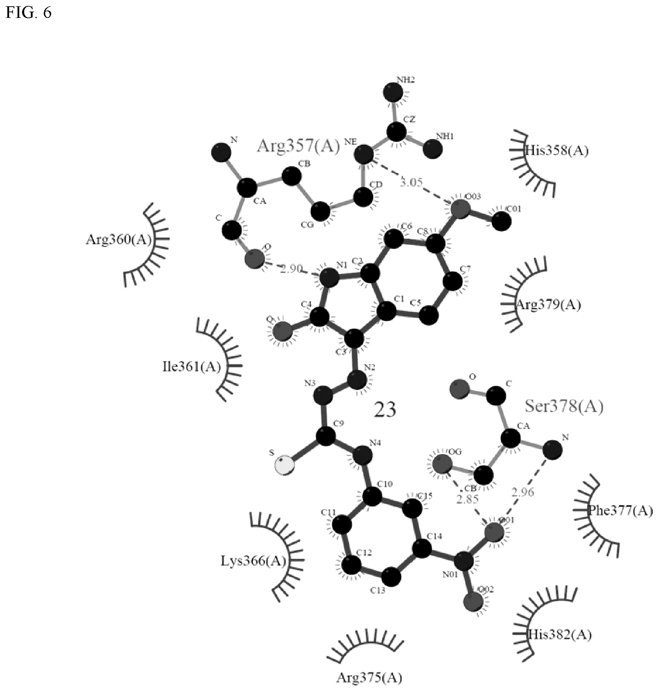

FIG. 6 shows the interactions between Compound 23 and amino acid residues of EGR-1. Referring to FIG. 6, Compound 23 shows hydrophobic interactions with eight residues, H358, R379, F377, H382, R375, K366, I361, and R360, and forms a total of four hydrogen bonds at two sites with residues R357 and S378. Distances of the hydrogen bonds with R357 are 2.90 Å and 3.95 Å, and distances of the hydrogen bonds with S378 are 2.85 Å and 2.96 Å, respectively. These residues are included in the binding site determined using the MOLCAD module included in the Sybyl program mentioned above, and it was determined that Compound 23 was well bound to EGR-1.

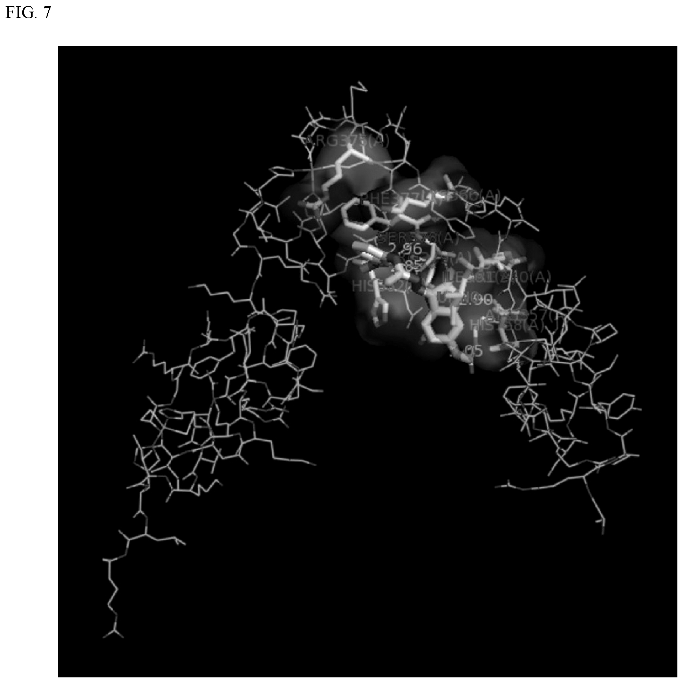

In addition, FIG. 7 is an image of a three-dimensional structure of the complex between Compound 23 and EGR-1 predicted by using the PyMOL program. Referring to FIG. 7, it was confirmed that Compound 23 represented in white was well bound within EGR-1 composed of green amino acids.

The same docking experiment as described above was performed using Compound 25. All nine complexes of Compound 25 bound to EGR-1 were obtained. The binding energies of the nine complexes ranged from -6.4 kcal/mol to -5.3 kcal/mol, indicating that they are stably bound. Among the nine complexes, the complex exhibiting the lowest binding energy showed a stable binding pose of Compound 25 to EGR-1. Accordingly, this complex was determined to represent the binding mode of Compound 25 to EGR-1.

The results were analyzed for interactions between Compound 25 and the residues of EGR-1 using the program LigPlot.

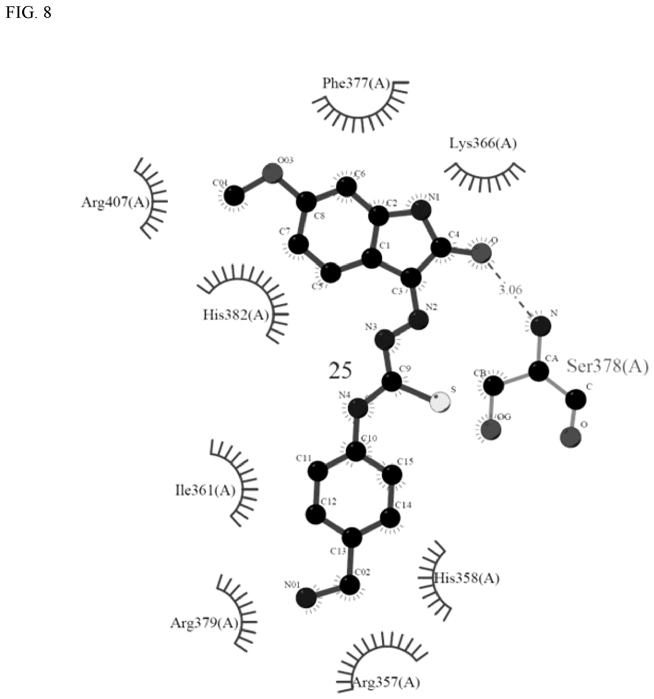

FIG. 8 shows the interactions between Compound 25 and amino acid residues of EGR-1. Referring to FIG. 8, compound 25 has hydrophobic interactions with eight residues (F377, K366, R407, H382, I361, R379, R357, and H358), and forms one hydrogen bond with residue S378. A distance of the hydrogen bond with S378 is 3.06 Å. These residues are included in the binding site determined using the MOLCAD module included in the Sybyl program mentioned above, and it was determined that Compound 25 was well bound to EGR-1.

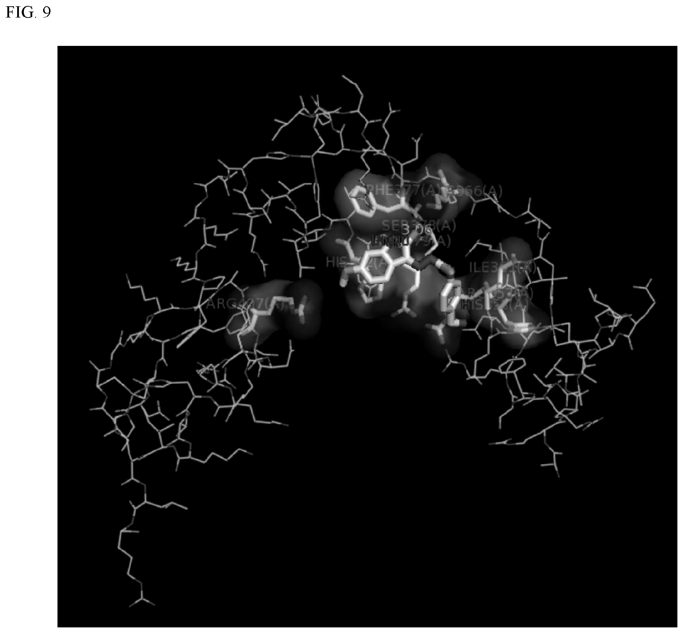

In addition, FIG. 9 is an image of a three-dimensional structure of the complex between Compound 25 and EGR-1 predicted by using the PyMOL program. Referring to FIG. 9, it was confirmed that Compound 25 represented in white was well bound within EGR-1 composed of green amino acids. The result of forming hydrogen bonds with S378 shows a distance of 3.06 Å on the three-dimensional image.

### Experimental Example 4: In silico ADME analysis of the compounds of the present invention

In this experiment, absorption-distribution-metabolism-excretion (ADME) was predicted in silico using the method provided by SwissADME [Sci. Rep. 7, 42717(2017)].

Compound 21 is moderately soluble in water and has a good gastrointestinal absorption property, but may interact with other drugs using the enzymes as substrates because of its inhibitory effects on CYP1A2, CYP2C9, and CYP3A4 among cytochrome P450 isoenzymes. However, since Compound 21 does not inhibit CYP2C19 and CYP2D6, it will not interact with other drugs using these enzymes as substrates. Although a saturation value, represented by the fraction of sp³ carbons relative to the total number of carbon atoms in Compound 21, was outside the recommended range, which may adversely affect bioavailability, the other parameters, including molecular size, molecular polarity, solubility, flexibility, and lipophilicity, all satisfied the recommended criteria. Accordingly, the bioavailability of Compound 21 was considered to be favorable.

Compound 23 is well soluble in water and has a low gastrointestinal absorption rate, but inhibits the activity of CYP1A2, CYP2C9, and CYP3A4 among cytochrome P450 isoenzymes, so that there is a possibility of interaction with other drugs using these enzymes as substrates. However, Compound 23 does not inhibit CYP2C19 or CYP2D6, so that there is no possibility of interaction with other drugs using these enzymes as substrates. The bioavailability of Compound 23 was good because its saturation degree and polarity value were out of range, while other factors such as molecular size, solubility, flexibility, and lipophilicity satisfied reference values.

Compound 25 is well soluble in water and has good gastrointestinal absorption, and thus may interact with other drugs using them as substrates due to the inhibitory effects on CYP1A2, CYP2C9, and CYP3A4 among cytochrome P450 isoenzymes. However, Compound 25 does not inhibit CYP2C19 and CYP2D6, so that it is unlikely to interact with other drugs using them as substrates. The bioavailability of Compound 25 was good because factors other than saturation, such as molecular size, polarity, solubility, flexibility, and lipophilicity, all satisfied the reference values.

### Experimental Example 5: In silico toxicity analysis of the compounds of the present invention

In this experiment, toxicity was predicted in silico. Toxicity was evaluated using a method provided by Pro-Tox-II [Nucleic Acids Res 46(W1), W257 (2018)].

Compound 21 had an LD50 of 2100 mg/kg and showed toxicity in all areas except for liver toxicity, carcinogenicity, aryl hydrocarbon receptor, and mitochondrial membrane potential pathway.

Compound 23 had an LD50 of 2100 mg/kg and showed toxicity in all areas except for immunotoxicity, mutagenicity, aryl hydrocarbon receptor, and mitochondrial membrane potential pathway.

Compound 23 had an LD50 of 2100 mg/kg and showed toxicity in all areas except for liver toxicity, carcinogenicity, mutagenicity, and mitochondrial membrane potential pathway.

### Experimental Example 6: Off-target effects of the compounds of the present invention

In this experiment, target proteins were predicted in silico to avoid targeting proteins other than inhibiting the binding of EGR-1 to DNA. The prediction of target proteins was performed using a method provided by SwissTarget [Nucleic Acids Research 47(W1), W357 (2019)].

Among the top ten target proteins predicted as non-specific targets of Compound 21, the highest probability was 0.109, indicating that there is no non-specific target effect.

Among the top ten target proteins predicted as non-specific targets of Compound 23, the highest probability was 0.109, indicating that there is no non-specific target effect.

Among the top ten target proteins predicted as non-specific targets of Compound 25, the highest probability was 0.097, indicating that there is no non-specific target effect.

### Experimental Example 7: Inhibition effect of the compounds of the present invention on EGR-1 binding using an EMSA technique

When a nucleic acid and a protein are bound to each other, the complex of the nucleic acid and the protein moves more slowly than the nucleic acid alone, so that in an EMSA, a band of the nucleic acid bound to the protein appears higher than a band of only the nucleic acid. Using this experimental principle, compounds (Compound 21, 23, and 25) that showed the highest inhibition rate of EGR-1 DNA binding in Experimental Example 1 were selected, and the inhibitory effect of the compounds of the present invention on EGR-1 binding was evaluated by analyzing electrophoretic mobility shift.

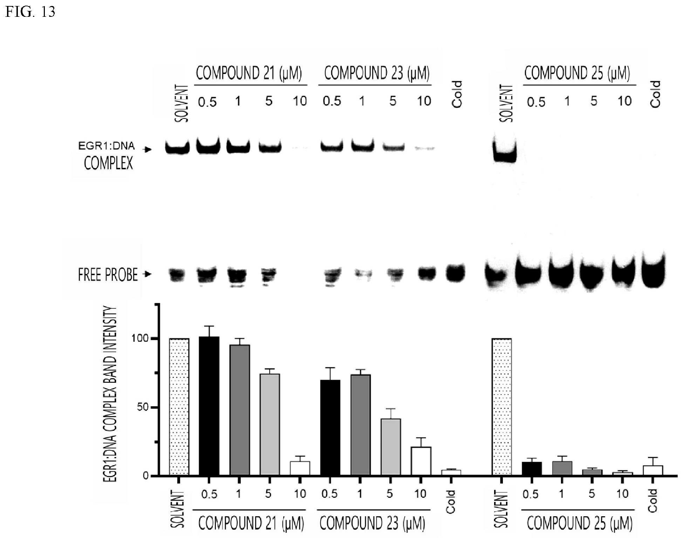

In detail, the protein-DNA binding was analyzed by purchasing a LightShift Chemiluminescence EMSA kit (ThermoFisher Scientific, Waltham, MA, USA). The probe sequence for EGR-1 was synthesized by Macrogen company (Seoul, Korea) by ordering a deoxynucleotide labeled with biotin at the EGR-1 binding sequence (5'-biotin-AGA GTG TGT CTC CTT CGC ACA CAT C-3'). The EGR-1 protein used in the experiment was an Sf21 insect cell extract overexpressing human EGR-1 protein as described in Yeo et al. [Journal of Investigative Dermatology 2021; 141:1851-1855]. Wild-type Sf21 cell extract was used as a negative control. After reacting 3 µg of EGR-1 overexpressing Sf21 cell extract, 1 µl of the compound of the present invention at a concentration of 20 mM, 50 fmol of the biotin-labeled EGR-1 binding deoxyoligonucleotide probe, and 1 µg poly(dI-dC) (Amersham Pharmacia Biotech Inc) for 20 minutes, electrophoresis was performed on a 6% non-denaturing polyacrylamide gel. The electrophoresed sample was transferred to a nylon membrane (Immobilon-Ny+ Transfer membrane, Millipore) and soaked in 5% skim milk solution for 30 minutes. Then, horseradish peroxidase-conjugated streptavidin was added and reacted for 15 minutes, and after washing unbound proteins from the membrane, the EGR-1-DNA complex was detected on X-ray film using an enhanced chemiluminescence (ECL) detection system (ThermoFisher Scientific). The intensity of the EGR-1-DNA complex band was analyzed using the ImageJ program (National Institute of Health, USA), and the intensity of the sample without the compound was set to 100%, and relative intensities were measured. The experimental results are shown in FIG. 13.

FIG. 13 shows the results of an EMSA after administration of compounds (Compounds 21, 23, and 25) to keratinocytes according to the present invention. As shown in FIG. 13, all three selected compounds (Compounds 21, 23, and 25) inhibited DNA binding of EGR-1 in a concentration-dependent manner.

### Experimental Example 8: Inhibitory effect on mRNA expression of an EGR-1 target gene TSLP

To determine whether the compounds of the present invention (Compounds 21, 23, and 25) inhibit expression of TSLP, IL-1β, and IL-6 genes, which are EGR-1 target genes playing a key role in atopic dermatitis exacerbation, the inhibitory effect of the compounds on mRNA expression of an EGR-1 target gene TSLP was evaluated by reverse transcription-polymerase chain reaction (RT-PCR) in HaCaT keratinocyte cells.

Specifically, HaCaT keratinocyte cells (purchased from Cell Lines Service, Eppelheim, Germany) were passaged at a seeding density of 1x10⁶ cells per 100 mm cell culture dish in Dulbecco's modified Eagle's medium (Invitrogen Life Technologies) containing 10% fetal bovine serum (Invitrogen Life Technologies) and antibiotic-antifungal agent mix solution (Invitrogen Life Technologies), once every two days, and cultured at 37 °C in a 5% CO₂ incubator.

HaCaT cells were treated with the compounds of the present invention (Compound 21, Compound 23, and Compound 25) at concentrations of 0, 5, 10, and 20 µM, respectively, for 30 minutes, followed by treatment with TNFα in a concentration of 10 ng/mL for 12 hours, after which the cells were harvested. Total RNA was extracted using TRIzol RNA isolation reagents (TRIzol Life Technologies Korea). A total of 0.5 µg of total RNA was reverse transcribed to synthesize complementary DNA (cDNA) according to the method recommended by the manufacturer using an iScript cDNA synthesis kit (Bio-Rad, Hercules, Calif., U.S.A.). PCR was performed using 0.0125 µg of double-stranded cDNA. Primers for gene amplification were synthesized from Macrogen (Seoul, Korea), and primer sequences are shown in Table 3 below.

**Table 3**

| Gene | Primer Sequence | |
|---|---|---|
| TSLP | Forward | 5'-TAG CAA TCG GCC ACA TTG CCT-3' |
| | Reverse | 5'-GAA GCG ACG CCA CAA TCC TTG-3' |
| IL1β | Forward | 5'-AAA CAG ATG AAG TGC TCC TTC CAG G-3' |
| | Reverse | 5'-TGG AGA ACA CCA CTT GTT GCT CCA-3' |
| IL6 | Forward | 5'-GCC TTC GGT CCA GTT GCC TT-3' |
| | Reverse | 5'-AGTGCCTCTTTGCTGCTTTCA C-3' |
| IL23 | Forward | 5'-GAC ACA TGG ATC TAA GAG AAG AG-3' |
| | Reverse | 5'-AAC TGA CTG TTG TCC CTG AG-3 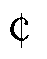 |
| CXCL1 | Forward | 5'-ATG GCC CGC CGC GCT GCT CTC TCC-3' |
| | Reverse | 5'-GTT GGA TTT GTC ACT GTT CAG-3 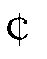 |
| CCL2 | Forward | 5'-CTT CTG TGC CTG CTG CTC ATA G-3' |
| | Reverse | 5'-CTG GAC AAG CAA ACC CAA AC-3 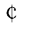 |
| CCL5 | Forward | 5'-ACA GGT ACC ATG AAG GTC TC-3' |
| | Reverse | 5'-GCA AAT TTG TGT AAG TTC AGG-3 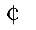 |
| GAPDH | Forward | 5'-ACC CAC TCC TCC ACC TTT G-3' |
| | Reverse | 5'-CTC TTG TGC TCT TGC TGG G-3' |

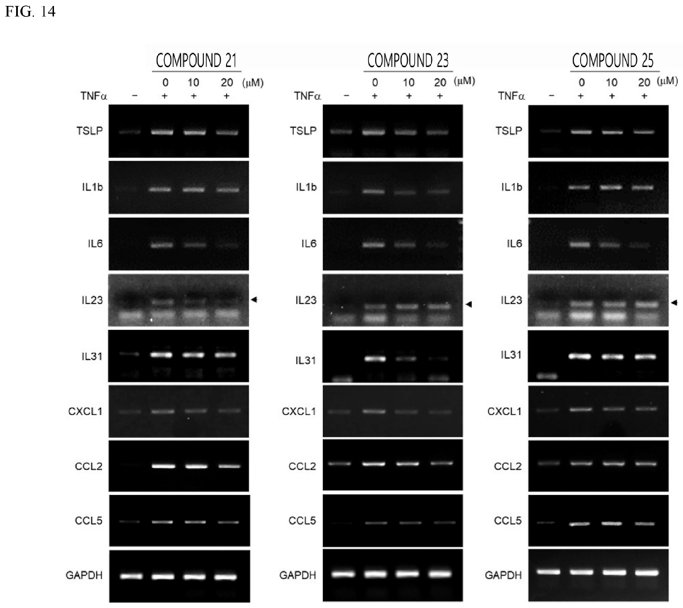

Glyceraldehyde-3-phosphate dehydrogenase (GAPDH), a housekeeping gene, was used as the control group. The double-stranded cDNA was denatured at 95 °C for 5 minutes and then subjected to cycles of reaction at 94 °C for 1 minute, 65 °C for 2 minutes, and 70 °C for 1 minute, repeated 30 times. The final product of PCR was electrophoresed on 1% agarose gel, stained with ethidium bromide (EtBr), and confirmed. The experimental results are shown in FIG. 14.

FIG. 14 shows the results of RT-PCR performed after administration of three compounds (Compound 21, Compound 23, and Compound 25) according to the present invention to keratinocytes. As shown in FIG. 14, expression levels of TSLP, IL-1β, and IL-6 mRNAs, which are EGR-1 target genes induced by TNFα stimulation, were decreased dose-dependently by the compounds (Compound 21, Compound 23, and Compound 25).

### Experimental Example 9: Therapeutic Effect on atopic dermatitis in an induced animal model of atopic dermatitis

### (1) Construction of an animal model of atopic dermatitis.

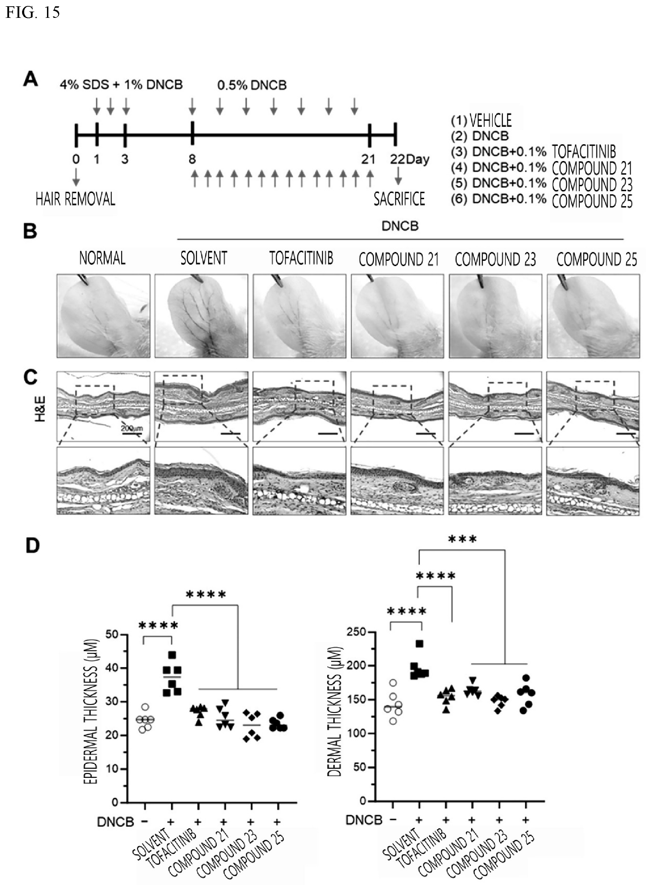

Male BALB/c mice of 6 to 8 weeks old were adapted in an animal laboratory maintained at a temperature of 20±2 °C, humidity of 50±10%, and a light cycle of 12 hours for one week. In the atopic dermatitis control group, 4% sodium dodecyl sulfate (SDS) was applied to the ear portion of the mouse to increase skin sensitivity, followed by application of a mixture solution of acetone and olive oil (3:1, v/v) containing 1% 2,4-dinitrochlorobenzene (DNCB; Sigma Chemical Co., St. Louis, Mo., USA) on the ear portion of the mouse once daily for three days. After a rest period of four days, 100 µL of 4% SDS and 0.5% DNCB was applied to the same part five times per week for two weeks to induce atopic dermatitis. About 2 to 3 hours later, (i) drug solvent (atopic positive control), (ii) 0.1% Tofacitinib (JAK kinase inhibitor; drug positive control), (iii) 0.1% Compound 21, (iv) 0.1% Compound 23, or (v) 0.1% Compound 25 was applied to the same part, and the symptom amelioration effect was observed (see FIG. 15A).

### (2) Therapeutic Effect on atopic dermatitis by topical application of the compounds of the present invention

### (2-1) Macroscopic amelioration of skin inflammation

On the day of experiment termination (day 22), all experimental mice were sacrificed and macroscopic skin clinical symptoms were observed. In mice treated with only DNCB, there was a significant improvement in skin condition in mice treated with Compounds 21, 23, and 25 as compared to that of the positive control drug, tofacitinib, due to inflammatory response on the skin (see FIG. 15B).

### (2-2) Histopathological amelioration of the skin

In atopic dermatitis, repeated inflammatory responses increase the proliferation of keratinocytes and cause an imbalance in the differentiation process into keratin, resulting in increased skin thickness and infiltration of a large number of primary immune cells into the tissue to promote inflammatory responses.

### (a) Hematoxylin and eosin (H&E) staining

The ear skin of the experimental mice was removed and fixed in a 100% acetone solution for 24 hours to prepare paraffin blocks. The paraffin blocks were thinly cut to a thickness of 5 µm, and then hematoxylin/eosin staining was performed using an H&E staining kit (Abcam, Cambridge, UK). As a result, it was observed that the thicknesses of the epidermis layer and dermis layer were significantly increased compared to normal tissue in the experimental group skin tissue induced with atopic dermatitis by treating DNCB compared to the normal control group (see FIG. 15C).

When the change in skin thickness was quantitatively measured using the stained skin tissue, the epidermis of normal skin tissue had a thickness of 24.47±2.353 µm and the dermis had a thickness of 143.2±19.61 µm, whereas the epidermis of the tissue treated with only DNCB had a thickness of 37.27±4.393 µm and the dermis had a thickness of 197.5±17.83 µm. The mouse treated with tofacitinib as a control drug had an epidermis having a thickness of 27.09±1.719 µm and a dermis having a thickness of 154.5±11.32 µm. The skin tissues injected with the compounds of the present invention (Compounds 21, 23, and 25) had an epidermal thickness of 25.22±2.993, 22.92±3.617, and 23.46±1.530 µm, respectively, and a dermal thickness of 163.4±7.905, 147.5±8.302, and 157.8±17.05 µm, respectively (see FIG. 15D).

### (b) Inhibitory effect on mast cell infiltration

During the inflammatory response, when inflammatory cells such as mast cells, neutrophils, and macrophages infiltrate into a site of skin inflammation, various inflammatory mediators are released to intensify the inflammatory response. Thus, the inhibitory effect of the compounds of the present invention (Compounds 21, 23, and 25) on mast cell infiltration was evaluated.

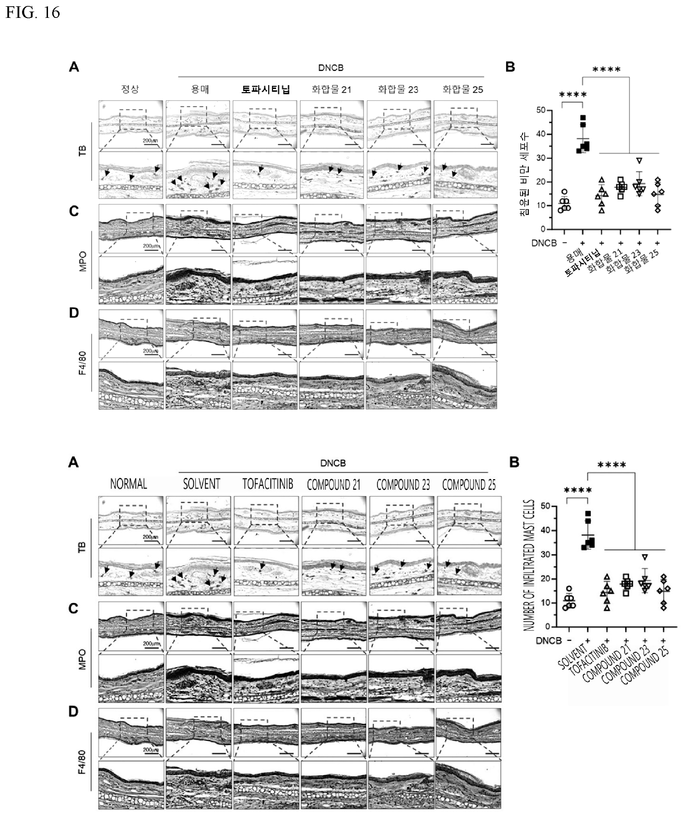

To measure the number of mast cells infiltrated into the inflammatory site, 0.5% toluidine blue (TB) staining was performed on paraffin sections prepared in step (a). As a result, it was observed that mast cell infiltration was significantly increased in atopic dermatitis tissue induced by DNCB compared to the normal group, and mast cell infiltration was significantly reduced in experimental groups treated with tofacitinib as a control drug or compounds according to the present invention (Compounds 21, 23, and 25) compared to the positive control group (see FIG. 16A).

The number of infiltrated mast cells was quantified and the results showed that the number of mast cells infiltrated in normal skin tissue was 11±3 per 2.5 cm², whereas it was 38±6 in atopic dermatitis induced by DNCB. In contrast, when topically applied with a control drug (i.e., tofacitinib), the number of mast cells was 14±5. The number of mast cells in the skin tissues treated with compounds of the present invention (Compounds 21, 23, and 25) were 18±2, 19±5, and 15±5, respectively (see FIG. 16B).

Neutrophils and macrophages were analyzed by immunohistochemistry using a myeloperoxidase (MPO) antibody and an F4/80 antibody, respectively. The paraffin sections prepared in (a) above were treated with xylene to remove the paraffin, hydrated with ethanol, reacted at 70 °C for 20 minutes in 1 mM EDTA (pH 8.0), and then reacted at room temperature for 1 hour in blocking solution containing 7% goat serum. Then, each tissue section was added with an antibody against F4/80 as a macrophage marker (Cell Signaling Biotechnology, Beverly, USA) and an antibody against MPO as a neutrophil marker (DAKO, Glostrup, Denmark), and incubated overnight at 4 °C. After washing the tissue slices with phosphate buffer, horseradish peroxidase (HRPO)-conjugated secondary antibodies were added and reacted at room temperature for 1 hour. After washing the tissue slices with phosphate buffer, they were reacted with diaminobenzidine tetrahydrochloride substrate for 5 minutes, followed by counterstaining with hematoxylin and eosin (H&E).

As a result, it was observed that the expression of MPO (neutrophil marker) (see FIG. 16C) and F4/80 (macrophage marker) (see FIG. 16D) in atopic dermatitis tissue induced by DNCB was significantly increased compared to normal group, whereas the expression was decreased similar to normal tissue in experimental groups treated with tofacitinib or Compounds 21, 23, and 25.

As a result of the above experiment, it was confirmed that the compounds of the present invention (Compounds 21, 23, and 25) have an effect similar to that of the control drug, i.e., tofacitinib, in reducing infiltration of mast cells, neutrophils, and macrophages into inflammatory sites which exacerbate atopic dermatitis symptoms.

### (c) TSLP inhibition and filaggrin restoration effect

TSLP is a member of the IL-7 family of cytokines that are secreted by various non-immune cells such as epithelial cells, smooth muscle cells, fibroblasts and keratinocytes. TSLP is produced in large amounts from keratinocytes by inflammatory responses in skin, promotes differentiation of T-lymphocytes, activates various inflammatory cells, stimulates cutaneous sensory nerves to cause itching, and thus is an important pro-inflammatory cytokine for exacerbating atopic dermatitis.

Filaggrin is a protein that binds to keratin in the stratum corneum of skin tissue and plays a role in generating an important skin barrier for skin moisturization and producing natural moisturizing factor. As skin inflammation worsens, filaggrin production decreases, resulting in impairment of the skin barrier. Consequently, xerosis and pruritus are aggravated, facilitating the penetration of various microorganisms, fine particulate matter, allergens, and the like, thereby exacerbating the symptoms of atopic dermatitis.

Accordingly, the effects of the compounds of the present invention (Compounds 21, 23, and 25) on inhibition of TSLP production and restoration of filaggrin production were analyzed. Specifically, after removing paraffin from tissue sections as described in (b), the tissue sections were reacted with blocking solution containing 1% bovine serum albumin for one hour. Then, each tissue section was added with a TSLP antibody (Novus Biologicals, Centennial, USA) and an FLG antibody (Santa Cruz Biotechnology, Dallas, TX, USA) and reacted at 4 °C overnight. After washing the sections with phosphate buffer, they were reacted with secondary antibodies conjugated with rhodamine red-X (Jackson ImmunoResearch Laboratories, West Grove, Pa., USA; diluted to 1:300) at 25 °C for one hour. For control staining, nuclei DNA was stained with Hoechst 33258 solution, and then fluorescent images were analyzed using an EVOS FL fluorescence microscope (Advanced Microscopy Group; Bothell, Wash., USA).

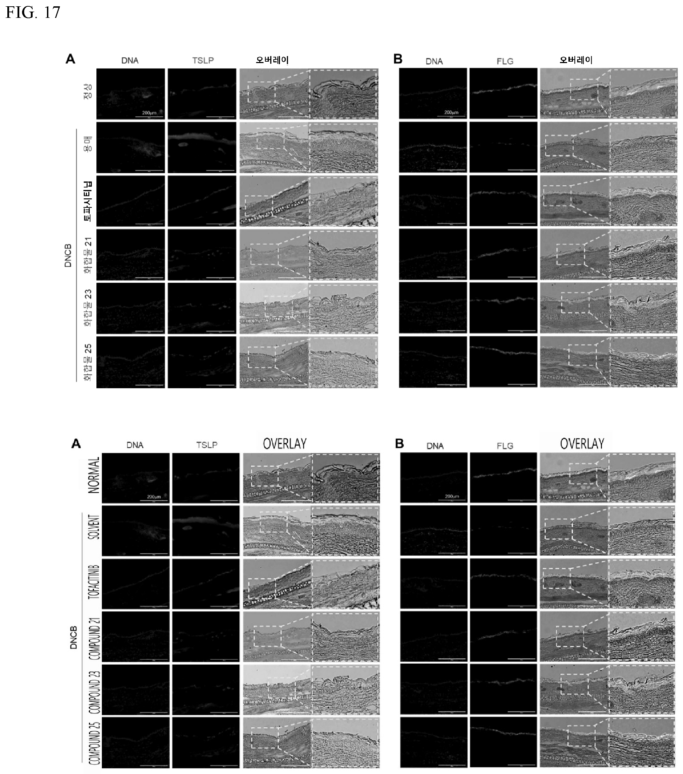

As a result, it was observed that the expression of TSLP in tissues of atopic dermatitis induced by DNCB was significantly increased compared to normal group, and the expression of TSLP was significantly decreased in experimental groups treated with the control drug (tofacitinib) or the compounds of the present invention (Compounds 21, 23, and 25) compared to positive control group (FIG. 17A). In addition, although filaggrin was significantly reduced in tissues of atopic dermatitis, it was observed that the expression of filaggrin was recovered to a level similar to normal tissue in experimental groups treated with the control drug (tofacitinib) or the compounds of the present invention (Compounds 21, 23, and 25) (FIG. 17B).

As a result of the experiment, it was confirmed that the compounds of the present invention (Compound 21, Compound 23, and Compound 25) have an effect of reducing expression of TSLP which exacerbates atopic dermatitis symptoms, similar to that of the positive control drug, tofacitinib, while restoring production of filaggrin protein essential for skin barrier recovery.

The foregoing description of the present invention is provided for illustrative purposes only, and those skilled in the art to which the present invention pertains will understand that various modifications can be easily made without departing from the technical spirit or essential characteristics of the present invention. Therefore, the embodiments described above should be understood in all respects as illustrative and not restrictive.

## Claims

1. A pharmaceutical composition for preventing, ameliorating, or treating atopic dermatitis comprising a compound represented by Formula 1 below, an isomer thereof, or a pharmaceutically acceptable salt thereof as an active ingredient: 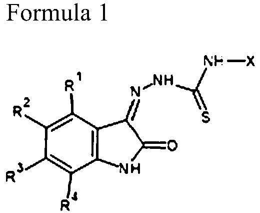
wherein in Formula 1,
X is hydrogen, C₁-C₆ alkyl, -CH₂-C₆₋₁₀ aryl, or 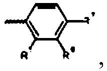
wherein C₁-C₆ alkyl and -CH₂-C₆₋₁₀ aryl are each independently unsubstituted or substituted with 1 to 3 halogens,
R¹ is hydrogen or halogen,
R² is hydrogen, halogen, hydroxy, C₁-C₆ alkyl, or C₁-C₆ alkoxy, wherein C₁-C₆ alkyl and C₁-C₆ alkoxy are each independently unsubstituted or substituted with 1 to 3 halogens,
R³, R⁴, and R⁵ are each independently hydrogen, halogen, hydroxy, or C₁-C₆ alkoxy, wherein C₁-C₆ alkoxy is unsubstituted or substituted with 1 to 3 halogens,
R⁶ is hydrogen, halogen, hydroxy, -NO₂, C₁-C₆ alkyl, or C₁-C₆ alkoxy, wherein C₁-C₆ alkyl and C₁-C₆ alkoxy are each independently unsubstituted or substituted with 1 to 3 halogens, and
R⁷ is hydrogen, halogen, hydroxy, -NO₂, -CN, C₁-C₆ alkyl, or C₁-C₆ alkoxy, wherein C₁-C₆ alkyl and C₁-C₆ alkoxy are each independently unsubstituted or substituted with 1 to 3 halogens.

2. The pharmaceutical composition of claim 1, wherein the compound is any one selected from the group consisting of the following compounds, an isomer thereof, or a pharmaceutically acceptable salt thereof:
N-(3-(trifluoromethyl)phenyl)hydrazinethioamide
N-(4-fluorophenyl)hydrazinethioamide;
N-(4-bromophenyl)hydrazinethioamide;
N-(4-cyanophenyl)hydrazinethioamide;
N-(4-(trifluoromethyl)phenyl)hydrazinethioamide;
N-ethylhydrazinethioamide;
N-isopropylhydrazinethioamide;
N-isobutylhydrazinethioamide;
N-(hydrazinecarbonothioyl)benzamide;
N-benzylhydrazinethioamide;
N-(m-tolyl)hydrazinethioamide;
(Z)-2-(5-fluoro-2-oxoindolin-3-ylidene)-N-phenylhydrazinethioamide;
(Z)-2-(5-fluoro-2-oxoindolin-3-ylidene)-N-(3-nitrophenyl)hydrazinethioamide;
(Z)-2-(5-fluoro-2-oxoindolin-3-ylidene)-N-(4-fluorophenyl)hydrazinethioamide;
(Z)-N-(4-bromophenyl)-2-(5-fluoro-2-oxoindolin-3-ylidene)hydrazinethioamide;
(Z)-N-(4-cyanophenyl)-2-(5-fluoro-2-oxoindolin-3-ylidene)hydrazinethioamide;
(Z)-2-(5-fluoro-2-oxoindolin-3-ylidene)-N-(4-(trifluoromethyl)phenyl)hydrazinethioamide;
(Z)-2-(7-fluoro-2-oxoindolin-3-ylidene)-N-(4-fluorophenyl)hydrazinethioamide;
(Z)-N-(4-bromophenyl)-2-(7-fluoro-2-oxoindolin-3-ylidene)hydrazinethioamide;
(Z)-N-(4-cyanophenyl)-2-(7-fluoro-2-oxoindolin-3-ylidene)hydrazinethioamide;
(Z)-2-(7-fluoro-2-oxoindolin-3-ylidene)-N-(4-(trifluoromethyl)phenyl)hydrazinethioamide;
(Z)-2-(6-methoxy-2-oxoindolin-3-ylidene)-N-phenylhydrazinethioamide;
(Z)-2-(6-methoxy-2-oxoindolin-3-ylidene)-N-(3-nitrophenyl)hydrazinethioamide;
(Z)-2-(6-methoxy-2-oxoindolin-3-ylidene)-N-(2-methoxyphenyl)hydrazinethioamide;
(Z)-N-(4-cyanophenyl)-2-(6-methoxy-2-oxoindolin-3-ylidene)hydrazinethioamide;
(Z)-2-(6-methoxy-2-oxoindolin-3-ylidene)-N-(4-(trifluoromethyl)phenyl)hydrazinethioamide;
(Z)-N-ethyl-2-(6-methoxy-2-oxoindolin-3-ylidene)hydrazinethioamide;
(Z)-2-(6-methoxy-2-oxoindolin-3-ylidene)-N-(m-tolyl)hydrazinethioamide;
(Z)-2-(5-methyl-2-oxoindolin-3-ylidene)-N-phenylhydrazinethioamide;
(Z)-2-(5-methyl-2-oxoindolin-3-ylidene)-N-(3-nitrophenyl)hydrazinethioamide;
(Z)-N-(2-methoxyphenyl)-2-(5-methyl-2-oxoindolin-3-ylidene)hydrazinethioamide;
(Z)-2-(5-methyl-2-oxoindolin-3-ylidene)-N-(3-(trifluoromethyl)phenyl)hydrazinethioamide;
(Z)-N-(4-fluorophenyl)-2-(5-methyl-2-oxoindolin-3-ylidene)hydrazinethioamide;
(Z)-2-(5-bromo-2-oxoindolin-3-ylidene)hydrazinethioamide;
(Z)-2-(4-bromo-2-oxoindolin-3-ylidene)-N-(3-nitrophenyl)hydrazinethioamide;
(Z)-2-(6-bromo-2-oxoindolin-3-ylidene)-N-(3-nitrophenyl)hydrazinethioamide;
(Z)-2-(4-bromo-2-oxoindolin-3-ylidene)-N-(4-nitrophenyl)hydrazinethioamide;
(Z)-2-(5-bromo-2-oxoindolin-3-ylidene)-N-(4-nitrophenyl)hydrazinethioamide;
(Z)-N-(2-methoxyphenyl)-2-(2-oxoindolin-3-ylidene)hydrazinethioamide;
(Z)-2-(4-bromo-2-oxoindolin-3-ylidene)hydrazinethioamide;
(Z)-2-(6-bromo-2-oxoindolin-3-ylidene)hydrazinethioamide;
(Z)-2-(7-bromo-2-oxoindolin-3-ylidene)hydrazinethioamide;
(Z)-2-(4-bromo-2-oxoindolin-3-ylidene)-N-phenylhydrazinethioamide;
(Z)-2-(5-bromo-2-oxoindolin-3-ylidene)-N-phenylhydrazinethioamide;
(Z)-2-(6-bromo-2-oxoindolin-3-ylidene)-N-phenylhydrazinethioamide;
(Z)-2-(7-bromo-2-oxoindolin-3-ylidene)-N-phenylhydrazinethioamide;
(Z)-N-(3-nitrophenyl)-2-(2-oxoindolin-3-ylidene)hydrazinethioamide;
(Z)-2-(5-bromo-2-oxoindolin-3-ylidene)-N-(3-nitrophenyl)hydrazinethioamide;
(Z)-2-(7-bromo-2-oxoindolin-3-ylidene)-N-(3-nitrophenyl)hydrazinethioamide;
(Z)-N-(3-methoxyphenyl)-2-(2-oxoindolin-3-ylidene)hydrazinethioamide;
(Z)-2-(4-bromo-2-oxoindolin-3-ylidene)-N-(3-methoxyphenyl)hydrazinethioamide;
(Z)-2-(5-bromo-2-oxoindolin-3-ylidene)-N-(3-methoxyphenyl)hydrazinethioamide;
(Z)-N-(4-methoxyphenyl)-2-(2-oxoindolin-3-ylidene)hydrazinethioamide;
(Z)-2-(4-bromo-2-oxoindolin-3-ylidene)-N-(4-methoxyphenyl)hydrazinethioamide;
(Z)-2-(5-bromo-2-oxoindolin-3-ylidene)-N-(4-methoxyphenyl)hydrazinethioamide;
N-(3,4-dimethoxyphenyl)hydrazinethioamide;
(Z)-N-(4-fluorophenyl)-2-(2-oxoindolin-3-ylidene)hydrazinethioamide;
(Z)-2-(6-bromo-2-oxoindolin-3-ylidene)-N-(4-nitrophenyl)hydrazinethioamide;
(Z)-2-(4-chloro-2-oxoindolin-3-ylidene)-N-phenylhydrazinethioamide;
(Z)-2-(6-chloro-2-oxoindolin-3-ylidene)-N-phenylhydrazinethioamide;
(Z)-2-(4-chloro-2-oxoindolin-3-ylidene)-N-(2-methoxyphenyl)hydrazinethioamide;
(Z)-2-(5-fluoro-2-oxoindolin-3-ylidene)-N-(3-(trifluoromethyl)phenyl)hydrazinethioamide;
(Z)-N-benzyl-2-(5-fluoro-2-oxoindolin-3-ylidene)hydrazinethioamide;
(Z)-2-(5-fluoro-2-oxoindolin-3-ylidene)-N-(m-tolyl)hydrazinethioamide;
(Z)-2-(7-fluoro-2-oxoindolin-3-ylidene)-N-(4-nitrophenyl)hydrazinethioamide;
(Z)-2-(6-methoxy-2-oxoindolin-3-ylidene)-N-(3-(trifluoromethyl)phenyl)hydrazinethioamide;
(Z)-N-(4-fluorophenyl)-2-(6-methoxy-2-oxoindolin-3-ylidene)hydrazinethioamide;
(Z)-N-(4-bromophenyl)-2-(6-methoxy-2-oxoindolin-3-ylidene)hydrazinethioamide;
(Z)-N-benzyl-2-(6-methoxy-2-oxoindolin-3-ylidene)hydrazinethioamide;
(Z)-N-(4-bromophenyl)-2-(5-methyl-2-oxoindolin-3-ylidene)hydrazinethioamide;
(Z)-N-(4-cyanophenyl)-2-(5-methyl-2-oxoindolin-3-ylidene)hydrazinethioamide;
(Z)-2-(5-methyl-2-oxoindolin-3-ylidene)-N-phenylhydrazinethioamide;
(Z)-N-ethyl-2-(5-methyl-2-oxoindolin-3-ylidene)hydrazinethioamide;
(Z)-N-benzyl-2-(5-methyl-2-oxoindolin-3-ylidene)hydrazinethioamide; and
(Z)-2-(5-methyl-2-oxoindolin-3-ylidene)-N-(m-tolyl)hydrazinethioamide.

3. The pharmaceutical composition of claim 1 or 2, which is administered orally, parenterally, or dermally.

4. A quasi-drug composition for preventing or ameliorating atopic dermatitis comprising a compound represented by Formula 1 below, an isomer thereof, or a pharmaceutically acceptable salt thereof as an active ingredient: 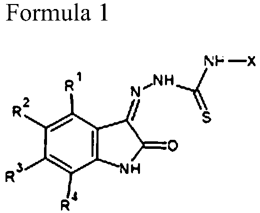
wherein in Formula 1,
X is hydrogen, C₁-C₆ alkyl, -CH₂-C₆₋₁₀ aryl, or 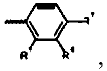
wherein C₁-C₆ alkyl and -CH₂-C₆₋₁₀ aryl are each independently unsubstituted or substituted with 1 to 3 halogens,
R¹ is hydrogen or halogen,
R² is hydrogen, halogen, hydroxy, C₁-C₆ alkyl, or C₁-C₆ alkoxy, wherein C₁-C₆ alkyl and C₁-C₆ alkoxy are each independently unsubstituted or substituted with 1 to 3 halogens,
R³, R⁴, and R⁵ are each independently hydrogen, halogen, hydroxy, or C₁-C₆ alkoxy, wherein C₁-C₆ alkoxy is unsubstituted or substituted with 1 to 3 halogens,
R⁶ is hydrogen, halogen, hydroxy, -NO₂, C₁-C₆ alkyl, or C₁-C₆ alkoxy, wherein C₁-C₆ alkyl and C₁-C₆ alkoxy are each independently unsubstituted or substituted with 1 to 3 halogens, and
R⁷ is hydrogen, halogen, hydroxy, -NO₂, -CN, C₁-C₆ alkyl, or C₁-C₆ alkoxy, wherein C₁-C₆ alkyl and C₁-C₆ alkoxy are each independently unsubstituted or substituted with 1 to 3 halogens.

5. A method for preventing or treating atopic dermatitis comprising administering to a subject a compound represented by Formula 1 below, an isomer thereof, or a pharmaceutically acceptable salt thereof: 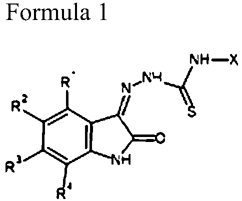
wherein in Formula 1,
X is hydrogen, C₁-C₆ alkyl, -CH₂-C₆₋₁₀ aryl, or 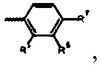
wherein C₁-C₆ alkyl and -CH₂-C₆₋₁₀ aryl are each independently unsubstituted or substituted with 1 to 3 halogens,
R¹ is hydrogen or halogen,
R² is hydrogen, halogen, hydroxy, C₁-C₆ alkyl, or C₁-C₆ alkoxy, wherein C₁-C₆ alkyl and C₁-C₆ alkoxy are each independently unsubstituted or substituted with 1 to 3 halogens,
R³, R⁴, and R⁵ are each independently hydrogen, halogen, hydroxy, or C₁-C₆ alkoxy, wherein C₁-C₆ alkoxy is unsubstituted or substituted with 1 to 3 halogens,
R⁶ is hydrogen, halogen, hydroxy, -NO₂, C₁-C₆ alkyl, or C₁-C₆ alkoxy, wherein C₁-C₆ alkyl and C₁-C₆ alkoxy are each independently unsubstituted or substituted with 1 to 3 halogens, and
R⁷ is hydrogen, halogen, hydroxy, -NO₂, -CN, C₁-C₆ alkyl, or C₁-C₆ alkoxy, wherein C₁-C₆ alkyl and C₁-C₆ alkoxy are each independently unsubstituted or substituted with 1 to 3 halogens.

6. Any one selected from the group consisting of the following compounds, an isomer thereof, or a pharmaceutically acceptable salt thereof:
(Z)-N-(4-cyanophenyl)-2-(7-fluoro-2-oxoindolin-3-ylidene)hydrazinethioamide;
(Z)-2-(7-fluoro-2-oxoindolin-3-ylidene)-N-(4-(trifluoromethyl)phenyl)hydrazinethioamide;
(Z)-2-(6-methoxy-2-oxoindolin-3-ylidene)-N-phenylhydrazinethioamide;
(Z)-2-(6-methoxy-2-oxoindolin-3-ylidene)-N-(3-nitrophenyl)hydrazinethioamide;
(Z)-2-(6-methoxy-2-oxoindolin-3-ylidene)-N-(2-methoxyphenyl)hydrazinethioamide;
(Z)-N-(4-cyanophenyl)-2-(6-methoxy-2-oxoindolin-3-ylidene)hydrazinethioamide;
(Z)-2-(6-methoxy-2-oxoindolin-3-ylidene)-N-(4-(trifluoromethyl)phenyl)hydrazinethioamide;
(Z)-N-ethyl-2-(6-methoxy-2-oxoindolin-3-ylidene)hydrazinethioamide;
(Z)-2-(5-methyl-2-oxoindolin-3-ylidene)-N-(3-nitrophenyl)hydrazinethioamide;
(Z)-2-(4-bromo-2-oxoindolin-3-ylidene)-N-(3-nitrophenyl)hydrazinethioamide;
(Z)-2-(6-bromo-2-oxoindolin-3-ylidene)-N-(3-nitrophenyl)hydrazinethioamide;
(Z)-2-(4-bromo-2-oxoindolin-3-ylidene)-N-(4-nitrophenyl)hydrazinethioamide;
(Z)-2-(4-bromo-2-oxoindolin-3-ylidene)-N-phenylhydrazinethioamide;
(Z)-2-(6-bromo-2-oxoindolin-3-ylidene)-N-phenylhydrazinethioamide;
(Z)-2-(7-bromo-2-oxoindolin-3-ylidene)-N-(3-nitrophenyl)hydrazinethioamide;
(Z)-2-(4-bromo-2-oxoindolin-3-ylidene)-N-(3-methoxyphenyl)hydrazinethioamide;
(Z)-2-(4-bromo-2-oxoindolin-3-ylidene)-N-(4-methoxyphenyl)hydrazinethioamide;
(Z)-2-(6-bromo-2-oxoindolin-3-ylidene)-N-(4-nitrophenyl)hydrazinethioamide;
(Z)-2-(4-chloro-2-oxoindolin-3-ylidene)-N-(2-methoxyphenyl)hydrazinethioamide;
(Z)-2-(7-fluoro-2-oxoindolin-3-ylidene)-N-(4-nitrophenyl)hydrazinethioamide;
(Z)-2-(6-methoxy-2-oxoindolin-3-ylidene)-N-(3-(trifluoromethyl)phenyl)hydrazinethioamide;
(Z)-N-(4-fluorophenyl)-2-(6-methoxy-2-oxoindolin-3-ylidene)hydrazinethioamide;
(Z)-N-(4-bromophenyl)-2-(6-methoxy-2-oxoindolin-3-ylidene)hydrazinethioamide;
(Z)-N-benzyl-2-(6-methoxy-2-oxoindolin-3-ylidene)hydrazinethioamide;
(Z)-N-(4-cyanophenyl)-2-(5-methyl-2-oxoindolin-3-ylidene)hydrazinethioamide; and
(Z)-2-(5-methyl-2-oxoindolin-3-ylidene)-N-(m-tolyl)hydrazinethioamide.

7. A composition comprising any one selected from the group consisting of the following compounds, an isomer thereof, or a pharmaceutically acceptable salt thereof:
(Z)-N-(4-cyanophenyl)-2-(7-fluoro-2-oxoindolin-3-ylidene)hydrazinethioamide;
(Z)-2-(7-fluoro-2-oxoindolin-3-ylidene)-N-(4-(trifluoromethyl)phenyl)hydrazinethioamide;
(Z)-2-(6-methoxy-2-oxoindolin-3-ylidene)-N-phenylhydrazinethioamide;
(Z)-2-(6-methoxy-2-oxoindolin-3-ylidene)-N-(3-nitrophenyl)hydrazinethioamide;
(Z)-2-(6-methoxy-2-oxoindolin-3-ylidene)-N-(2-methoxyphenyl)hydrazinethioamide;
(Z)-N-(4-cyanophenyl)-2-(6-methoxy-2-oxoindolin-3-ylidene)hydrazinethioamide;
(Z)-2-(6-methoxy-2-oxoindolin-3-ylidene)-N-(4-(trifluoromethyl)phenyl)hydrazinethioamide;
(Z)-N-ethyl-2-(6-methoxy-2-oxoindolin-3-ylidene)hydrazinethioamide;
(Z)-2-(5-methyl-2-oxoindolin-3-ylidene)-N-(3-nitrophenyl)hydrazinethioamide;
(Z)-2-(4-bromo-2-oxoindolin-3-ylidene)-N-(3-nitrophenyl)hydrazinethioamide;
(Z)-2-(6-bromo-2-oxoindolin-3-ylidene)-N-(3-nitrophenyl)hydrazinethioamide;
(Z)-2-(4-bromo-2-oxoindolin-3-ylidene)-N-(4-nitrophenyl)hydrazinethioamide;
(Z)-2-(4-bromo-2-oxoindolin-3-ylidene)-N-phenylhydrazinethioamide;
(Z)-2-(6-bromo-2-oxoindolin-3-ylidene)-N-phenylhydrazinethioamide;
(Z)-2-(7-bromo-2-oxoindolin-3-ylidene)-N-(3-nitrophenyl)hydrazinethioamide;
(Z)-2-(4-bromo-2-oxoindolin-3-ylidene)-N-(3-methoxyphenyl)hydrazinethioamide;
(Z)-2-(4-bromo-2-oxoindolin-3-ylidene)-N-(4-methoxyphenyl)hydrazinethioamide;
(Z)-2-(6-bromo-2-oxoindolin-3-ylidene)-N-(4-nitrophenyl)hydrazinethioamide;
(Z)-2-(4-chloro-2-oxoindolin-3-ylidene)-N-(2-methoxyphenyl)hydrazinethioamide;
(Z)-2-(7-fluoro-2-oxoindolin-3-ylidene)-N-(4-nitrophenyl)hydrazinethioamide;
(Z)-2-(6-methoxy-2-oxoindolin-3-ylidene)-N-(3-(trifluoromethyl)phenyl)hydrazinethioamide;
(Z)-N-(4-fluorophenyl)-2-(6-methoxy-2-oxoindolin-3-ylidene)hydrazinethioamide;
(Z)-N-(4-bromophenyl)-2-(6-methoxy-2-oxoindolin-3-ylidene)hydrazinethioamide;
(Z)-N-benzyl-2-(6-methoxy-2-oxoindolin-3-ylidene)hydrazinethioamide;
(Z)-N-(4-cyanophenyl)-2-(5-methyl-2-oxoindolin-3-ylidene)hydrazinethioamide; and
(Z)-2-(5-methyl-2-oxoindolin-3-ylidene)-N-(m-tolyl)hydrazinethioamide.
